(19) **Europäisches Patentamt** / **European Patent Office** / **Office européen des brevets**

(11) **EP 4 317 436 A1**

(12) **EUROPEAN PATENT APPLICATION**
published in accordance with Art. 153(4) EPC

(43) Date of publication:
**07.02.2024 Bulletin 2024/06**

(21) Application number: **22780953.0**

(22) Date of filing: **29.03.2022**

(51) International Patent Classification (IPC):
*C12N 15/40* [(2006.01)]   *A61K 39/12* [(2006.01)]
*A61P 1/12* [(2006.01)]   *A61P 31/12* [(2006.01)]
*C07K 14/08* [(2006.01)]   *C07K 16/10* [(2006.01)]
*C12N 15/63* [(2006.01)]

(52) Cooperative Patent Classification (CPC):
**A61K 39/12; A61P 1/12; A61P 31/12; C07K 14/08;
C07K 16/10; C12N 15/63**

(86) International application number:
**PCT/JP2022/015581**

(87) International publication number:
**WO 2022/210742 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2021 JP 2021055049**

(71) Applicant: **Denka Company Limited
Tokyo 103-8338 (JP)**

(72) Inventors:
• **FUKUSHIMA, Naoshi
Tokyo 103-8338 (JP)**

• **HOSHIGA, Fumiya
Tokyo 103-8338 (JP)**
• **MIKI, Motohiro
Tokyo 103-8338 (JP)**
• **HISAIE, Kota
Tokyo 103-8338 (JP)**

(74) Representative: **Vossius & Partner
Patentanwälte Rechtsanwälte mbB
Siebertstrasse 3
81675 München (DE)**

Remarks:
The complete document including Reference
Table(s) and the Sequence Listing(s) can be
downloaded from the EPO website

(54) **PEPTIDE OF NOROVIRUS ORIGIN, POLYNUCLEOTIDE, ANTIBODY, COMPOSITION, METHOD FOR IDENTIFYING NEUTRALIZING EPITOPE FOR NOROVIRUS**

(57)     The present invention provides a peptide that can be used to induce protective immunity to a norovirus belonging to a genotype of GII. A peptide comprising a portion of an amino acid sequence of a P domain of a VP1 protein of a norovirus, in which the norovirus belongs to any of genotypes of genogroup II (GII) (except genotype 4).

EP 4 317 436 A1

**Description**

TECHNICAL FIELD

The present application relates to the field of norovirus vaccines. In particular, it relates to a peptide comprising a neutralizing epitope for a norovirus, an antibody that recognizes the peptide, a polynucleotide encoding the peptide, a composition for treating a norovirus, and a method for identifying a neutralizing epitope.

BACKGROUND ART

Norovirus infections occur throughout the year, but are known to become epidemic particularly during the winter months. Symptoms include abdominal pain, diarrhea, nauseous feeling, vomiting, and fever. The routes of infection include ingestion of food or water contaminated with norovirus, contact with humans or objects that harbor norovirus, and droplets from persons infected with norovirus. Since noroviruses are generally resistant to disinfection and heat and are highly contagious, secondary infections can easily occur.

Antibiotics are an effective treatment for bacterial infections. However, with norovirus infections, the circumstances are different from bacterial infections, and there are no specific drugs. Therefore, when infected with norovirus, one has to wait for the virus to be excreted out of the body and recover naturally.

Against this background, vaccines are being developed with the purpose of preventing norovirus infection. Patent Literature 1 discloses a method for administering parenterally a vaccine composition to humans to induce protective immunity to a norovirus. Here, the vaccine composition comprises virus-like proteins (sometimes also referred to as "VLP") of genogroup I genotype 1 (GI.1) and/or genogroup II genotype 4 (GII.4) norovirus.

Noroviruses are currently classified into 10 genogroups based on their genome and capsid protein sequences (Non Patent Literature 1). Then there are one or more genotypes in each genogroup. For example, there are 27 genotypes (GII.1, GII.2, ..., GII.27) in genogroup II (GII). In this regard, Non Patent Literature 2 discloses the identification of a blockade epitope (also referred to as an inhibitory epitope) for GII.4. However, noroviruses are known to differ antigenically from one another among different genotypes, even if the genogroup is the same. Much of the research on noroviruses has been directed toward GII.4, which accounts for the majority of norovirus infections.

CITATION LIST

PATENT LITERATURE

PTL 1: Japanese Patent Laid-Open No. 2017-214357

NON PATENT LITERATURE

NPL 1: Chhabra et al., Journal of General Virology 2019;100:1393-1406
NPL 2: Lindesmith LC, Baric RS: Immunity 50, 1530, 2019

SUMMARY OF INVENTION

TECHNICAL PROBLEM

The genotypes of GII are diverse. Therefore, vaccine compositions of prior art may not be effective in inducing protective immunity to noroviruses belonging to GII. The present invention provides a peptide that can be used to induce protective immunity to a norovirus belonging to a GII genotype.

SOLUTION TO PROBLEM

In light of the above, the inventor of the present application focused on the P (protruding) domain of the VP1 (viral protein 1) protein of norovirus and conducted extensive studies from the aspect of three-dimensional structure. This led to the identification of a plurality of potential epitope regions. Based on such findings, the inventor of the present application has completed the present invention.

The present invention provides, but is not limited to the following.

(1) A peptide comprising a portion of an amino acid sequence of a P domain of a VP1 protein of a norovirus, wherein the norovirus belongs to any of genotypes of genogroup II (GII) (except genotype 4).

(2) The peptide of (1), wherein the norovirus belongs to a genotype selected from the group consisting of GII.1, 2, 3, 5, 6, 7, 12, 13, 14, 17, and 21.

(3) The peptide of (1) or (2), wherein the portion of an amino acid sequence of a P domain comprises the whole or a portion of an amino acid sequence of a neutralizing epitope selected from the group consisting of epitope A, epitope D, epitope E, epitope F, and combinations thereof.

(4) The peptide of any of (1) to (3), wherein the epitope A has an amino acid sequence selected from the group consisting of SEQ ID NOs: 1 to 267,

the epitope D has an amino acid sequence selected from the group consisting of SEQ ID NOs: 268 to 351,
the epitope E has an amino acid sequence selected from the group consisting of SEQ ID NOs: 352 to 390, and
the epitope F has an amino acid sequence selected from the group consisting of SEQ ID NOs: 391 to 425.

(5) The peptide of any of (1) to (4), further comprising an amino acid sequence which is not present in a P domain of a VP1 protein of a naturally occurring norovirus belonging to any of genotypes of GII (except genotype 4).

(6) The peptide of any of (1) to (5), further comprising an amino acid sequence which is not present in a P domain of a VP1 protein of a naturally occurring norovirus belonging to a genotype selected from the group consisting of GII. 1, 2, 3, 5, 6, 7, 12, 13, 14, 17, and 21.

(7) A polynucleotide encoding the peptide of any of (1) to (6).

(8) The polynucleotide of (7), incorporated into an expression vector.

(9) The polynucleotide of (7) or (8), introduced into a host cell.

(10) An antibody that recognizes the peptide of any of (1) to (6).

(11) The antibody of (10), wherein the antibody recognizes the P domain of a VP1 protein of a norovirus.

(12) A composition comprising the peptide of any of (1) to (6).

(13) A composition comprising the polynucleotide of any of (7) to (9).

(14) The composition of (12) or (13), which is a vaccine.

(15) The composition of any of (12) to (14), for use in inducing protective immunity to a norovirus belonging to any of genotypes of GII (except genotype 4).

(16) The composition of any of (12) to (15), for use in inducing protective immunity to a norovirus belonging to a genotype selected from the group consisting of GII. 1, 2, 3, 5, 6, 7, 12, 13, 14, 17, and 21.

(17) A composition comprising the antibody of (10) or (11).

(18) The composition of any of (12) to (17), for use in treating an infection with a norovirus belonging to any of genotypes of GII (except genotype 4).

(19) The composition of any of (12) to (18), for use in treating an infection with a norovirus belonging to a genotype selected from the group consisting of GII.1, 2, 3, 5, 6, 7, 12, 13, 14, 17, and 21.

(20) A method for identifying a neutralizing epitope for a norovirus, the method comprising:

collecting genome sequences of one or more norovirus strains belonging to a specific genotype, and extracting gene sequences of a VP1 protein from the genome sequences,
aligning amino acid sequences of the VP1 protein, and classifying the norovirus strains into one or more clusters based on the similarity of the amino acid sequences, the phylogenetic closeness of the strains from which these amino acid sequences are derived, or a combination thereof,
predicting, by homology modeling, a three-dimensional structure of a P domain of the VP1 protein of the norovirus strains representing the clusters,
comparing the predicted three-dimensional structure of the P domain with a three-dimensional structure of a P domain of a VP1 protein of a reference strain of norovirus to identify, as a neutralizing epitope, a predicted portion of the P domain that structurally corresponds to a neutralizing epitope of the P domain of the reference strain and an amino acid sequence in the vicinity of that portion, which is a highly variable region between genotypes, and
comparing an amino acid sequence of the P domain of the VP1, which comprises the identified neutralizing epitope, with an amino acid sequence of a P domain of VP1 from a different strain that belongs to the same genotype as the above-described strain from which the amino acid sequence is derived and whose three-dimensional structure was not compared above, to determine a portion in the amino acid sequence of the P domain of VP1 from the different strain corresponding to the amino acid sequence of the neutralizing epitope, and identify that portion as the neutralizing epitope of the P domain of VP1 from the different strain,
wherein the reference strain of norovirus belongs to a genotype different from one or more norovirus strains belonging to that specific genotype, and

the neutralizing epitope of the P domain of the VP1 protein of the reference strain is known.

(21) The method of (20), wherein the norovirus belonging to the specific genotype is a norovirus belonging to any of genotypes of genogroup II (GII) (except genotype 4).

(22) The method of (20) or (21), wherein the norovirus belonging to the specific genotype is a norovirus belonging to a genotype selected from the group consisting of GII.1, 2, 3, 5, 6, 7, 12, 13, 14, 17, and 21.

(23) The method of any of (20) to (22), wherein the reference strain of norovirus is selected from norovirus strains belonging to GII.4.

BRIEF DESCRIPTION OF DRAWINGS

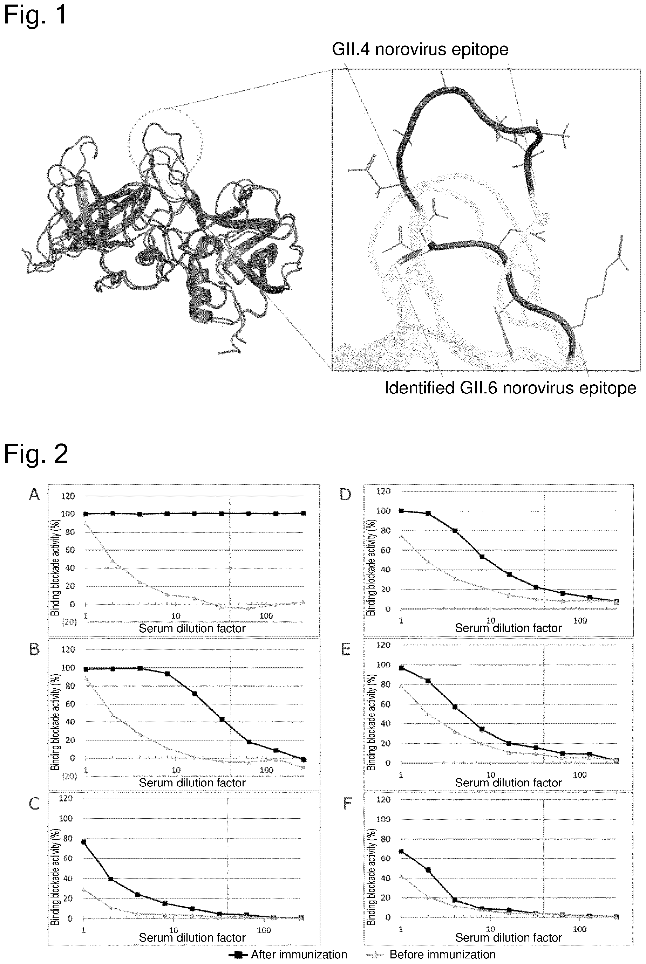

[Fig. 1] Left: three-dimensional structure of the P domain of the VP1 protein of a GII.4 norovirus (PDB ID: 4oov) and structural alignment of the three-dimensional structure model of a GII.6 norovirus (AB078337). Right: the known neutralizing epitope (D) of a GII.4 norovirus and the epitope of a GII.6 norovirus identified by structural similarity thereto (SEQ ID NO:310).

[Fig. 2] Results of PGM binding blockade activity. (A) Serum collected from rabbits immunized with GII.6 epitope A, (B) serum collected from rabbits immunized with GII.6 epitope D, (C) serum collected from rabbits immunized with GII.6 epitope E, (D) serum collected from rabbits immunized with GII.17 epitope A, (E) serum collected from rabbits immunized with GII.17 epitope D, and (F) serum collected from rabbits immunized with GII.17 epitope E.

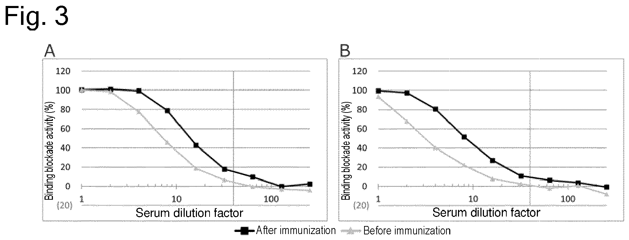

[Fig. 3] Results of PGM binding blockade activity. (A) Serum collected from rabbits immunized with GII.2 epitope A, and (B) serum collected from rabbits immunized with GII.2 epitope D.

DESCRIPTION OF EMBODIMENTS

<Peptide>

The present invention provides a peptide comprising a specific amino acid sequence. The specific amino acid sequence comprises a portion of an amino acid sequence of a P domain of a VP1 protein of a norovirus or an amino acid sequence derived from the P domain. However, the specific amino acid sequence does not comprise the whole P domain of a VP1 protein of a norovirus.

Norovirus is a virus belonging to the family Caliciviridae. Norovirus is known to have 10 genogroups (GI, GII, GIII, GIV, GV, GVI, GVII, GVIII, GIX, and GX). Noroviruses belonging to GI, GII, GIV, GVIII, and GIX are known to infect humans, noroviruses belonging to GIII infect cattle, noroviruses belonging to GV infect mice, noroviruses belonging to GVI infect cats, noroviruses belonging to GVII infect dogs, and noroviruses belonging to GX infect bats. Each genogroup has genotypes, classified or clustered based on the gene sequence of the norovirus. For example, with respect to GI and GII, various genotypes are known to exist. At present, GI has nine genotypes (GI. 1 to 9) and GII has 27 genotypes (GII.1 to 27).

The specific amino acid sequence constituting the peptide provided by the present invention comprises a portion of an amino acid sequence of a P domain of a VP1 protein of a norovirus belonging to any of genotypes of GII. Here, genotype 4 is excluded from any of genotypes of GII. Any of genotypes of GII can be selected, for example, from the group consisting of 1, 2, 3, 5, 6, 7, 12, 13, 14, 17, and 21, but is not limited thereto.

Furthermore, the portion of an amino acid sequence of a P domain of a VP1 protein comprises the whole or a portion of an amino acid sequence corresponding to a neutralizing epitope selected from the group consisting of epitope A, epitope D, epitope E, epitope F, and combinations thereof. Here, the combination of epitope A, epitope D, epitope E, and epitope F can be a plurality of combinations of these epitopes, for example, two, three, four, and more. Furthermore, the combination may be a combination of the same epitope or a combination of different epitopes. The plurality of combinations of these epitopes can be directly linked epitopes or epitopes linked via any amino acid sequence. A method for linking a plurality of peptides or epitopes and a method for designing any amino acid sequence are well known and can be appropriately performed by those skilled in the art.

Epitope A comprises an amino acid sequence selected from the group consisting of A1 region, A2 region, A3 region, and combination thereof, in amino acid sequences of a P domain of a VP1 protein of any of genotypes of naturally occurring GII (except GII.4) noroviruses. Here, the combination is a combination of the A1 and A2 regions, a combination of the A1 and A3 regions, a combination of the A2 and A3 regions, or a combination of the A1, A2, and A3 regions. For example, the A1, A2, and A3 regions can exist at the following positions in the amino acid sequence of the VP1 protein, counting from the N-terminus.

(1) GII.1

A1 region: positions 288 to 305, preferably 293 to 300
A2 region: positions 350 to 363, preferably 355 to 360
A3 region: positions 366 to 382, preferably 369 to 376

(2) GII.2

A1 region: positions 284 to 305, preferably 289 to 300
A2 region: positions 355 to 369, preferably 360 to 365
A3 region: positions 372 to 393, preferably 375 to 388 or 375 to 388

(3) GII.3

A1 region: positions 284 to 318, preferably 289 to 313 or 289 to 312
A2 region: positions 361 to 376, preferably 366 to 373 or 365 to 372
A3 region: positions 378 to 397, preferably 380 to 392 or 381 to 393

(4) GII.5

A1 region: positions 282 to 303, preferably 287 to 298 or 289 to 300
A2 region: positions 351 to 364, preferably 356 to 361 or 358 to 363
A3 region: positions 366 to 380, preferably 370 to 375 or 372 to 377

(5) GII.6

A1 region: positions 285 to 316, preferably 290 to 311
A2 region: positions 364 to 380, preferably 369 to 374 or 372 to 377
A3 region: positions 381 to 397, preferably 382 to 392 or positions 385 to 395

(6) GII.7

A1 region: positions 290 to 306, preferably 295 to 301
A2 region: positions 354 to 367, preferably 359 to 364
A3 region: positions 369 to 386, preferably 373 to 381

(7) GII.12

A1 region: positions 285 to 305, preferably 290 to 300
A2 region: positions 350 to 363, preferably 355 to 360
A3 region: positions 365 to 379, preferably 369 to 374

(8) GII.13

A1 region: positions 284 to 305, preferably 289 to 300, 291 to 301, or 291 to 302
A2 region: positions 352 to 366, preferably 357 to 362, 358 to 363, or 359 to 364
A3 region: positions 369 to 384, preferably 371 to 379, 372 to 380, or 373 to 381

(9) GII.14

A1 region: positions 288 to 304, preferably 293 to 299 or 293 to 301
A2 region: positions 349 to 366, preferably 354 to 359, 355 to 360, or 357 to 362
A3 region: positions 367 to 378, preferably 368 to 373, 369 to 374, or 371 to 376

(10) GII.17

A1 region: positions 282 to 305, preferably 287 to 297, 289 to 299, or 289 to 301
A2 region: positions 349 to 367, preferably 354 to 362, 356 to 364, 357 to 365, or 358 to 366

A3 region: positions 368 to 385, preferably 369 to 380, 369 to 381, 371 to 382, 371 to 383, 372 to 384, 373 to 385, or 370 to 384

(11) GII.21

A1 region: positions 283 to 304, preferably 288 to 299 or 289 to 300
A2 region: positions 352 to 365, preferably 357 to 362 or 358 to 363
A3 region: positions 368 to 382, preferably 371 to 377 or 372 to 378.

The amino acid sequence of epitope A may have mutations such as substitutions, deletions, truncations, insertions, and modifications introduced into the corresponding natural amino acid sequence, or may not have such mutations introduced. If the amino acid sequence of epitope A has such mutations, mutations that do not substantially affect the three-dimensional structure are preferred, and examples thereof include conservative substitutions. Those skilled in the art are familiar with conservative substitutions and can perform them as appropriate. In addition, the amino acid sequence of epitope A may have 70% or more, 80% or more, 90% or more, 95% or more, 98% or more, or 99% or more homology to the corresponding natural amino acid sequence. The introduction of mutations and/or variations in sequence homology to the amino acid sequence of epitope A are acceptable to the extent that an immune response to a norovirus can be induced. Although not limited, epitope A can have, for example, an amino acid sequence selected from the group consisting of SEQ ID NOs: 1 to 267 (Table 1), an amino acid sequence in which a mutation described above is introduced into the amino acid sequence, or an amino acid sequence having the above sequence homology to the amino acid sequence.
More specific examples of the amino acid sequence constituting epitope A comprise, but are not limited to the following:

- an amino acid sequence selected from the group consisting of SEQ ID NOs: 1 to 113 as the A1 region,
- an amino acid sequence selected from the group consisting of SEQ ID NOs: 114 to 160 as the A2 region,
- an amino acid sequence selected from the group consisting of SEQ ID NOs: 161 to 267 and 428 as the A3 region,
- an amino acid sequence having the A1 region and the A2 region,
- an amino acid sequence having the A1 region and the A3 region,
- an amino acid sequence having the A2 region and the A3 region,
- an amino acid sequence having the A1 region, the A2 region, and the A3 region,
- the above amino acid sequence in which a substitution, deletion, truncation, insertion, modification, or the like of an amino acid residue is introduced in any of the A1 region, A2 region, and/or A3 region,
- an amino acid sequence having 70% or more, 80% or more, 90% or more, 95% or more, 98% or more, or 99% or more homology to the amino acid sequence of any of the A1 region, A2 region, or A3 region.

When two or three of the A1 region, A2 region, and A3 region are combined, they may be adjacent to each other or separated via any amino acid sequence within epitope A.
Epitope D comprises a portion of the amino acid sequence of the P domain of the VP1 protein of any of genotypes of GII (except GII.4) in a naturally occurring norovirus. For example, epitope D can exist at the following positions in the amino acid sequence of the VP1 protein, counting from the N-terminus.

(1) GII.1: positions 385 to 395, preferably 390 to 394
(2) GII.2: positions 394 to 405, 394 to 403, preferably 396 to 403, 395 to 405
(3) GII.3: positions 398 to 410, preferably 403 to 409 or 404 to 410
(4) GII.5: positions 387 to 399, preferably 392 to 397 or 394 to 399
(5) GII.6: positions 399 to 411, preferably 404 to 408, or 407 to 411, 401 to 410
(6) GII.7: positions 390 to 400, preferably 395 to 399
(7) GII. 12: positions 386 to 396, preferably 391 to 396
(8) GII. 13: positions 387 to 401, preferably 392 to 399, 393 to 400, or 394 to 401
(9) GII.14: positions 385 to 399, preferably 390 to 396, 391 to 397, or 393 to 399
(10) GII. 17: positions 386 to 403, 386 to 402, preferably 387 to 397, 388 to 399, 389 to 399, 390 to 400, 390 to 401, 390 to 402, 391 to 401, 392 to 402, or 388 to 403
(11) GII.21: positions 388 to 400, preferably 393 to 399 or 394 to 400

The amino acid sequence of epitope D may have mutations such as substitutions, deletions, truncations, insertions, and modifications introduced into the corresponding natural amino acid sequence, or may not have such mutations introduced. If the amino acid sequence of epitope D has such mutations, mutations that do not substantially affect the three-dimensional structure are preferred, and examples thereof include conservative substitutions. Those skilled in the art are familiar with conservative substitutions and can perform them as appropriate. In addition, the amino acid sequence of epitope D may have 70% or more, 80% or more, 90% or more, 95% or more, 98% or more, or 99% or more homology to the corresponding natural amino acid sequence. The introduction of mutations and/or variations in sequence homology to the amino acid sequence of epitope D are acceptable to the extent that an immune response to a norovirus can be induced. Although not limited, epitope D can have, for example, an amino acid sequence selected from the group consisting of SEQ ID NOs: 268 to 351 (Table 1), 426, 429, and 431, an amino acid sequence in which a mutation described above is introduced into the amino acid sequence, or an amino acid sequence having the above sequence homology to the amino acid sequence.

Epitope E comprises a portion of the amino acid sequence of the P domain of the VP1 protein of any of genotypes of GII (except GII.4) in a naturally occurring norovirus. For example, epitope E can exist at the following positions in the amino acid sequence of the VP1 protein, counting from the N-terminus.

(1) GII.1: positions 401 to 413, preferably 402 to 408
(2) GII.2: positions 408 to 420, preferably 409 to 415
(3) GII.3: positions 415 to 427, preferably 415 to 421 or 416 to 422
(4) GII.5: positions 405 to 418, preferably 405 to 411 or 407 to 413
(5) GII.6: positions 413 to 428, 414 to 428, preferably 414 to 420, 417 to 423, 413 to 423
(6) GII.7: positions 407 to 418, preferably 407 to 413
(7) GII.12: positions 402 to 413, preferably 402 to 408
(8) GII. 13: positions 405 to 418, preferably 405 to 411, 406 to 412, or 407 to 413
(9) GII. 14: positions 402 to 416, preferably 402 to 408, 403 to 409, or 405 to 411
(10) GII. 17: positions 403 to 420, preferably 403 to 410, 405 to 412, 406 to 413, 407 to 414, 408 to 415, or 406 to 415
(11) GII.21: positions 405 to 417, preferably 405 to 411 or 406 to 412

The amino acid sequence of epitope E may have mutations such as substitutions, deletions, truncations, insertions, and modifications introduced into the corresponding natural amino acid sequence, or may not have such mutations introduced. If the amino acid sequence of epitope E has such mutations, mutations that do not substantially affect the three-dimensional structure are preferred, and examples thereof include conservative substitutions. Those skilled in the art are familiar with conservative substitutions and can perform them as appropriate. In addition, the amino acid sequence of epitope E may have 70% or more, 80% or more, 90% or more, 95% or more, 98% or more, or 99% or more homology to the corresponding natural amino acid sequence. The introduction of mutations and/or variations in sequence homology to the amino acid sequence of epitope E are acceptable to the extent that an immune response to a norovirus can be induced. Although not limited, epitope E can have, for example, an amino acid sequence selected from the group consisting of SEQ ID NOs: 352 to 390 (Table 1), 427, and 430, an amino acid sequence in which a mutation described above is introduced into the amino acid sequence, or an amino acid sequence having the above sequence homology to the amino acid sequence.

Epitope F comprises an amino acid sequence selected from the group consisting of F1 region, F2 region, and combination thereof, in amino acid sequences of a P domain of a VP1 protein of any of genotypes of naturally occurring GII (except GII.4) noroviruses. For example, the F1 and F2 regions can exist at the following positions in the amino acid sequence of the VP1 protein, counting from the N-terminus.

(1) GII.1

F1 region: positions 322 to 337, preferably 327 to 332
F2 region: positions 396 to 400, preferably 397 to 400

(2) GII.2

F1 region: positions 322 to 337, preferably 327 to 332
F2 region: positions 404 to 407

(3) GII.3

F1 region: positions 334 to 350, preferably 339 to 344 or 340 to 345
F2 region: positions 410 to 414, preferably 410 to 413 or 411 to 414

(4) GII.5

F1 region: positions 320 to 337, preferably 325 to 330 or 327 to 332
F2 region: positions 400 to 405, preferably 400 to 403 or 402 to 405

(5) GII.6

F1 region: positions 332 to 347, preferably 337 to 342
F2 region: positions 409 to 415, preferably 409 to 412 or 412 to 415

(6) GII.7

F1 region: positions 323 to 338, preferably 328 to 333
F2 region: positions 401 to 406, preferably 402 to 405

(7) GII.12

F1 region: positions 322 to 337, preferably 327 to 332
F2 region: positions 397 to 401, preferably 397 to 400

(8) GII.13

F1 region: positions 320 to 337, preferably 325 to 330, 326 to 331, or 327 to 332
F2 region: positions 400 to 405, preferably 400 to 403, 401 to 404, or 402 to 405

(9) GII.14

F1 region: positions 321 to 338, preferably 326 to 331 or 328 to 333
F2 region: positions 397 to 403, preferably 397 to 400, 398 to 401 or 400 to 403

(10) GII.17

F1 region: positions 319 to 338, preferably 324 to 329, 326 to 331, or 328 to 333
F2 region: positions 398 to 406, preferably 398 to 401, 400 to 403, 401 to 404, 402 to 405, or 403 to 406

(11) GII.21

F1 region: positions 321 to 337, preferably 326 to 331 or 327 to 332
F2 region: positions 400 to 404, preferably 400 to 403 or 401 to 404.

The amino acid sequence of epitope F may have mutations such as substitutions, deletions, truncations, insertions, and modifications introduced into the corresponding natural amino acid sequence, or may not have such mutations introduced. If the amino acid sequence of epitope F has such mutations, mutations that do not substantially affect the three-dimensional structure are preferred, and examples thereof include conservative substitutions. Those skilled in the art are familiar with conservative substitutions and can perform them as appropriate. In addition, the amino acid sequence of epitope F may have 70% or more, 80% or more, 90% or more, 95% or more, 98% or more, or 99% or more homology to the corresponding natural amino acid sequence. The introduction of mutations and/or variations in sequence homology to the amino acid sequence of epitope F are acceptable to the extent that an immune response to a norovirus can be induced. Although not limited, epitope F can have, for example, an amino acid sequence selected from the group consisting of SEQ ID NOs: 391 to 425 (Table 1), an amino acid sequence in which a mutation described above is introduced into the amino acid sequence, or an amino acid sequence having the above sequence homology to the amino acid sequence.

More specific examples of the amino acid sequence constituting epitope F include, but are not limited to the following:

- an amino acid sequence selected from the group consisting of SEQ ID NOs: 391 to 415 as the F1 region,
- an amino acid sequence selected from the group consisting of SEQ ID NOs: 416 to 425 as the F2 region,
- an amino acid sequence having the F1 region and the F2 region,
- the above amino acid sequence in which a substitution, deletion, truncation, insertion, modification, or the like of an amino acid residue is introduced in any of the F 1 region, and/or F2 region,

- an amino acid sequence having 70% or more, 80% or more, 90% or more, 95% or more, 98% or more, or 99% or more homology to the amino acid sequence of any of the F1 region or F2 region.

When the F1 region and F2 region are combined, they may be adjacent to each other or separated via any amino acid sequence within epitope F.

In addition to the amino acid sequences described above for epitopes A to F, the peptide of the present invention can have any amino acid sequence within the epitopes A to F or at their N-terminal or C-terminal side. For example, the peptide can have an amino acid sequence other than the P domain of the VP1protein derived from naturally occurring noroviruses (noroviruses belonging to any of genotypes of GII (except genotype 4), e.g., norovirus belonging to a genotype selected from the group consisting of Gil. 1, 2, 3, 5, 6, 7, 12, 13, 14, 17, and 21), an amino acid sequence not derived from naturally occurring noroviruses (noroviruses belonging to any of genotypes of GII (except genotype 4), e.g., norovirus belonging to a genotype selected from the group consisting of Gil. 1, 2, 3, 5, 6, 7, 12, 13, 14, 17, and 21), or a non-natural amino acid sequence. The use of any amino acid sequence is acceptable as long as the peptide of the present invention can induce an immune response to a norovirus. Therefore, the amino acid sequence of the peptide of the present invention, taken as a whole, can be an amino acid sequence that does not occur naturally.

The peptide of the present invention can be obtained by chemical synthesis. Peptide synthesis can be performed in-house or outsourced to an external institution. The peptide can be synthesized by any known method. For example, liquid-phase peptide synthesis methods or solid-phase peptide synthesis methods are well known. Examples of solid-phase peptide synthesis methods include the Boc solid-phase method and the Fmoc solid-phase method. Those skilled in the art are familiar with the methods and conditions for peptide synthesis.

Alternatively, the peptide of the present invention can be biologically produced using a polynucleotide encoding the peptide. The peptide of the present invention can be produced by introducing such polynucleotide into appropriate host cells and culturing the host cells. The polynucleotide (nucleic acid sequence) encoding the peptide of the present invention, the expression vector comprising the polynucleotide, and the host cells into which the polynucleotide is introduced are described elsewhere in the present description.

The obtained peptide can also be purified if necessary. Purification can be performed based on the physicochemical properties such as the size, charge, and hydrophobicity of the peptide. Examples thereof include size exclusion chromatography, ion exchange chromatography, partition chromatography, and high-performance liquid (normal or reversed phase) chromatography. Those skilled in the art are familiar with the methods and conditions for peptide purification.

<Antibody>

The present invention provides an antibody that recognizes at least one peptide of the present invention. Here, "recognize" means that the antibody selectively interacts (binds) with the target peptide, and is distinguished from non-selective interaction. The interaction between the antibody and the target peptide can be evaluated by known means. Examples thereof include ELISA, immunoblotting, and immunoprecipitation. Those skilled in the art can use these means as appropriate.

The antibody may be a polyclonal antibody or a monoclonal antibody. Furthermore, the antibody may be a chimeric antibody such as humanized antibodies. Chimeric antibodies can be produced by any method known to those skilled in the art. The antibody can be at least one selected from the group consisting of IgG, IgA, IgY, IgD, IgM, IgE, and fragments thereof, but is not limited thereto. The fragments can include, for example, heavy chains, light chains, Fc, or F(ab). In addition, the antibody may consist of a single antibody or fragment thereof, or it may consist of two or more antibodies or fragments thereof.

The antibody of the present invention can recognize at least one norovirus belonging to any of genotypes of GII (except G II.4). For example, the antibody of the present invention can recognize at least one norovirus belonging to a specific genotype of GII. For example, the antibody can recognize noroviruses belonging to at least one selected from the group consisting of GII.1, GII.2, GII.3, GII.5, GII.6, GII.7, GII.12, GII.13, GII. 14, GII. 17, and GII.21.

The antibody is expected to bind to the P domain of the VP1 protein of the norovirus. For example, the antibody can bind to at least one of epitope A, epitope D, epitope E, and epitope F in the P domain of the VP1 protein of the norovirus.

The antibody of the present invention can be produced using the peptide of the present invention. The methods for producing antibodies using peptides are well known to those skilled in the art. A method for producing antibodies is provided that comprises, but is not limited to, the following steps.

The method for producing antibodies comprises a step of administering the peptide of the present invention to a subject. This step can immunize the subject and make it produce antibodies to the peptide. Here, the subject can be clinically and/or experimentally used and is capable of producing antibodies. Examples of the subject include, but are not limited to, humans, pigs, cattle, rodents (mice, rats, guinea pigs, etc.), dogs, cats, sheep, rabbits, and birds (chickens, ostriches, etc.). The conditions of immunization (dosage of antigen peptide, frequency of administration, timing of administration, site of administration, etc.) are well known to those skilled in the art and can be set appropriately without requiring explanation.

The method for producing antibodies further comprises a step of collecting the antibodies produced in the subject. In the collection step, the antibody of interest can be obtained by collecting body fluids (e.g., blood, serum, plasma, and ascites) from the immunized subject. Alternatively, in the collection step, the antibody of interest can also be obtained by collecting antibody-producing cells from the immunized subject. In this case, a hybridoma strain with the ability to proliferate can be established by fusing the antibody-producing cells with other cells and the like, as necessary. The method for generating hybridoma cells is well known to those skilled in the art and requires no particular explanation.

The method for producing antibodies can further comprises a step of purifying the collected antibodies and/or a step of evaluating the interaction between the collected antibodies and the target peptide, if necessary. The purification of the antibodies and/or the evaluation of the interaction between the antibodies and the target peptide can be performed by known means.

<Polynucleotide, expression vector, and host cells>

The present invention provides a polynucleotide encoding the peptide of the present invention. Since the correspondence between amino acid residues and gene codons has already been established, it is easy to convert a peptide sequence into its corresponding polynucleotide sequence. The polynucleotide may be either RNA or DNA, but from the aspect of handling and storage, DNA is more convenient.

The polynucleotide of the present invention can be obtained by chemical synthesis. Polynucleotide synthesis can be performed in-house or outsourced to an external institution. The polynucleotide may be synthesized by any known method. For example, solid-phase synthesis methods are well known. Those skilled in the art are familiar with the methods and conditions for polynucleotide synthesis.

Alternatively, the polynucleotide of the present invention can be obtained by a biological method. For example, the nucleic acid sequence encoding the amino acid sequence of the peptide of the present invention can be amplified by reverse transcribing RNA encoding a P domain of a VP 1 protein of a norovirus into cDNA, and performing a gene amplification reaction (reverse transcription PCR) using the cDNA as the template. The primers for the PCR are designed so that the region comprising the nucleic acid sequence encoding the amino acid sequence of the peptide of the present invention is amplified. That is, the primers should be designed to anneal to nucleic acid sequences that are upstream and/or downstream of the nucleic acid sequence encoding the amino acid sequence of the peptide of the present invention. For example, since the polynucleotide encoding the amino acid sequence represented by any of the SEQ ID NOs: 1 to 425 (Table 1) is present on the gene for the P domain of the VP1 protein of norovirus, the primers can be created based on the nucleic acid sequences upstream and/or downstream thereof.

Information on the gene sequence comprising the gene encoding the P domain of the VP1 protein of norovirus can be obtained from databases publicly available on the Internet and the like (including, but not limited to, GenBank, EMBL, and DDBJ (Japanese DNA database)).

The polynucleotide described above can be subjected to extraction and/or purification as necessary at any time during its preparation. Those skilled in the art can appropriately select a known method for the extraction and/or purification of the polynucleotide.

The polynucleotide of the present invention can be incorporated into an expression vector. Therefore, the present invention also provides such expression vector. Examples of expression vectors include pET for *E. coli* expression, pAUR for yeast expression, pIEx-1 for insect cell expression, and pBApo-CMV for animal cell expression, but other known vectors can also be used. The incorporation of the polynucleotide into an expression vector can be performed by a known method.

The expression vector of the present invention can be introduced into appropriate host cells. Therefore, the present invention provides the host cells into which the polynucleotide has been introduced. The host cells are not particularly limited as long as they are capable of producing the peptide of the present invention. For example, insect-derived cells (e.g., Sf9 and Hi5 cells), *E. coli,* yeasts (e.g., *Saccharomyces cerevisiae, Saccharomyces pombe,* and *Pichia pastoris*), mammalian cells (CHO, HEK, etc.) and any other cells can be used as host cells. The peptide of the present invention can be produced by culturing the host cells of the present invention. The introduction of the expression vector into the host cells and the culture of the host cells can be performed by a known method.

<Composition>

The present invention provides a composition comprising at least one peptide of the present invention described above. Moreover, the present invention provides a composition comprising at least one polynucleotide described above. Furthermore, the present invention provides a composition comprising at least one antibody of the present invention described above. In the following description, simply referring to "the composition of the present invention" is intended to comprehensively describe these three compositions.

The composition of the present invention may comprise additional components as long as the effects of the invention are exhibited. The additional components include, but are not limited to, excipients, diluents, pH adjusters, preservatives, carriers, suspending agents, solubilizers, thickeners, stabilizers, antiseptic agents, penetrating agents, and adjuvants. Known components can be appropriately selected as additional components.

The composition of the present invention may be for either oral or parenteral use, and may be formulated in a form suitable for the intended route of administration. A parenteral composition can be administered by any route, including intravenous, intraarterial, intramuscular, intraperitoneal, intranasal, transdermal, subcutaneous, buccal, sublingual, rectal, oral, ocular, vaginal, and pulmonary.

The form of the composition of the present invention is not limited, but can be, for example, a tablet, a capsule, a pill, a syrup, an elixir, an emulsion, an aerosol, an aqueous or non-aqueous injectable solution, or a powder, granules, or tablet for an injectable solution (the injectable solution is prepared by adding an aqueous or non-aqueous liquid excipient to the powder, the granules, or the tablet).

The composition of the present invention can prevent, treat, alleviate, or ameliorate (improve) norovirus infection, disease induced by norovirus, or at least one symptom associated with the infection and/or disease. Here, norovirus infection, disease induced by norovirus, or at least one symptom associated with the infection and/or disease includes acute gastroenteritis and the associated symptoms (at least one of nausea, diarrhea, loose stool, vomiting, nauseous feeling, fever, malaise, fatigue, stomach cramps, chills, myalgia, and headache). Norovirus infections, or diseases or conditions resulting therefrom, are known to those skilled in the art, but it should be understood that they may not be limited to those listed here. The above prevention, treatment, alleviation, or amelioration may be accomplished by, but is not limited to, neutralizing the infectious agent, inhibiting entry of the infectious agent into the cell, inhibiting replication of the infectious agent, protecting host cells from infection or destruction, or stimulating antibody production. Therefore, the composition of the present invention can be a pharmaceutical composition for use in the treatment of norovirus.

The norovirus to which the composition of the present invention can be applied may be a norovirus belonging to GII, but GII. 4 can be excluded. Examples thereof include noroviruses belonging to at least one selected from the group consisting of GII. 1, GII.2, GII.3, GII.5, GII.6, GII.7, GII. 12, GII. 13, GII.14, GII.17, and GII.21.

The composition of the present invention can be administered to a subject in an amount effective to prevent, treat, alleviate, or ameliorate norovirus infection, disease induced by norovirus, or at least one symptom associated with the infection and/or disease. The subject can be any subject that can be infected with norovirus, and for example, can be a mammal (human, pig, cattle, rodent, dog, cat, etc.). The effective amount can be appropriately set in consideration of the subject to which the composition is applied, the route of administration, the form of administration, and the like.

The composition of the present invention can also be administered in combination with another protein and/or peptide, and the like. The other protein or peptide may be administered as an additional component blended in the composition of the present invention. Alternatively, the other protein or peptide may be administered at the same time or at a different time as the composition of the present invention, as a component blended in a separate composition. Furthermore, the other protein or peptide may be administered to a subject with the intent to prevent, treat, alleviate, or ameliorate norovirus infection, but may also be administered to a subject with the intent to prevent, treat, alleviate, or ameliorate another disease.

Of the compositions of the present invention, the composition comprising the peptide of the present invention or the polynucleotide encoding the peptide can be, for example, a vaccine composition. The vaccine composition can be used to induce protective immunity to a norovirus, and thereby prevent, treat, alleviate, or ameliorate norovirus infection, disease induced by norovirus, or at least one symptom associated with the infection and/or disease.

The norovirus here may be a norovirus belonging to any of genotypes of GII, but GII. 4 can be excluded. Examples thereof include noroviruses belonging to at least one selected from the group consisting of GII.1, GII.2, GII.3, GII.5, GII.6, GII.7, GII. 12, GII.13, GII.14, GII. 17, and GII.21.

Furthermore, induction of "protective immunity" to a norovirus means inducing immunity or an immune response to the infectious agent (norovirus, substances derived therefrom, or substances produced thereby). The protective immune response may result from either a humoral or cell-mediated immune response. When protective immunity to a norovirus is induced, the antibody titers against the norovirus can become higher in a subject compared to before the induction. Antibody titers can be measured by a known method. Examples thereof include a neutralizing activity test, as described in the examples.

The vaccine composition can be administered to a subject in an amount effective to induce protective immunity to a norovirus. The subject can be any subject that can be infected with norovirus, and for example, can be a mammal (human, pig, cattle, rodent, dog, cat, etc.). The effective amount to induce protective immunity can be appropriately set in consideration of the subject to which the vaccine composition is applied, the route of administration, the form of administration, and the like.

The vaccine composition can be a peptide vaccine comprising the peptide of the present invention. The peptide vaccine may comprise a single epitope or two or more different epitopes. Each of the epitopes in the peptide vaccine may be present in a free state, or with two or more linked together (multi-epitope peptide).

Alternatively, the vaccine composition can be a virus-like particle (VLP) vaccine. Examples thereof include a VLP vaccine in which the peptide of the present invention is expressed in VLPs. For example, VLPs of papillomavirus, human hepatitis B virus (HBV), norovirus, and the like can be used as VLPs, but are not limited thereto. Methods for preparing VLP vaccines are well known, and those skilled in the art can appropriately select the necessary materials, such as the VLPs, vectors for their expression, expression vectors, and hosts.

Alternatively, the vaccine composition can be a gene vaccine comprising the polynucleotide encoding the peptide of the present invention. Examples thereof include a recombinant vector vaccine comprising a vector into which the polynucleotide is incorporated.

Alternatively, the vaccine composition can comprise particles having the peptide of the present invention or the polynucleotide encoding the peptide. The peptide of the present invention or the polynucleotide encoding the peptide may be present on the surface of the particles or inside the particles. In addition, the peptide of the present invention or the polynucleotide encoding the peptide may be covalently or non-covalently bound to the particles, or may even be bound to the particles via a linker.

<Method for identifying neutralizing epitope>

The present invention provides a method for identifying a neutralizing epitope for a norovirus.

The method for identifying a neutralizing epitope of the present invention comprises a step of collecting genome sequences of one or more norovirus strains belonging to a specific genotype, and extracting gene sequences of a capsid protein VP1 (VP1 protein) from the genome sequence. The specific genotype may belong to any of GI, GII, GIII, GIV, GV, GVI, GVII, GVIII, GIX, and GX, but preferably belongs to GII. For example, it is possible to collect genome sequences from one or more norovirus strains belonging to a genogroup selected from the group consisting of GII.2, GII.3, GII.5, GII.6, GII.7, GII. 12, GII. 13, GII. 14, GII. 17, and GII.21, and extract gene sequences of the VP1 protein. In this step, the genome sequences are preferably collected from as many norovirus strains as possible to extract many VP1 protein gene sequences. Information on the genome sequences of the norovirus strains and the gene sequences of VP 1 proteins can be obtained from databases publicly available on the Internet and the like (including, but not limited to, GenBank, EMBL, and DDBJ (Japanese DNA database)).

Next is included a step of aligning the extracted gene sequences of the VP1 protein, and classifying the norovirus strains into one or more clusters based on the similarity of these gene sequences, the phylogenetic closeness of the strains from which these gene sequences are derived, or a combination thereof. For example, if there is similarity in the gene sequences and/or the strains from which the gene sequences are derived are phylogenetically close, the gene sequences can be classified into the same cluster. Then, any norovirus strain is selected from the classified cluster, and a three-dimensional structure of a P domain of the VP1 protein of the strain is predicted by homology modeling. This operation does not necessarily need to be performed for all of the norovirus strains classified in a cluster. This is because once the neutralizing epitope is predicted for some of the norovirus strains, e.g., about three to five strains, the neutralizing epitopes of other norovirus strains belonging to the same cluster can also be predicted by comparing amino acid sequences. Software for predicting three-dimensional structure is generally accessible or available. Examples thereof include Swiss-model.

The neutralizing epitope is then determined from the predicted three-dimensional structure of the P domain of the VP1 protein. The neutralizing epitope is determined by comparing the predicted three-dimensional structure with a three-dimensional structure of a P domain of a VP1 protein of a reference strain of norovirus. A portion of the predicted three-dimensional structure that structurally corresponds to a neutralizing epitope of the P domain of the reference strain is determined as the neutralizing epitope. Here, the reference strain of norovirus refers to a norovirus strain belonging to the same genogroup as the strain subject to the identification of the neutralizing epitope. For example, if the strain subject to the identification of the neutralizing epitope is a norovirus strain belonging to GII, the reference strain can be a norovirus strain belonging to GII (but different from the subject strain). To give a more specific example, if the subject strain is a norovirus strain belonging to GII.1, GII.2, GII.3, GII.5, GII.6, GII.7, GII.12, GII.13, GII.14, GII.17, or GII. 2 1, the reference strain can be a norovirus strain belonging to GII.4. In addition, the presence of a neutralizing epitope on the P domain of the VP1 protein is better to be known (previously confirmed) for the reference strain. The neutralizing epitope can be identified by a method of analyzing crystals of antigen-antibody conjugates by X-ray, epitope mapping, or other known methods. Alternatively, a norovirus strain for which the presence of a neutralizing epitope on the P domain of the VP1 protein has already been reported can also be used as the reference strain. For example, a GII.4 norovirus strain with an identified epitope on the P domain of the VP1 protein (Non Patent Literature 2 (Lindesmith LC, Baric RS: Immunity 50, 1530, 2019)) can be used as the reference strain.

Furthermore, an amino acid sequence of the P domain of the VP1 protein, which comprises the identified neutralizing epitope as described above, is compared with an amino acid sequence of a P domain of a VP1 protein from a different strain that belongs to the same genotype as the above-described strain from which the amino acid sequence is derived and whose three-dimensional structure was not compared above, to determine a portion in the amino acid sequence of the P domain of the VP1 protein of the different strain corresponding to the amino acid sequence of the neutralizing epitope, and identify that portion as the neutralizing epitope of the P domain of the VP1 protein of the different strain.

EXAMPLES

The following examples describe the present invention in more detail. The examples are provided for the purpose of better understanding the invention and are not intended to limit the scope of the invention.

[Example 1] Identification of neutralizing epitope for norovirus of GII.6 genotype

The neutralizing epitope for a GII.6 norovirus was identified as follows. From the NCBI (National Center for Biotechnology Information: www.ncbi.nlm.nih.gov) database, the data (99 data) that could be confirmed to be the gene sequences of the VP1 protein of noroviruses belonging to GII.6 based on the annotation information were collected and translated into amino acid sequences. Clustering of these amino acid sequences based on sequence homology revealed that they can be classified into three major clusters. From these three clusters, three strains were selected as representatives of each. The NCBI accession numbers for the genetic information of the three strains are AB078337, MH279838, and JX989075, respectively.

The three-dimensional structures of the P domain of the VP1 protein of these representative strains were predicted by homology modeling based on the amino acid sequences. The predicted three-dimensional structure of the P domain of the VP1 protein of the representative strains of GII.6 was compared by three-dimensional structure alignment with the three-dimensional structure of the P domain of the VP1 protein of GII.4 (reference strain) (for details, reference literature: Lindesmith LC, Baric RS: Immunity 50, 1530, 2019 (Non Patent Literature 2)), for which the neutralizing epitope is known. The identification of epitope D of AB078337 is shown as an example (Fig. 1). In this way, a predicted portion of the P domain of the representative strain of GII.6 that structurally corresponds to a neutralizing epitope of the P domain of the reference strain was identified.

The gene sequences of all of the GII.6 VP1 proteins obtained above were converted to amino acid sequences, and the amino acid sequences were compared by multiple sequence alignment. This allowed to calculate the degree of conservation of each amino acid residue of the VP1 proteins within GII.6 (between the same genotypes) to identify a region with low conservation (highly mutated) of the amino acid residues of the P domain of the VP1 protein.

The amino acid sequence identified as described above, comprising a predicted portion of the P domain of the GII.6 representative strain and the region of low amino acid conservation (high mutation) in the vicinity of that portion, was identified as the neutralizing epitope of the GII.6 representative strain (The neutralizing epitope of GII.6 is considered to have a three-dimensional structure similar to the neutralizing epitope of GII.4). In addition, neutralizing epitopes are generally prone to amino acid mutation to evade host immunity. Thus, the portion that meet these conditions can be presumed to be the neutralizing epitope.). Known neutralizing epitopes of GII.4 include A (divided into three regions, which are designated as the A1, A2, and A3 regions for convenience), D, E, and F (divided into two regions, which are designated as the F1 and F2 regions for convenience). The neutralizing epitopes identified in the corresponding GII.6 representative strain were confirmed to correspond to the following amino acid positions in the amino acid sequence of the VP1 protein, counting from the N-terminus.

- AB078337

    Neutralizing epitope A: positions 290 to 311 (A1 region) (SEQ ID NO: 51), positions 372 to 377 (A2 region) (SEQ ID NO: 135), positions 385 to 395 (A3 region) (SEQ ID NO: 218);
    Neutralizing epitope D: positions 407 to 411 (SEQ ID NO: 310);
    Neutralizing epitope E: positions 417 to 423 (SEQ ID NO: 371); and
    Neutralizing epitope F: positions 337 to 342 (F1 region) (SEQ ID NO: 403), positions 412 to 415 (F2 region) (SEQ ID NO: 424).

- MH279838

    Neutralizing epitope A: positions 290 to 311 (A1 region) (SEQ ID NO: 54), positions 369 to 374 (A2 region) (SEQ ID NO: 136), positions 382 to 392 (A3 region) (SEQ ID NO: 217);
    Neutralizing epitope D: positions 404 to 408 (SEQ ID NO: 312);
    Neutralizing epitope E: positions 414 to 420 (SEQ ID NO: 372); and
    Neutralizing epitope F: positions 337 to 342 (F1 region) (SEQ ID NO: 403), positions 409 to 412 (F2 region) (SEQ ID NO: 424).

- JX989075

Neutralizing epitope A: positions 290 to 311 (A1 region) (SEQ ID NO: 54), positions 369 to 374 (A2 region) (SEQ ID NO: 136), positions 382 to 392 (A3 region) (SEQ ID NO: 221);
Neutralizing epitope D: positions 404 to 408 (SEQ ID NO: 312);
Neutralizing epitope E: positions 414 to 420 (SEQ ID NO: 372); and
Neutralizing epitope F: positions 337 to 342 (F1 region) (SEQ ID NO: 403), positions 409 to 412 (F2 region) (SEQ ID NO: 424).

Furthermore, based on the results of the comparison of the amino acid sequences of the GII.6 VP1 proteins performed above by multiple sequence alignment, the amino acid sequences (i.e., neutralizing epitopes) of GII.6 other than the GII.6 representative strains (AB078337, MH279838, and JX989075) corresponding to the identified neutralizing epitopes of the representative strains were identified (Table 1).

[Example 2] Identification of additional neutralizing epitopes for norovirus

The neutralizing epitopes for GII.1, GII.2, GII.3, GII.5, GII.7, GII.12, GII.13, GII. 14, GII. 17, and GII.21 noroviruses were identified according to the method described in Example 1 (Table 1).

[Table 1-1]

Table 1. Neutralizing epitopes identified for norovirus GII

| Norovirus GII genotype | Epitope region of P domain of VP1 protein | Amino acid sequence | SEQ ID NO: | Accession No. |
|---|---|---|---|---|
| GII.1 | A1 | QVPDDHHQ | SEQ ID NO: 1 | GII.1-JX289822:JN797508:KC463911:KC464322:KC464323:MF668937:MK753033:MG572182:MK753034: MK483908:MK616585:MK616584 |
| GII.1 | A1 | RVPDDHHQ | SEQ ID NO: 2 | GII. 1-MH218731 |
| GII.2 | A1 | EVTAHLHDNEHL | SEQ ID NO: 3 | GII.2-MF405169:AB662861:AB662868:LC213890 |
| GII.2 | A1 | EVTAHLQDNDHL | SEQ ID NO: 4 | GII.2-JX846925:JN699037:MG746000:MG746127: MG746128:MG746129 |
| GII.2 | A1 | EVTAHLHDNDHL | SEQ ID NO: 5 | GII.2-AB281081:AB281082:AB281083:AB281084:AB281085:AB281086:AB281087:AB195225:AB281088:LC209464:LC209435:LC209436:LC209438:LC209437:DQ456824:AB662850:AB662851:AB662852:AB662853:AB281089:AB281090:LC209462:AB662854:AB662856:AB662863:AB662855:AB662858:AB662864:AB662865:LC209463:AB662859:AB662860:AB662866:AB662867:AB662869:AB535749:LC209461:AB662862:AB662870:AB662871:AB662872:AB662873:AB662874:AB662881:AB662882:AB662883:AB662884:AB662885:AB662886:LC209480:LC209481:LC209460:LC209465:LC209459:LC209472:LC209473:LC209474:LC209454:AB662875:AB662876:AB662877:AB662878:AB662879:AB662880:AB662887:AB662888:AB662889:AB662890:AB662891:AB662892:AB662893:AB662894:AB662895:AB662896:AB662897:AB662898:AB662899:AB662900:AB662901:AB662902:LC209451:LC209449:LC209479:LC209467:LC209452:LC209447:L |

C209453:LC209471:LC209448:KJ407074:KC464505:LC209468:LC209433:LC209445: LC209466:LC209478:LC209446:LC209432:LC145787:LC145786:LC145788:LC145789 :LC145790:LC145791:LC145792:LC145793:LC145794:LC145795:LC145796:LC14579 7:MK752949:MH938340:LC209442:LC209455:LC209476:LC209456:LC209444:LC2094 43:LC209475:LC209477:LC209431:MH218733:MK762626:MK775028:MH702265:MH702 261:MH702260:KY457721:KY457722:MH938341:LC209450:LC209439:LC209469:LC20 9441:LC209434:LC209470:LC209458:LC228948:LC145798:LC145799:LC145800:LC1 45801:LC145802:LC145803:LC145804:LC145805:LC145806:LC145807:LC145808:MH 218697:MH218655:MH218735:MH218737:MH218734:MH218657:MK729086:MK752945:M K752944:MK753011:KY457724:KY457725:LC209457:LC209440:LC213885:KT962983: MG745995:MG745996:MG745997:MG745998:MG745999:MG746001:MG746002:MG746003 :MG746004:MG746008:MG746009:MG746010:MG746013:MG746014:MG746015:MG74601 6:MG746017:MG746027:MG746028:MG746029:MG746033:MG746035:MG746036:MG7460 38:MG746040:MH671553:MK753015:MK789430:MK753007:MK753035:MK764040:MK764 042:KY905336:KY905337:KY905338:MK764039:MK753012:MK775029:KY865306:KY86 5307:MK773583:MK753013:MK764043:MK773581:MK762641:MK773580:MK773582:MK7 53014:MK753031:MK907802:KY457727:KY457734:KY457731:KY457729:KY457735:LC 413792:LC413793:LC413794:LC413791:MG763365:KY457728:KY457730:KY677827:K Y677828:KY677829:KY677830:KY677831:KY677832:KY677833:KY817742:KY817743: KY817744:KY817745:KY817746:KY817747:MH158635:MH671554:MH938342:MH938343 :MH938344:MH938345:MH938346:MH938347:MG746046:MG746047:MG746048:MG74604 9:MG746050:MG746051:MG746052:MG746053:MG746054:MG746055:MG746057:MG7460 58:MG746059:MG746060:MG746061:MG746062:MG746063:MG746064:MG746065:MG746 066:MG746067:MG746068:MG746069:MG746070:MG746071:MG746072:MG746073:MG74 6074:MG746075:MG746076:MG746077:MG746078:MG746079:MG746080:MG746081:MG7 46082:MG746083:MG746084:MG746085:MG746086:MG746087:MG746088:MG746089:MG

[Table 1-2]

[Table 1-3]

746090:MG746091:MG746092:MG746093:MG746094:MG746095:MG746096:MG746097:MG746098:MG746099:MG746100:MG746101:MG746102:MG746103:MG746104:MG746105:MG746106:MG746107:MG746108:MG746121:MG746122:MG746123:MG746124:MG746125:MG746126:MG746133:MG746134:MG746135:MG746136:MG746137:MG746138:MG746139:MG746140:MG746141:MG746147:MG746148:MG746149:MG746150:MG746167:KY421121:KY421122:NC:LC349981:LC349982:LC349983:LC279234:LC279235:LC279236:LC279237:LC279239:LC279240:LC279241:LC279242:LC279243:LC279238:LC213886:LC213887:LC213888:LC213889:LC213891:LC213892:LC213893:LC213894:LC213895:LC213896:LC213897:LC213898:LC213899:LC213900:LC213901:LC215413:LC215414:LC215415:KY421123:KY421124:KY421125:KY421126:KY421127:KY421128:KY421129:KY421130:KY421131:KY421132:KY421133:KY421134:KY421135:KY421136:KY421137:KY421138:KY421139:KY421140:KY421141:KY421142:KY421143:KY421144:KY421145:KY421146:KY421147:KY421148:KY421149:KY421150:KY421151:KY421152:KY421153:KY421154:KY421155:KY421156:KY421157:LC325213:LC325214:LC325215:LC325216:KY421044:KY485115:KY485116:KY485117:KY485118:KY485119:KY485120:KY485121:KY485122:KY485123:KY485124:KY485125:KY485126:KY407217:KY407218:KY407219:KY407220:KY407221:KY457580:KY457581:KY457582:KY457583:KY457584:KY457585:KY457586:MG892974:MG745985:MG745986:MG745987:MG745988:MG745989:MG745990:MG745991:MG745992:MG745993:MG745994:MG746005:MG746006:MG746007:MG746011:MG746012:MG746018:MG746019:MG746020:MG746021:MG746022:MG746023:MG746024:MG746025:MG746026:MG746030:MG746031:MG746032:MG746034:MG746039:MG746041:MG746042:MG746043:MG746044:MG746045:MH321825:MN493873:MK614124:MK614125:MK614126:MK614127:MK614128:MK614129:MH041321:MH041322:MK762625:MK762631:MK753587:MK753016:MK762633:MK752941:MK752939:MK590974:MK762634:MK753021:MK590972:MK764017:MK752940:MK775030:MK340748:MK590973:MK762625... :MK614142:LC413795:LC413796:LC413797:LC413798:LC413799:LC413800:MG763354:

[Table 1-4]

MG763355:MG763356:MG763357:MG763358:MG763359:MG763360:MG763361:MG763362:MG763363:MG763364:MG763366:MG763367:MG763368:MG763369:MG763370:MG763371:MG763372:MG763373:MG763374:MG763375:MG763376:MG763377:KY817748:KY817749:KY817750:KY817751:KY817752:KY817753:KY817754:MH068791:MH068792:MH068793:MH068794:MH068795:MH068796:MH068797:MH068798:MH068799:MH068800:MH068801:MH068802:MH068803:MH068804:MH068805:MH068806:MH068807:MH068808:MH068810:MH068811:MH068812:MH068813:MH068814:MH068815:MH068816:MH068817:MH068818:MH068819:MH068820:MH938348:MH938349:MH938350:MH938351:MH938352:MH938353:MG746109:MG746110:MG746111:MG746112:MG746113:MG746114:MG746115:MG746116:MG746117:MG746118:MG746119:MG746120:MG746130:MG746131:MG746132:MG746142:MG746143:MG746144:MG746145:MG746146:MG746151:MG746152:MG746153:MG746154:MG746155:MG746156:MG746157:MG746158:MG746159:MG746160:MG746161:MG746162:MG746163:MG746164:MG746165:MG746166:MG746168:MG746169:MG746170:MG746171:MG746172:MG746173:MG746174:MG746175:MG746176:MG746177:MG746178:MG746179:MG746180:MG746181:MG746182:MG746183:MG746184:MG746185:MG746186:MG746187:MG746188:MG746189:MG746190:MG746191:MG746192:MG746193:MG746194:MG746195:MG746196:MG746197:MG746198:MG746199:MG746200:MG746201:MG746202:MG746203:MG746204:MG746205:MG746206:MG746207:MG746208:MG746209:MG746210:MG746211:MG746212:MG746213:MG746214:MG746215:MG746216:MG746217:MG746218:MG746219:MG746220:MG746221:MG746222:MG746223:MG746224:MG746225:MK614143:MK614144:MK614145:MK614146:MK614147:MK614148:MK614149:MK614150:MK614151:MK614152:MK614153:MK614154:MK614156:MK614159:MK614161:LC349984:LC349985:MK752935:MK614130:MK614131:MK614132:MK614133:MK614134:MK614135:MK614136:MK614137:MK762628:MK773571:MK614139:MK614140:MK614141:LC413801:LC413802:LC413803:LC413804:MK614155:MK614157:MK614158:MK614160:MN394542:MN394544

[Table 1-5]

| | | | | |
|---|---|---|---|---|
| GII.2 | A1 | QVTAHLQDNEHL | SEQ ID NO: 6 | GII.2-JQ320072:KC998960 |
| GII.2 | A1 | EVTAHLHDDDHL | SEQ ID NO: 7 | GII.2-MH218648 |
| GII.2 | A1 | EVTAHLHDNGHL | SEQ ID NO: 8 | GII.2-MH218736 |
| GII.2 | A1 | KVTAHLHDNDHL | SEQ ID NO: 9 | GII.2-MH938338:MH938339 |
| GII.2 | A1 | EVTTHLHDNDHL | SEQ ID NO: 10 | GII.2-MG746037: MG746056: MH068809 |
| GII.2 | A1 | EVTAHLRDDEHL | SEQ ID NO: 11 | GII.2-MH979229:MK864096 |
| GII.3 | A1 | TLTRSTSRASDQADTATPRLFNYY | SEQ ID NO: 12 | GII.3-KY442319:KY442320:KC597144:JN699040:JX846924:JN699039:HM072041:KF944165:KF944166:KF306213:KJ499443:MH218585:MK907787:MH218598:MH218603:KY767665 |
| GII.3 | A1 | TLTRSTSRAGDQADTATPRLFNYY | SEQ ID NO: 13 | GII.3-HM072045:HM072046 |
| GII.3 | A1 | TLTRSTSRTGDQADTASPRLFNYY | SEQ ID NO: 14 | GII.3-HM072042 |
| GII.3 | A1 | TLTRSTNRVSDQADTATPRLFNYH | SEQ ID NO: 15 | GII.3-HM072043 |
| GII.3 | A1 | TLTRSTNRASDQADTATPRLFNHH | SEQ ID NO: 16 | GII.3-HM072044 |
| GII.3 | A1 | TLIRSTSRASDQADTSTPRLFNYY | SEQ ID NO: 17 | GII.3-HM072040 |
| GII.3 | A1 | TLTRSTSRASDQADTPTPRLFNHR | SEQ ID NO: 18 | GII.3-AB067542:JQ743333 |
| GII.3 | A1 | TLTRPTNRASDQADTATPRLFNHQ | SEQ ID NO: 19 | GII.3-DQ093063 |
| GII.3 | A1 | VLTRSTSRASDQADTATPRLFNYY | SEQ ID NO: 20 | GII.3-KC464324:GU292851:JN899244:AB365435:AB385634:HM590538:GU980585:AB385626:AB385641:AB385642:KC464326:KF931267:KC464327:MK907798:KC464328:KP064097:GU138208:KF931324:AB758450:KF944065:KC464495:KC464329:KJ184256:KF944203:KF944119:KF944147:KF944266:MG892076:KJ634708:MH702267:MK396777:KY348697:KJ499442:KY407172:KY407173:KY407174:KY407175:MH218570:MH218738:MH218712:MH218572:MH218573:MH218574:MH218576:MH218577:MH218584:MH218586:MH218587:MH218590:MH218732:MH218592:MK764020:MK762640:LC133339:LC133343:KJ499444:MH218594:MH218597:MH218599:MH218600:MH218601:MH218604:MH218660:MH218668:MH218671:MH218672:MH218675:MH218676:MH218677:MH218678:MH2 |

[Table 1-6]

18679：MH218680：MH218630：KT732274：KY767664：KY406923：KY406924：KY406925：KY 406926：KY406927：KY406929：KY406930：KY406931：KY406932：KY406933：KY406934：K Y406937：KY406938：KY406939：KY407176：KY407177：KY407178：KY407179：KY407180： KY407190：KY407191：KY407192：KY407193：KY407194：KY887597：KY887606：MH218681 ：MH218682：MH218683：MH218686：MH218688：MH218690：MH218693：KY887598：KY90533 4：KY210918：KY210919：MG892915：MG892911：MG892910：MK073886：KX989464：KX9894 65：KX989466：KX989467：KX989468：KY406928：KY406935：KY406936：KY406941：KY406 942：KY406943：KY406944：KY406945：KY406946：KY406947：KY407195：KY407196：KY40 7197：KY407198：MG892953：MG892955：MG892952：MG892947：MG892951：MG892949：MG8 92950：MG892956：MK773588：MH279487

| | | | |
|---|---|---|---|
| GII. 3 | A1 | VLTRSTSRTSDQADTATPRLFNYY SEQ ID NO: 21 | GII. 3-KF006265:KJ499441:MN199033:KY348698:KJ499445 |
| GII. 3 | A1 | VLTRSTSRASDQADTVTPRLFNYYSEQ ID NO: 22 | GII. 3-KC464325:MH218583 |
| GII. 3 | A1 | VLTRSTSRASDQADAATPRLFNYYSEQ ID NO: 23 | GII. 3-KF931180:KF944111:KF944110:MH702287:MK396772:MH702259 |
| GII. 3 | A1 | VLTRSTSRASDQADTATPRLFDYYSEQ ID NO: 24 | GII. 3-AB385627:MH218571:MH218580 |
| GII. 3 | A1 | VLTRSTSRASDHADTATPRLFNYYSEQ ID NO: 25 | GII. 3-KF931243:KF931234:KF895841 |
| GII. 3 | A1 | VLTRSTSRASDQADTAAPRLFNYYSEQ ID NO: 26 | GII. 3-LC101823 |
| GII. 3 | A1 | VMTRSTSRASDQADTAIPRLFNYYSEQ ID NO: 27 | GII. 3-KJ184223 |
| GII. 3 | A1 | VLTRSTSRASDQADTATPRLFNYH SEQ ID NO: 28 | GII. 3-JN565063:MH218578:MH218579 |
| GII. 3 | A1 | MLTRSTSRASDQADTATPRLFNYYSEQ ID NO: 29 | GII. 3-JX984948:KX355506 |
| GII. 3 | A1 | VLTRSTSRASDQADTAIPRLFNYY SEQ ID NO: 30 | GII. 3-KF944179:MG892920:KY406940:MG892946:MG892954 |
| GII. 3 | A1 | VLTQRTSRASDQADTAVTPRAFDHR SEQ ID NO: 31 | GII. 3-KC597140 |
| GII. 3 | A1 | VLTRSTSRASDQADAATPRLFNYHSEQ ID NO: 32 | GII. 3-KT779557 |
| GII. 3 | A1 | VLTRSTSRASDQSETTTPRLFNYYSEQ ID NO: 33 | GII. 3-KF895859 |
| GII. 3 | A1 | VLTRSTSRASDQADTTTPRLFNYYSEQ ID NO: 34 | GII. 3-KF944227:KF944232:KF895848:MH218593 |
| GII. 3 | A1 | VLIRSTSRASDQADTATPRLFNYY SEQ ID NO: 35 | GII. 3-KM056394:KY406922 |

[Table 1-7]

| | | | | |
|---|---|---|---|---|
| GII.3 | A1 | VLTRSTSRASDQADAATPRLFNHY | ID NO: 36 | GII.3-MH218575 |
| GII.3 | A1 | VLTRSTSRASDQADIATPRLFNYY | ID NO: 37 | GII. 3-MH218581 :MH218582:MH218588 |
| GII.3 | A1 | VLTRSTSRASDQADTVTPRLFNHY | ID NO: 38 | GII. 3-MH218589 |
| GII.3 | A1 | VLTRSTSRANDQADTATPRFFDHS | SEQ ID NO: 39 | GII.3-MH218595:MH218596 |
| GII.3 | A1 | VLTRSTSRANDQADTATPRHFDLS | ID NO: 40 | GII. 3-MH218602:MH218653 |
| GII.3 | A1 | VLIGSTSRASDQADTATPRSFNYY | ID NO: 41 | GII. 3-MH218618 |
| GII.3 | A1 | VLTRSTSRATDQADTATPRHFDHS | ID NO: 42 | GII. 3-MH218654 |
| GII.3 | A1 | VLTGSTSRASDQADTATPRLFNYY | ID NO: 43 | GII. 3-MH218696 |
| GII.3 | A1 | VLIGSTSRASDQADTATPRLFNYY | ID NO: 44 | GII. 3-MH218717 |
| GII.3 | A1 | VLTRSTSRANDQADTATPRHFGLS | ID NO: 45 | GI I. 3-MH218646 |
| GII.5 | A1 | KVTGQVPNEQHM | SEQ ID NO: 46 | GII.5-JN699044:KJ196277:KJ196288:KM386680:KM386681:KM036379:KM036378:KU311160 |
| GII.5 | A1 | RITGQVPNEQHM | SEQ ID NO: 47 | GII. 5-HM596590 |
| GII.6 | A1 | IGQTSRSSDSTDSAPRRRDHPL | SEQ ID NO: 48 | GII. 6‑KY424345：KY424346：KY424347：KY424348：GU969054：GU969055：GU969057：HM633213：AB682736：AB818397：AB818398：AB818399：AB758451：AB685739：AB685740：KJ407072：AB818400：MH218642：MH218645：MH218666：MH218673：MK956198：MK956199：MK956197 |
| GII.6 | A1 | ISQTARAADSTDSPQRARDHPL | SEQ ID NO: 49 | GII.6-KC576910:JN699035:JN699036:KP064098 |
| GII.6 | A1 | ISQTSRSAESTDSAPRVRNHPL | SEQ ID NO: 50 | GII. 6-JN699041 |
| GII.6 | A1 | ISQTARAADSTDSPQRARNHPL | SEQ ID NO: 51 | GII. 6-AB078337 |
| GII.6 | A1 | ISQTARATDSTDSPQRARDHPL | SEQ ID NO: 52 | GII. 6-DQ093064 |
| GII.6 | A1 | ISQTARAADSVDSPQRARDHPL | SEQ ID NO: 53 | GII.6-AB818404 |

| GII.6 | A1 | ISQTSRSADSTDSAPRVRNHPL | SEQ ID NO: 54 | GII.6-AB818403:AB818402:AB685742:AB818401:AB685741:JX984945:JX989075:JX 984953:KY424341:KY424342:KY424343:KY424344:MH218639:MK907789:MK907786:M H279837:MH279838:KM036373:KM036375:LN854568:MH218641:MH218650:MH218719: |

[Table 1-8]

MH279827：MH279839：MH279835：MH279828：MG571778：MH218687：MH702284：MH702286
：KY406919：KY406920

| GII.6 | A1 | VSQTSRSADSTDSAPRVRNHPL | SEQ ID NO: 55 | GII.6-HQ169542 |
|---|---|---|---|---|
| GII.6 | A1 | ISQAARAADSTDSPQRARDHPL | SEQ ID NO: 56 | GII.6-JN183165 |
| GII.6 | A1 | ISQTARAVDSTDSPQRARDHPL | SEQ ID NO: 57 | GII.6-AB685738:KX752057 |
| GII.6 | A1 | IGQISRSSDSTDSAPRRRDHPL | SEQ ID NO: 58 | GII.6-GU969056 |
| GII.6 | A1 | ISQTSRSADSTDSAPRVRNHPP | SEQ ID NO: 59 | GII.6-JX984949 |
| GII.6 | A1 | ISQNARAADSPDSPQRARNHPL | SEQ ID NO: 60 | GII.6-MH279832 |
| GII.6 | A1 | ISQNARAADLTDSPQRARNHPL | SEQ ID NO: 61 | GII.6-KM461694:KM461693:MH114014 |
| GII.6 | A1 | ISQNARAADSTDSPQRARNHPL | SEQ ID NO: 62 | GII.6-MH279834:MH279831 |
| GII.6 | A1 | IGQTSRSPDSTDSAPRRRDHPL | SEQ ID NO: 63 | GII.6-KC464321 |
| GII.6 | A1 | ISQTSRSADSIDSAPRVRNHPL | SEQ ID NO: 64 | GII.6-MH702288 |
| GII.6 | A1 | ISQASRSADSTDSAPRVRNHPL | SEQ ID NO: 65 | GII.6-KM036374 |
| GII.6 | A1 | ISQTSRSADSTDSAPRARNHPL | SEQ ID NO: 66 | GII.6-KX268709:KU935739:KY406918:KY406921 |
| GII. 6 | A1 | ISQTSRSVDSTDSAPRARNHPL | SEQ ID NO: 67 | GII.6-LC133342:LC133338 |
| GII. 6 | A1 | ISQTSRSADSTDSAQRVRNHPL | SEQ ID NO: 68 | GII.6-MH218640:MH218644 |
| GII. 6 | A1 | ISQTSRSADSTDSAQRVRDHPL | SEQ ID NO: 69 | GII.6-MH218643 |
| GII.6 | A1 | VSQTSRSADSTDSASRVRNHPL | SEQ ID NO: 70 | GII.6-MN248516:MN248517:MN248514 |
| GII.6 | A1 | IGQTSRTSDSTDSAPRRRDHPL | SEQ ID NO: 71 | GI I. 6-MH218667 |
| GII.6 | A1 | VSQTSRSADSTDSAPRVRSHPL | SEQ ID NO: 72 | GII.6-KY407213:KY407214:KY407215:KY407216 |
| GII.6 | A1 | ISQTSRSSDSTDSAPRRRDHPL | SEQ ID NO: 73 | GII.6-MK301293 |
| GII.7 | A1 | GDVRSYR | SEQ ID NO: 74 | GII. 7-JN699042：KC832474：GQ849129：MH279833：MH279830：GQ849130：GU134965：KJ 196295：MH279829： MH218658:MH218661:MH218692:MN038126 |
| GII.7 | A1 | RDDRSYR | SEQ ID NO: 75 | GII.7-KF006266 |

[Table 1-9]

| | | | | |
|---|---|---|---|---|
| GII.12 | A1 | INQKVSGENHV | SEQ ID NO: 76 | GII.12-LC342059:KJ196294:KJ196282:KJ196299:GQ845370:JQ613568:HQ688986:HQ449728:HQ664990:KC464498:KC464496:KC464500:KC464499:KC464497:HQ401025:MK754445:MK762627:MK753036:MK754447:MK764041:MK616561:MK616560:MK355712:MK355713 |
| GII.12 | A1 | VNQKVSGENHV | SEQ ID NO: 77 | GII.12-KF006267 |
| GII.12 | A1 | INQKVSGDNHV | SEQ ID NO: 78 | GII.12-JQ613569:KP064099 |
| GII.12 | A1 | INQKVNGENHV | SEQ ID NO: 79 | GII.12-MK616558:MK616559 |
| GII.13 | A1 | VANNGDNWDQNL | SEQ ID NO: 80 | GII.13-KJ196276 |
| GII.13 | A1 | LANNDSWDQNL | SEQ ID NO:81 | GII.13-AB809973:AB809974 |
| GII.13 | A1 | VANSGDNWDQNL | SEQ ID NO: 82 | GII.13-AB809993:AB809997:AB809990:AB809975:AB809976:AB809994:AB809977:AB809992:AB809985:AB809996:AB809987:AB809978:AB809988:AB809979:AB809986:AB809991:AB810001:AB810014:MK753009:MK753008:MH702276:MH702277:KM036380:MK753010:MK754443:MK762560:MK752947:MK753020:MK762559:MH702263:MH702283:MH218651:MK752946:MK762745:MK764014:KY406982:KY406983:KY406984:KY947548:MK762565:MK396778:KY406985:KY406986:MG892908:MK775031:MN394543:MN394545 |
| GII.13 | A1 | VANSGDNWDQNV | SEQ ID NO: 83 | GII.13-JN899242 |
| GII.13 | A1 | VANRGDNWDQNL | SEQ ID NO: 84 | GII.13-AB810004:AB810006:AB810011:AB810003 |
| GII.13 | A1 | VANHGDNWDQNL | SEQ ID NO: 85 | GII.13-KY210920 |
| GII.14 | A1 | ELSDQPRYQ | SEQ ID NO: 86 | GII.14-JN699038 |
| GII.14 | A1 | KLNEEPR | SEQ ID NO: 87 | GII.14-KJ196278:KJ196297:GU017900:GU017903:GU594162:GU017901:GU017902:GU017904:GU017905:GU017906:GU017907:GU017908:MH279826:MK588004:MK641589 |
| GII.14 | A1 | KLSEEPR | SEQ ID NO: 88 | GII.14-MK850443:LC556388: LC556389: LC556391: LC556390: LC556392 |

[Table 1-10]

| | | | | |
|---|---|---|---|---|
| GII.17 | A1 | KLTADVHQSHDDR | SEQ ID NO: 89 | GII. 17-KC597139:JN699043 |
| GII.17 | A1 | RLTADVDGSHDDR | SEQ ID NO: 90 | GII. 17-KJ196286 |
| GII.17 | A1 | KISADVQNSHQDR | SEQ ID NO: 91 | GII. 17-DQ438972 |
| GII.17 | A1 | RVTAQINQRDR | SEQ ID NO: 92 | GII.17-MK907801:MK907800:KU557801:KP998539:KT380915:KT780394:KT780395:KT780396:KT780397:KT780398:KT780399:KU561252:KU561253:KR083017:KY424349:KY424350:KR020503:KT326180:KU557790:KU557785:KU557786:KU557787:KP698928:KP698929:KP902566:KP902567:KP902568:KP902569:KP902570:KP902571:KP902572:KP902573:KP902574:KP902576:KP902577:KP902578:KT315668:KT315669:KT315670:KT315671:KT315672:KT315673:KP864103:KP864104:KU561224:KU561225:KU561226:KU561227:KU561228:KU561229:KU557797:KU557798:KU557799:KU557802:KU557803:KU557806:KU557807:KU557808:KU557809:KU557810:KU557811:KU557815:KU557816:KU557817:KU557822:KU557823:KU557824:KU557827:KU557828:KU557829:KU557833:KU557834:KU557835:KU557846:KU557847:KU557848:KU557849:KU557885:KY069114:KX346699:KP902575:LC369228:LC369214:MG692610:LC369229:LC369230:KT970369:KT970370:MK907791:MK396775:MK907792:MK907793:LC433721:KT149168:KT149169:MK077685:MK077706:MF918359:KT780400:KT780401:KT780402:KT780403:KT780404:KT780405:KT780406:KT780407:KT780408:KT780409:KT780410:KT780411:KT780412:KT780413:KT780414:KT780415:KT780416:KU561248:KU561249:KT253245:KU561254:KU561255:KU561256:KX356908:NC:LC037415:KU557783:KU557784:KR154230:KR154231:KT992790:KT326181:KT326182:KT992789:KT992786:KT992787:KT992785:KT992788:KY392867:KY392868:KR052021:KR052019:KR052020:KR052022:LC349987:LC349988:LC349992:KT346356:KX024652:LC486739:LC486740:LC486741:LC486747:LC486751:LC486761:LC486762:LC486763:LC148844:LC148845:LC148846:LC148847:LC148848:LC148849:LC148850:LC148851:LC101820:LC177662:KX171414:KX420892:KX420891:KP698930:KP698931:KP902579:KP902580:KP902581:KP902582:KP902583:KP902584:KP902585:KP902586:KP902587:KP902588:KP902589:KP9 |

[Table 1-11]

02590:KT315674:KT315675:KT315676:KT315677:KT315678:KT315679:KT315680:KT
315681:KT315682:KT315683:KT315684:KT315685:KT315686:KT315687:KT315688:K
T315689:KT315690:KT315691:KT315692:KT315693:KT315694:KT315695:KT315696:
KT315697:KT315698:KT315699:KT315700:KT315701:KT315702:KT315703:KT315704
:KT315705:KT315706:KT315707:KT315708:KT315709:KT315710:KT315711:KT31571
2:KT315713:KT315714:KT315715:KT315716:KT315717:KT315718:KT315719:KP8641
02:KT591501:KU953391:KU953392:KU953393:KU561230:KU561231:KU561232:KU561
233:KU561234:KU561236:KU561237:KU561238:KU561239:KU561240:KU561241:KU56
1242:KU561243:KU561244:KU561245:KU561246:KU561247:KU557800:KU557804:KU5
57805:KU557812:KU557813:KU557814:KU557818:KU557819:KU557820:KU557821:KU
557825:KU557826:KU557830:KU557831:KU557832:KU557836:KU557837:KU557838:K
U557840:KU557841:KU557842:KU557843:KU557844:KU557845:KU557850:KU557851:
KU557852:KU557853:KU557854:KU557855:KU557856:KU557857:KU557858:KU557859
:KU557860:KU557861:KU557862:KU557863:KU557864:KU557865:KU557866:KU55786
7:KU557868:KU557869:KU557870:KU557871:KU557872:KU557873:KU557874:KU5578
75:KU557876:KU557877:KU557878:KU557879:KU557880:KU557881:KU557882:KU557
883:KU557884:KU557886:KU557887:KU557888:KU557889:KU557890:KU557891:KU55
7892:KU557893:KX244850:KX244851:KX244852:KX244853:KU587626:KU587627:KU5
87628:KU587629:KX134669:KX134670:KX168437:KX168438:KX371107:KX371108:KX
371109:KY397953:KY397954:KY397955:KY397956:KY397957:KY397958:KX346700:K
X346701:KX346702:KX346703:KX346704:KX346705:MF172092:MF172093:MF172094:
KY406954:KY406955:KY406956:KY406957:KY406958:KY406959:KY406960:KY407181
:KY407182:KY407183:KY407184:KY407185:KY407186:LC369234:LC369237:LC369923
3:LC369257:LC369242:LC369255:LC369235:LC369222:LC369215:LC369227:LC3692
19:LC369236:LC369244:LC369220:LC369232:LC369238:MN853414:LC369245:LC369
256:LC369224:LC369240:LC369241:LC369231:MH997861:LC369243:LC369225:LC36

[Table 1-12]

9239:LC369249:LC369246:LC369247:LC369248:LC369254:LC369253:LC369252:MK7
89431:LC369218:LC369221:LC369216:LC369217:KT970371:KT970372:KT970373:KT
970374:KT970375:KT970376:KT970377:MH747479:MH747481:MH890538:MH747480:L
C369258:MH747482:MH890539:LC433696:LC433698:LC433699:LC433709:LC433710:
LC433711:LC433712:LC433713:LC433722:LC433723:LC433724:LC433726:LC433727
:LC433729:LC433730:LC433731:LC433732:KX424646:KX424647:KX424648:KX42464
9:KX424650:KT149170:KT149171:KT149172:KT149173:KT149174:KT149175:KT1491
76:KX216782:KX216783:KX216784:KX216785:KX216786:KX216788:KX216789:KX216
790:KX216791:KX216792:KX216793:KX216794:KX216795:KX216796:KX216797:KX21
6798:KX216799:KX216800:KX216801:KX216802:KX216803:KX216804:KX216805:KX2
16806:MF073239:MF073240:MF073241:MK077686:MK077687:MK077688:MK077689:MK
077690:MK077691:MK077701:MK077702:MK077703:MK077704:MK077705:MK077708:L
C349986:LC349989:LC349990:LC349991:LC349993:LC486742:LC486746:LC486748:
LC486749:LC486750:LC486752:LC486753:LC486754:LC486764:LC486765:LC486766
:LC148852:LC148853:LC148854:LC148855:LC148856:KX420895:KX420894:KX42089
3:KU953394:KU953395:KU953396:KU953397:KU953398:KX244854:KX134671:KY0691
15:KX168439:KX168440:KX168441:KX168442:KX168443:KX168444:KX168445:KX168
446:KX168447:KX168448:KX168449:KX168450:KX168451:KX168452:KX168453:KX16
8454:KX168455:KX168456:KX371111:KX371112:MF172095:MF172096:KX989474:KX9
89475:KX989476:KX989477:KX989478:KY406961:KY406962:KY406963:KY406964:KY
406965:KY406966:KY406967:KY406968:KY406969:KY406970:KY406971:KY406972:K
Y406973:KY406975:KY406976:KY406977:KY406978:KY406979:KY407203:KY407204:
KY407205:KY407206:KU555841:KY905330:MH746922:MH746923:MH746924:MH746925
:LC318746:LC318745:LC318747:LC318748:LC333866:LC333867:LC333868:LC33386
9:LC333870:LC333871:LC333872:LC333873:LC333874:LC333875:LC333876:LC3338
77:LC333878:LC333879:LC333880:LC333881:LC333883:LC333884:LC333885:LC433

[Table 1-13]

716:LC433718:LC433719:LC433733:LC433734:LC433735:LC433736:MF073242:MF07
3243:MF073244:MF073245:MF073246:MF073247:MF073248:MF073249:MF073250:MFO
73251:MF073252:MF073253:MF073254:MH375801:MH375802:MH375803:MH375804:MH
375805:MH572223:MH572224:MH572225:MH572226:MH572227:MH572228:MH572229:M
H572230:MK077707:MK077709:MK077710:MK077712:MK077713:LC318750:MF421538:
MF421539:MF421540:MF421541:MF421542:LC258403:LC311767:LC311768:LC311769
:LC311770:LC311771:LC311772:LC311773:LC486743:LC486744:LC486745:LC48675
5:LC486756:LC486757:LC486758:LC486759:LC486760:LC486767:LC486768:LC4867
69:LC486770:MK789432:MK789433:LC318753:LC318754:LC318752:LC333886:LC333
887:LC333888:LC333889:LC333890:LC333891:LC333892:LC333893:LC333894:LC33
3896:LC333897:LC333899:LC333900:LC333901:LC318755:LC318756:LC318757:LC3
18758:LC333895:MH375806:MH375808:MH572232:MK077692:MK077693:MK077694:MK
077696:MK077697:MK077711:MK077714:MH375813:MK077695:MK077698:MK077699:M
K077700:MN394546

| GII.17 | A1 | RVTAETDHRDK | SEQ ID NO: 93 | GII.17-LC433694:KU561250:KU561251:LC043168:LC043167:KU557788:KJ156329:LC486737:KU557839:MK282256:MK282258:LC433720:MK077684:LC043139:LC043305:LC486738:KT285173:KP902563:KP902564:KP902565:LC433695:AB983218:KX171419:KX171418:KX171416:LC369251:KY905332 |
| GII.17 | A1 | RVTAKTDHRDK | SEQ ID NO: 94 | GII.17-MK282257 |
| GII.17 | A1 | RVTTETDHRDK | SEQ ID NO: 95 | GII.17-KX171413 |
| GII.17 | A1 | RVTAETDHHDK | SEQ ID NO: 96 | GII.17-KX171412 |
| GII.17 | A1 | RVTAKTDHSDK | SEQ ID NO: 97 | GII.17-KX171417 |
| GII.17 | A1 | RVTAETDNPDK | SEQ ID NO: 98 | GII.17-KU587625:MK907790 |
| GII.17 | A1 | KITADVRPSHDDR | SEQ ID NO: 99 | GII.17-MH218591 |
| GII.17 | A1 | KITADVRPNHDDR | SEQ ID NO: 100 | GII.17-KT589391 : KT346358: KY406980: KY406981 : MH375799:LC333898:MH375809:M |

[Table 1-14]
H375810:MH375811:MH375814

| | | | | |
|---|---|---|---|---|
| GII.17 | A1 | RVTAETDHRDR | SEQ ID NO: 101 | GII.17-KX171415 |
| GII.17 | A1 | RVTAQINQRDK | SEQ ID NO: 102 | GII.17-KU561235 |
| GII.17 | A1 | RVTAQINQHDR | SEQ ID NO: 103 | GII.17-KX371110:MH572231 |
| GII.17 | A1 | RVTAQINQSDR | SEQ ID NO: 104 | GII.17-KY406974 |
| GII.17 | A1 | RVTAEPSRSDR | SEQ ID NO: 105 | GII.17-MH218689 |
| GII.17 | A1 | RVTAQINQCDR | SEQ ID NO: 106 | GII.17-LC333882 |
| GII.17 | A1 | RVTAEVDQRDK | SEQ ID NO: 107 | GII.17-MG995040 |
| GII.21 | A1 | ITSNPTSDYWDG | SEQ ID NO: 108 | GII.21-GU138162:GU138163:GU138176:GU138177 |
| GII.21 | A1 | TTSNDTSDYWDD | SEQ ID NO: 109 | GII.21-HM590541 |
| GII.21 | A1 | MTSNPTSDYWDG | SEQ ID NO: 110 | GII.21-HM590519 |
| GII.21 | A1 | MTSNPTSDYWDD | SEQ ID NO: 111 | GII.21-KJ196284:JN899245 |
| GII.21 | A1 | TTSNPTSDYWDD | SEQ ID NO: 112 | GII.21-MH702268:MH702279:MH702281:MH702280:MH702278:MH702282:MH702264:MK396773:KR921935:KR921936:KR921937:MH702285:KX079488:KT962982:KR921938:KR921939:KR921940:KR921941:KR921942:KY406949:KY406950:KY210925:KY406951:KY406952:KY406953:KY407199:KY407200:KY407201:KY407202 |
| GII.21 | A1 | STSNPTSDYWDD | SEQ ID NO: 113 | GII.21-MK396771 |
| GII.1 | A2 | WSPKFT | SEQ ID NO: 114 | GII.1-JX289822:JN797508:KC463911:KC464322:KC464323:MH218731:MF668937:MK753033:MG572182:MK753034: MK483908:MK616585:MK616584 |
| GII.2 | A2 | PTYTAQ | SEQ ID NO: 115 | GII.2-MF405169:JX846925:JN699037:AB281081:AB281082:AB281083:AB281084:AB281085:JQ320072:KC998960:AB281086:AB281087:AB195225:AB281088:LC209435:AB662850:AB662851:AB281089:AB281090:LC209462:AB662856:AB662863:AB662855:AB662858:AB662864:AB662865:LC209463:AB662859:AB662860:AB662861:AB662866:AB662867:AB662868:AB662869:AB535749:LC209461:AB662862:AB662870:AB66287 |

[Table 1-15]

```
1:AB662872:AB662873:AB662874:AB662881:AB662882:AB662883:AB662884:AB6628
85:AB662886:LC209480:LC209481:LC209460:LC209465:LC209459:LC209472:LC209
473:LC209474:LC209454:AB662876:AB662875:AB662877:AB662878:AB662879:AB66
2880:AB662887:AB662888:AB662889:AB662890:AB662891:AB662892:AB662893:AB6
62894:AB662895:AB662896:AB662897:AB662898:AB662899:AB662900:AB662901:AB
662902:LC209451:LC209449:LC209479:LC209467:LC209452:LC209447:LC209453:L
C209471:LC209448:KJ407074:KC464505:LC209468:LC209433:LC209445:LC209466:
LC209478:LC209446:LC209432:LC145786:LC145787:LC145788:LC145789:LC145790
:LC145791:LC145792:LC145793:LC145794:LC145795:LC145796:LC145797:MK75294
9:MH938340:LC209442:LC209455:LC209476:LC209456:LC209444:LC209443:LC2094
75:LC209477:LC209431:MH218733:MK762626:MK775028:MH702265:MH702261:MH702
260:KY457721:KY457722:MH938341:LC209450:LC209439:LC209441:LC209434:LC20
9470:LC209458:LC228948:LC145798:LC145799:LC145800:LC145801:LC145802:LC1
45803:LC145804:LC145805:LC145806:LC145807:LC145808:MH218697:MH218648:MH
218655:MH218735:MH218736:MH218737:MH218734:MH218657:MK729086:MK752945:M
K752944:MK753011:KY457724:KY457725:MH938338:MH938339:LC209457:LC209440:
LC213885:KT962983:MG745983:MG745996:MG745997:MG745998:MG745999:MG746000
:MG746001:MG746002:MG746003:MG746004:MG746008:MG746009:MG746010:MG74601
3:MG746014:MG746015:MG746016:MG746017:MG746027:MG746028:MG746029:MG74601
33:MG746035:MG746036:MG746037:MG746038:MG746040:MH671553:MK753015:MK789
430:MK753007:MK753035:MK764040:MK764042:KY905336:KY905337:KY905338:MK76
4039:MK753012:MK775029:KY865306:KY865307:MK773583:MK753013:MK764043:MK7
73581:MK762641:MK773580:MK773582:MK753014:MK753031:MK907802:KY457727:KY
457734:KY457731:KY457729:KY457735:LC413792:LC413793:LC413794:LC413791:M
G763365:KY457728:KY457730:KY677827:KY677828:KY677829:KY677830:KY677831:
KY677832:KY677833:KY817742:KY817743:KY817744:KY817745:KY817746:KY817747
```

[Table 1-16]

EP 4 317 436 A1

:MH158635:MH671554:MH938342:MH938343:MH938344:MH938346:MH938347:MG74604
6:MG746047:MG746048:MG746049:MG746050:MG746051:MG746052:MG746053:MG7460
54:MG746055:MG746056:MG746057:MG746058:MG746059:MG746060:MG746061:MG746
062:MG746063:MG746064:MG746065:MG746066:MG746067:MG746068:MG746069:MG74
6070:MG746071:MG746072:MG746073:MG746074:MG746075:MG746076:MG746077:MG7
46078:MG746079:MG746080:MG746081:MG746082:MG746083:MG746084:MG746085:MG
746086:MG746087:MG746088:MG746089:MG746090:MG746091:MG746092:MG746093:M
G746094:MG746095:MG746096:MG746097:MG746098:MG746099:MG746100:MG746101:
MG746102:MG746103:MG746104:MG746105:MG746106:MG746107:MG746108:MG746121
:MG746122:MG746123:MG746124:MG746125:MG746126:MG746127:MG746128:MG74612
9:MG746133:MG746134:MG746135:MG746136:MG746137:MG746138:MG746139:MG7461
40:MG746141:MG746147:MG746148:MG746149:MG746150:MG746167:KY421121:KY421
122:NC:LC349981:LC349982:LC349983:LC279234:LC279235:LC279236:LC279237:L
C279239:LC279240:LC279241:LC279242:LC279243:LC279238:LC213886:LC213887:
LC213888:LC213889:LC213890:LC213891:LC213892:LC213893:LC213894:LC213895
:LC213896:LC213897:LC213898:LC213899:LC213900:LC213901:LC215413:LC21541
4:LC215415:KY421123:KY421124:KY421125:KY421126:KY421127:KY421128:KY4211
29:KY421130:KY421131:KY421132:KY421133:KY421134:KY421135:KY421136:KY421
137:KY421138:KY421139:KY421140:KY421141:KY421142:KY421143:KY421144:KY42
1145:KY421146:KY421147:KY421148:KY421149:KY421150:KY421151:KY421152:KY4
21153:KY421154:KY421155:KY421156:KY421157:LC325213:LC325214:LC325215:LC
325216:KY421044:KY485115:KY485116:KY485117:KY485118:KY485119:KY485120:K
Y485121:KY485122:KY485123:KY485124:KY485125:KY485126:KY407217:KY407218:
KY407219:KY407220:KY407221:KY457580:KY457581:KY457582:KY457583:KY457584
:KY457585:KY457586:MG892974:MG745985:MG745986:MG745987:MG745988:MG74598
9:MG745990:MG745991:MG745992:MG745993:MG745994:MG746005:MG746006:MG7460

07:MG746011:MG746012:MG746018:MG746019:MG746020:MG746021:MG746022:MG746
023:MG746024:MG746025:MG746026:MG746030:MG746031:MG746032:MG746034:MG74
6039:MG746041:MG746042:MG746043:MG746044:MG746045:MH321825:MK614124:MK6
14125:MK614126:MK614127:MK614128:MK614129:MH041321:MH041322:MN493873:MK
773587:MK753016:MK762633:MK752941:MK752939:MK590974:MK762625:MK762631:M
K764017:MK752940:MK775030:MK340748:MK590973:MK762634:MK753021:MK590972:
MK614142:LC413795:LC413796:LC413797:LC413798:LC413799:LC413800:MG763354
:MG763355:MG763356:MG763357:MG763358:MG763359:MG763360:MG763361:MG76336
2:MG763363:MG763364:MG763366:MG763367:MG763368:MG763369:MG763370:MG7633
71:MG763372:MG763373:MG763374:MG763375:MG763376:MG763377:KY817748:KY817
749:KY817750:KY817751:KY817753:KY817754:KY817752:MH068791:MH068792:MH06
8793:MH068794:MH068795:MH068796:MH068797:MH068798:MH068799:MH068800:MHO
68801:MH068802:MH068803:MH068804:MH068805:MH068806:MH068807:MH068808:MH
068809:MH068810:MH068811:MH068812:MH068813:MH068814:MH068815:MH068816:M
H068817:MH068818:MH068819:MH068820:MH938348:MH938349:MH938350:MH938351:
MH938352:MH938353:MG746109:MG746110:MG746111:MG746112:MG746113:MG746114
:MG746115:MG746116:MG746117:MG746118:MG746119:MG746120:MG746130:MG74613
1:MG746132:MG746142:MG746143:MG746144:MG746145:MG746146:MG746151:MG7461
52:MG746153:MG746154:MG746155:MG746156:MG746157:MG746158:MG746159:MG746
160:MG746161:MG746162:MG746163:MG746164:MG746165:MG746166:MG746168:MG74
6169:MG746170:MG746171:MG746172:MG746173:MG746174:MG746175:MG746176:MG7
46177:MG746178:MG746179:MG746180:MG746181:MG746182:MG746183:MG746184:MG
746185:MG746186:MG746187:MG746188:MG746189:MG746190:MG746191:MG746192:M
G746193:MG746194:MG746196:MG746197:MG746198:MG746199:MG746200:MG746201:
MG746202:MG746203:MG746204:MG746205:MG746206:MG746207:MG746208:MG746209
:MG746210:MG746211:MG746212:MG746213:MG746214:MG746215:MG746216:MG74621

[Table 1-18]

7:MG746219:MG746220:MG746221:MG746222:MG746223:MG746224:MG746225:MK6141

43:MK614144:MK614145:MK614146:MK614147:MK614148:MK614149:MK614150:MK614

151:MK614152:MK614153:MK614154:MK614156:MK614159:MK614161:LC349984:LC34

9985:MK752935:MK614130:MK614131:MK614132:MK614133:MK614134:MK614135:MK6

14136:MK614137:MK764022:MK762628:MK773571:MK614139:MK614140:MK614141:LC

413801:LC413802:LC413803:LC413804:MK614155:MK614157:MK614158:MK614160:M

N394542:MN394544

| | | | | |
|---|---|---|---|---|
| GII. 2 | A2 | PTYTPQ | SEQ ID NO: 116 | GII. 2-LC209464 |
| GII. 2 | A2 | PTYTSQ | SEQ ID NO: 117 | GII. 2-LC209436:LC209438:LC209437:DQ456824:AB662852:AB662853:AB662854:LC 209469 |
| GII. 2 | A2 | PTYTAK | SEQ ID NO: 118 | GII. 2-PIIH938345 |
| GII. 2 | A2 | PTYTTQ | SEQ ID NO: 119 | GII. 2-MG746195 |
| GII. 2 | A2 | PTYAAQ | SEQ ID NO: 120 | GII. 2-MG746218 |
| GII. 2 | A2 | PTHTAK | SEQ ID NO: 121 | GII. 2-MH979229:MK864096 |
| GII. 3 | A2 | DTTSGRFA | SEQ ID NO: 122 | GII. 3-KY442319:KY442320:HM072045:HM072046:HM072042:HM072041: MH218603 |
| GII. 3 | A2 | DTTSGRFT | SEQ ID NO: 123 | GII. 3-KC597144:JN699040:JX846924:JN699039:HM072043:HM072044:HM072040:AB 067542:JQ743333:DQ093063:AB385641:AB385642:KC464326:KC464327:MK907798:K C464328:KP064097:GU138208:KF931324:AB758450:KJ184223:KF944065:JN565063: JX984948:KC464329:KF944111:KF944110:KJ184256:KF944165:KF944166:KF944179 :KF944119:KX355506:KF895841:KT779557:KF944266:MG892076:KJ634708:KM05639 4:MH702267:MH702287:MK396777:MK396772:KF306213:KJ499443:MH218570:MH2187 38:MH218571:MH218712:MH218572:MH218573:MH218574:MH218575:MH218576:MH218 577:MH218578:MH218579:MH218580:MH218581:MH218582:MH218585:MH218587:MH21 8588:MH218590:MH218732:MH218592:MK764020:MK762640:MK907787:MH702259:LC1 33339:LC133343:MH218593:MH218594:MH218597:MH218599:MH218600:MH218601:MH |

[Table 1-19]

| | | | | 218604:MH218618:MH218660:MH218668:MH218672:MH218675:MH218676:MH218677:MH218678:MH218679:MH218680:MH218696:MH218717:MH218630:KT732274:KY767665:KY887597:KY887606:MH218681:MH218682:MH218683:MH218686:MH218688:MH218690:MH218693:KY887598:KY210918:KY210919:MG892946:MG892954:MG892955:MK773588:MH279487 |
|---|---|---|---|---|
| GII. 3 | A2 | DTTAGRFT | SEQ ID NO: 124 | GII. 3-KC464324:KF006265:KC464325:KF931180:GU292851:JN899244:AB365435:AB385634:HM590538:GU980585:KF931243:KF931267:KF931234:LC101823:KC464495:KF944203:KF895859:KF944227:KF944232:KF895848:KY348697:KJ499441:KJ499442:KY407172:KY407173:KY407174:KY407175:MN199033:MH218583:MH218584:MH218586:KY348698:KJ499444:KJ499445:KY767664:KY406922:KY406923:KY406924:KY406925:KY406926:KY406927:KY406929:KY406930:KY406931:KY406932:KY406933:KY406934:KY406937:KY406938:KY406939:KY407190:KY407191:KY407192:KY407193:KY407194:KY905334:MG892915:MG892920:MG892911:MG892910:MK073886:KX989464:KX989465:KX989466:KX989467:KX989468:KY406928:KY406935:KY406936:KY406940:KY406941:KY406942:KY406943:KY406944:KY406945:KY406946:KY406947:KY407195:KY407196:KY407197:KY407198:MG892953:MG892952:MG892947:MG892951:MG892949:MG892950:MG892956 |
| GII. 3 | A2 | DTASGRFT | SEQ ID NO: 125 | GII. 3-AB385626:AB385627 |
| GII. 3 | A2 | DTISGRFT | SEQ ID NO: 126 | GII. 3-KF944147 |
| GII. 3 | A2 | DTTSSRFT | SEQ ID NO: 127 | GII. 3-KC597140:MH218589 |
| GII. 3 | A2 | DTSSPRFT | SEQ ID NO: 128 | GII. 3-MH218595: MH218653: MH218654: MH218646 |
| GII. 3 | A2 | DTTSPRFT | SEQ ID NO: 129 | GII. 3-MH218596:MH218602 |
| GII. 3 | A2 | DTKSGRFA | SEQ ID NO: 130 | GII. 3-MH218598 |
| GII. 3 | A2 | NTTSGRFT | SEQ ID NO: 131 | GII. 3-MH218671 |
| GII. 3 | A2 | DTTAGHFT | SEQ ID NO: 132 | GII. 3-KY407176:KY407177:KY407178:KY407179:KY407180 |

[Table 1-20]

| | | | | |
|---|---|---|---|---|
| GII. 5 | A2 | YSDKYT | SEQ ID NO: 133 | GII.5-JN699044:KJ196277:KJ196288:KM386680:HM596590:KM386681:KM036379:KM036378:KU311160 |
| GII. 6 | A2 | TDPRYT | SEQ ID NO: 134 | GII.6-KY424345:KY424346:KY424347:KY424348:GU969054:GU969055:GU969056:GU969057:HM633213:AB682736:AB818397:AB818398:AB818399:AB758451:AB685739:AB685740:KJ407072:AB818400:KC464321:MH218642:MH218645:MH218666:MH218667:MH218673:MK956198:MK956199:MK956197 |
| GII. 6 | A2 | TDPKYA | SEQ ID NO: 135 | GII.6-KC576910:JN699035:JN699036:AB078337:DQ093064:AB818404:JN183165:AB685738:KX752057:MH279832:KM461694:KM461693:KP064098:MH279834:MH279831:MH114014 |
| GII. 6 | A2 | TNPRYT | SEQ ID NO: 136 | GII.6-JN699041:AB818403:AB818402:AB685742:HQ169542:AB818401:AB685741:JX984945:JX984949:JX989075:JX984953:KY424341:KY424342:KY424343:KY424344:MH218639:MK907789:MK907786:MH702288:MH279837:MH279838:KM036374:KM036373:KM036375:KX268709:LC133342:LC133338:KU935739:MH218640:MH218643:MH218650:MH218719:MN248516:MN248517:MN248514:MH279827:MH279839:MH279835:MH279828:MG571778:KY406918:MH218687:MH702284:MH702286:KY406919:KY406920:KY406921:KY407213:KY407214:KY407215:KY407216 |
| GII. 6 | A2 | TNPRYA | SEQ ID NO: 137 | GII.6-LN854568 |
| GII. 6 | A2 | KNPRYT | SEQ ID NO: 138 | GII.6-MH218641 |
| GII. 6 | A2 | TNSRYT | SEQ ID NO: 139 | GII.6-MH218644 |
| GII. 6 | A2 | TDPRYA | SEQ ID NO: 140 | GII.6-MK301293 |
| GII. 7 | A2 | GGDQYA | SEQ ID NO: 141 | GII.7-JN699042:KC832474:GQ849129:MH279833:MH279830:GQ849130:GU134965:KJ196295:MH279829:MH218658: MH218661:MH218692:MN038126 |
| GII. 7 | A2 | GGNQYA | SEQ ID NO: 142 | GII.7-KF006266 |

[Table 1-21]

| | | | | |
|---|---|---|---|---|
| GII. 12 | A2 | NSAKFT | SEQ ID NO: 143 | GII. 12-LC342059:KJ196294:KJ196282:KJ196299:GQ845370:JQ613568:HQ688986:JQ613569:HQ449728:HQ664990:KC464498:KC464496:KC464500:KC464499:KC464497:HQ401025 |
| GII. 12 | A2 | NSTKFT | SEQ ID NO: 144 | GII. 12-KF006267 |
| GII. 12 | A2 | DSAKFT | SEQ ID NO: 145 | GII. 12-KP064099 |
| GII. 12 | A2 | DSTKFT | SEQ ID NO: 146 | GII. 12-MK754445:MK762627:MK754447:MK616558:MK616561:MK616560:MK616559:MK355712:MK355713 |
| GII. 12 | A2 | HSTKFT | SEQ ID NO: 147 | GII. 12-MK753036:MK764041 |
| GII. 13 | A2 | TSGKFT | SEQ ID NO: 148 | GII. 13-KJ196276:AB809973:AB809974:AB809993:AB809997:AB809990:AB809975:AB809976:AB809994:AB809977:AB809992:AB809985:AB809996:AB809987:AB809978:AB809988:AB809979:AB809986:AB809991:JN899242:AB810004:AB810001:AB810014:AB810006:AB810011:AB810003:KY210920:MK396778:KY406985:KY406986 |
| GII. 13 | A2 | TSGRFT | SEQ ID NO: 149 | GII. 13-MK753009:MK753008:MH702276:MH702277:KM036380:MK753010:MK754443:MK762560:MK752947:MK753020:MK762559:MH702263:MH218651:MK752946:MK762745:MK764014:KY406982:KY406983:KY406984:KY947548:MK762565:MG892908:MK775031:MN394543:MN394545 |
| GII. 14 | A2 | AGDTFA | SEQ ID NO: 150 | GII. 14-JN699038:KJ196278:KJ196297:GU017900:GU017903:GU594162:GU017901:GU017902:GU017904:GU017905:GU017906:GU017907:GU017908:MH279826:MK850443:MK588004:MK641589:LC556388:LC556389:LC556391:LC556390:LC556392 |
| GII. 17 | A2 | STTSSQFVP | SEQ ID NO: 151 | GII. 17-KC597139:JN699043:KJ196286:MH218591:KT589391:KT346358:KY406980:KY406981:MH375799:LC333898:MH375809:MH375810:MH375811:MH375814 |

[Table 1-22]

| GII. 17 | A2 | ATTSTQFVP | SEQ ID NO: 152 | GII. 17-DQ438972 |
|---------|----|-----------|-----------------|-------------------|
| GII. 17 | A2 | STYSPQFVP | SEQ ID NO: 153 | GII. 17-MK907801:MK907800:LC433694:KU561250:KU561251:LC043168:LC043167:KU557788:KJ156329:LC486737:KU557801:KU557839:MK282256:MK282258:LC433720:MK077684:KP998539:KT380915:KT780394:KT780395:KT780396:KT780397:KT780398:KT780399:KU561252:KU561253:LC043139:LC043305:KR083017:KY424349:KY424350:KR020503:KT326180:KU557790:KU557785:KU557786:KU557787:LC486738:KT285173:KX171413:KX171412:KX171417:KP698928:KP698929:KP902566:KP902567:KP902568:KP902569:KP902570:KP902571:KP902572:KP902573:KP902574:KP902576:KP902577:KP902578:KT315668:KT315669:KT315670:KT315671:KT315672:KT315673:KP864103:KP864104:KU561224:KU561225:KU561226:KU561227:KU561228:KU561229:KU557797:KU557798:KU557799:KU557802:KU557803:KU557806:KU557807:KU557808:KU557809:KU557810:KU557811:KU557815:KU557816:KU557817:KU557822:KU557823:KU557824:KU557827:KU557828:KU557829:KU557833:KU557834:KU557835:KU557846:KU557847:KU557848:KU557849:KU557885:KY069114:KX346699:KP902575:KP902563:KP902564:KP902565:LC369228:LC369214:MG692610:LC369229:LC369230:KT970369:KT970370:MK907791:MK396775:MK907792:MK907793:LC433721:LC433695:KT149168:KT149169:MK077685:MK077706:AB983218:MF918359:KT780400:KT780401:KT780402:KT780403:KT780404:KT780405:KT780406:KT780407:KT780408:KT780409:KT780410:KT780411:KT780412:KT780413:KT780414:KT780415:KT780416:KU561248:KU561249:KT253245:KU561254:KU561255:KU561256:KX356908:NC:LC037415:KU557783:KU557784:KR154230:KR154231:KT992790:KT326181:KT326182:KT992789:KT992786:KT992787:KT992785:KT992788:KY392867:KY392868:KR052021:KR052019:KR052020:KR052022:LC349987:LC349988:LC349992:KT346356:KX024652:LC486739:LC486740:LC486741:LC486747:LC486751:LC486761:LC486762:LC486763:LC148844:LC148845:LC148846:LC148847:LC148848:LC148849:LC148850:LC148851:LC101820:LC177662:KX171414:KX171419:KX420892:KX420891:KX171418:KX171416:KP698930:K |

[Table 1-23]

```
P698931:KP902579:KP902580:KP902581:KP902582:KP902583:KP902584:KP902585:
KP902586:KP902587:KP902588:KP902589:KP902590:KT315674:KT315675:KT315676
:KT315677:KT315678:KT315679:KT315680:KT315681:KT315682:KT315683:KT315568
4:KT315685:KT315686:KT315687:KT315688:KT315689:KT315690:KT315691:KT3156
92:KT315693:KT315694:KT315695:KT315696:KT315697:KT315698:KT315699:KT315
700:KT315701:KT315702:KT315703:KT315704:KT315705:KT315706:KT315707:KT31
5708:KT315709:KT315710:KT315711:KT315712:KT315713:KT315714:KT315715:KT3
15716:KT315717:KT315718:KT315719:KP864102:KT591501:KU953392:KU
953393:KU561230:KU561231:KU561232:KU561233:KU561234:KU561235:KU561236:K
U561237:KU561238:KU561239:KU561240:KU561241:KU561243:KU561244:KU561245:
KU561246:KU561247:KU557800:KU557804:KU557805:KU557812:KU557813:KU557814
:KU557818:KU557819:KU557820:KU557821:KU557825:KU557826:KU557830:KU55783
1:KU557832:KU557836:KU557837:KU557838:KU557840:KU557841:KU557842:KU5578
43:KU557844:KU557845:KU557850:KU557851:KU557852:KU557853:KU557854:KU557
855:KU557856:KU557857:KU557858:KU557859:KU557860:KU557861:KU557862:KU55
7863:KU557864:KU557865:KU557866:KU557867:KU557868:KU557869:KU557870:KU5
57871:KU557872:KU557873:KU557874:KU557875:KU557876:KU557877:KU557878:KU
557879:KU557880:KU557881:KU557882:KU557883:KU557884:KU557886:KU557887:K
U557888:KU557889:KU557890:KU557891:KU557892:KU557893:KX244850:KX244851:
KX244852:KX244853:KU587626:KU587627:KU587628:KU587629:KX134669:KX134670
:KX164437:KX164438:KX371107:KX371108:KX371109:KX371110:KY397953:KY39795
4:KY397955:KY397956:KY397957:KY397958:KX346700:KX346701:KX346702:KX3467
03:KX346704:KX346705:MF172092:MF172093:MF172094:KY406954:KY406955:KY406
956:KY406957:KY406958:KY406959:KY406960:KY407181:KY407182:KY407183:KY40
7184:KY407185:KY407186:LC369237:LC369234:LC369233:LC369242:LC3
69255:LC369235:LC369222:LC369215:LC369227:LC369219:LC369236:LC369244:LC
```

[Table 1-24]

369220:LC369232:LC369238:MN853414:LC369245:LC369256:LC369224:LC369240:L
C369241:MH997861:LC369231:LC369243:LC369225:LC369239:LC369249:LC369246:
LC369247:LC369248:LC369254:LC369253:LC369252:LC369251:KY905332:MK789431
:LC369218:LC369221:LC369216:LC369217:KT970371:KT970372:KT970373:KT97037
4:KT970375:KT970376:KT970377:MH747479:MH747481:MH890538:MH747480:LC3692
58:MH747482:MH890539:LC433696:LC433698:LC433699:LC433709:LC433710:LC433
711:LC433712:LC433713:LC433722:LC433723:LC433724:LC433726:LC433727:LC43
3729:LC433730:LC433731:LC433732:KX424646:KX424647:KX424648:KX424649:KX4
24650:KT149170:KT149171:KT149172:KT149173:KT149174:KT149175:KT149176:KX
216782:KX216783:KX216784:KX216785:KX216786:KX216788:KX216789:KX216790:K
X216791:KX216792:KX216793:KX216794:KX216795:KX216796:KX216797:KX216798:
KX216799:KX216800:KX216801:KX216802:KX216803:KX216804:KX216805:KX216806
:MF073239:MF073240:MF073241:MK077686:MK077687:MK077688:MK077689:MK07769
0:MK077691:MK077701:MK077702:MK077703:MK077704:MK077705:MK077708:LC3499
86:LC349989:LC349990:LC349991:LC349993:LC486742:LC486746:LC486748:LC486
749:LC486750:LC486752:LC486753:LC486754:LC486764:LC486765:LC486766:LC14
8852:LC148853:LC148854:LC148855:LC148856:KX420895:KX420894:KX420893:KU9
53394:KU953395:KU953396:KU953397:KU953398:KX244854:KX134671:KY069115:KX
168439:KX168440:KX168441:KX168442:KX168443:KX168444:KX168445:KX168446:K
X168447:KX168448:KX168449:KX168450:KX168451:KX168452:KX168453:KX168454:
KX168455:KX168456:KX371111:KX371112:MF172095:MF172096:KX989474:KX989475
:KX989476:KX989477:KX989478:KY406961:KY406962:KY406963:KY406964:KY40696
5:KY406966:KY406967:KY406968:KY406969:KY406970:KY406971:KY406972:KY4069
73:KY406974:KY406975:KY406976:KY406977:KY406978:KY406979:KY407203:KY407
204:KY407205:KY407206:KU555841:MH218689:KY905330:MH746922:MH746923:MH74
6924:MH746925:LC318746:LC318745:LC318747:LC333866:LC333867:LC333868:LC3

[Table 1-25]

33869:LC333870:LC333871:LC333872:LC333873:LC333874:LC333875:LC333876:LC
333877:LC333878:LC333879:LC333880:LC333881:LC333882:LC333883:LC333884:L
C333885:LC433716:LC433718:LC433719:LC433733:LC433734:LC433735:LC433736:
MF073242:MF073243:MF073244:MF073245:MF073246:MF073247:MF073248:MF073249
:MF073250:MF073251:MF073252:MF073253:MF073254:MH375801:MH375802:MH37580
3:MH375804:MH375805:MH572223:MH572224:MH572225:MH572226:MH572227:MH5722
28:MH572229:MH572230:MH572231:MK077707:MK077710:MK077712:MK077713:LC318
750:MF421538:MF421539:MF421540:MF421541:MF421542:LC258403:LC311767:LC31
1768:LC311769:LC311770:LC311771:LC311772:LC311773:LC486743:LC486744:LC4
86745:LC486755:LC486756:LC486757:LC486758:LC486759:LC486760:LC486767:LC
486768:LC486769:LC486770:MK789432:MK789433:LC318753:LC318754:LC318752:L
C333886:LC333887:LC333888:LC333889:LC333890:LC333891:LC333892:LC333893:
LC333894:LC333896:LC333897:LC333899:LC333900:LC333901:LC318755:LC318756
:LC318757:LC318758:LC333895:MH375806:MH375808:MH572232:MK077692:MK07769
3:MK077694:MK077696:MK077697:MK077711:MK077714:MG995040:MH375813:MK0776
95:MK077698:MK077699:MK077700:MN394546

| GII.17 | A2 | STHSPOFVP | SEQ ID NO: 154 | GII. 17-MK282257:KU561242 |
|--------|----|-----------|----------------|----------------------------|
| GII.17 | A2 | STYSPKFVP | SEQ ID NO: 155 | GII. 17-KU587625:MK907790 |
| GII.17 | A2 | STYIPQFVP | SEQ ID NO: 156 | GII. 17-KX171415 |
| GII.17 | A2 | SPYSPQFVP | SEQ ID NO: 157 | GII. 17-LC318748 |
| GII.17 | A2 | STYSPQFVR | SEQ ID NO: 158 | GII. 17-MK077709 |
| GII.21 | A2 | TSEKFT | SEQ ID NO: 159 | GII.21-GU138162:GU138163:HM590541:GU138176:GU138177:HM590519:KJ196284:JN899245 |

[Table 1-26]

| | | | | |
|---|---|---|---|---|
| GII. 21 | A2 | TSDKFT | SEQ ID NO: 160 | GII. 21-MH702268:MH702279:MK396776:MH702281:MH702280:MH702278:MH702282:MH702264:MK396773:KR921935:KR921936:KR921937:MH702285:MK396771:KX079488:KT962982:KR921938:KR921939:KR921940:KR921941:KR921942:KY406949:KY406950:MH702257:KY210925:KY406951:KY406952:KY406953:KY407199:KY407200:KY407201:KY407202 |
| GII. 1 | A3 | GTWEESDL | SEQ ID NO: 161 | GII. 1-JX289822:JN797508:KC463911:KC464322:KC464323:MF668937 |
| GII. 1 | A3 | GTWEEHDL | SEQ ID NO: 162 | GII. 1-MH218731:MK753033 |
| GII. 1 | A3 | GAWEDRDF | SEQ ID NO: 163 | GII. 1-MG572182 |
| GII. 1 | A3 | GTWEERDL | SEQ ID NO: 164 | GII. 1-MK753034 |
| GII. 1 | A3 | GTWEDRDF | SEQ ID NO: 165 | GII. 1-MK483908 |
| GII. 1 | A3 | GTWEEQDL | SEQ ID NO: 166 | GII. 1-MK616585:MK616584 |
| GII. 2 | A3 | IGTWQTDDLTVNQP | SEQ ID NO: 167 | GII. 2-MF405169:AB281081:AB281082:AB281083:AB281084:AB281085:AB281086:AB281087:AB281088:LC209464:LC209436:LC209438:LC209437:DQ456824:AB662852:AB662853:AB281089:AB281090:LC209462:AB662854:AB662863:LC209463:AB662859:AB662867:LC209461:AB662870:AB662881:AB662884:LC209480:LC209481:LC209460:LC209459:LC209454:AB662876:AB662880:AB662889:AB662893:AB662894:AB662895:AB662896:AB662897:AB662899:AB662900:LC209451:LC209449:LC209479:LC209467:LC209452:LC209447:LC209453:LC209471:LC209448:KJ407074:KC464505:LC209468:LC209445:LC209466:LC209446:LC209432:LC145787:LC145786:LC145789:LC145790:LC145791:LC145792:LC145793:LC145794:LC145796:LC145797:MK752949:MH938340:LC209442:LC209455:LC209476:LC209444:LC209443:LC209475:LC209477:LC209431:MH218733:MK762626:MK775028:MH702265:MH702261:MH702260:KY457721:KY457722:MH938341:LC209450:LC209439:LC209469:LC209441:LC209434:LC209458:LC228948:LC145798:LC145799:LC145800:LC145801:LC145802:LC145803:LC145807:LC145808:MH218697:MH218655:MH218735:MH218736:MH218737:MH218734:MH21865 |

42

EP 4 317 436 A1

[Table 1-27]

EP 4 317 436 A1

7:MK729086:MK752945:MK752944:MK753011:KY457724:KY457725:MH938338:MH9383
39:LC209440:LC213885:KT962983:MG745995:MG745996:MG745997:MG745998:MG745
999:MG746000:MG746001:MG746002:MG746003:MG746004:MG746008:MG746009:MG74
6010:MG746013:MG746014:MG746015:MG746016:MG746017:MG746027:MG746028:MG7
46029:MG746033:MG746035:MG746036:MG746037:MG746038:MG746040:MH671553:MK
753015:MK789430:MK753007:MK753035:MK764040:MK764042:KY905336:KY905337:K
Y905338:MK764039:MK753012:MK775029:KY865306:KY865307:MK773583:MK753013:
MK764043:MK773581:MK762641:MK773580:MK773582:MK753014:MK753031:MK907802
:KY457727:KY457734:KY457731:KY457735:LC413792:LC413793:LC413794:LC41379
1:MG763365:KY457728:KY457730:KY677827:KY677828:KY677829:KY677830:KY6778
31:KY677832:KY677833:KY817742:KY817743:KY817744:KY817745:KY817746:KY817
747:MH158635:MH671554:MH938342:MH938343:MH938344:MH938345:MH938346:MH93
8347:MG746046:MG746047:MG746048:MG746049:MG746050:MG746051:MG746052:MG7
46053:MG746054:MG746055:MG746056:MG746057:MG746058:MG746059:MG746060:MG
746061:MG746062:MG746063:MG746064:MG746065:MG746066:MG746067:MG746068:M
G746070:MG746071:MG746072:MG746073:MG746074:MG746075:MG746076:MG746077:
MG746078:MG746079:MG746080:MG746081:MG746082:MG746083:MG746084:MG746085
:MG746086:MG746087:MG746088:MG746089:MG746090:MG746091:MG746092:MG74609
3:MG746094:MG746095:MG746096:MG746097:MG746098:MG746099:MG746100:MG7461
01:MG746102:MG746103:MG746104:MG746105:MG746106:MG746107:MG746108:MG746
121:MG746122:MG746123:MG746124:MG746125:MG746126:MG746127:MG746128:MG74
6129:MG746133:MG746134:MG746135:MG746136:MG746137:MG746138:MG746139:MG7
46140:MG746141:MG746147:MG746148:MG746149:MG746150:MG746167:KY421121:KY
421122:NC:LC349981:LC349982:LC349983:LC279234:LC279235:LC279236:LC27923
7:LC279239:LC279240:LC279241:LC279242:LC279243:LC279238:LC213886:LC2138
89:LC213890:LC213891:LC213892:LC213893:LC213894:LC213895:LC213896:LC213

[Table 1-28]

897:LC213898:LC213900:LC213901:LC215413:LC215414:LC215415:KY421123:KY42
1124:KY421125:KY421126:KY421127:KY421128:KY421129:KY421130:KY421131:KY4
21132:KY421133:KY421134:KY421135:KY421136:KY421137:KY421138:KY421139:KY
421140:KY421141:KY421142:KY421143:KY421144:KY421145:KY421146:KY421147:K
Y421148:KY421149:KY421150:KY421151:KY421152:KY421153:KY421154:KY421155:
KY421156:KY421157:LC325213:LC325214:LC325215:LC325216:KY421044:KY485115
:KY485116:KY485117:KY485118:KY485119:KY485120:KY485121:KY485122:KY48512
3:KY485124:KY485125:KY485126:KY407217:KY407218:KY407219:KY407220:KY4072
21:KY457580:KY457581:KY457582:KY457583:KY457584:KY457585:KY457586:MG892
974:MG745985:MG745986:MG745987:MG745988:MG745989:MG745990:MG745991:MG74
5992:MG745993:MG745994:MG746005:MG746006:MG746007:MG746011:MG746012:MG7
46018:MG746019:MG746020:MG746021:MG746022:MG746023:MG746024:MG746025:MG
746026:MG746030:MG746031:MG746032:MG746034:MG746039:MG746042:MG746043:M
G746044:MG746045:MH321825:MK614125:MK614126:MK614127:MK614128:MK614129:
MH041321:MH041322:MN493873:MK773587:MK753016:MK762633:MK752941:MK752939
:MK590974:MK762631:MK764017:MK775030:MK340748:MK590973:MK762634:MK75302
1:MK590972:MK614142:LC413795:LC413796:LC413797:LC413798:LC413799:LC4138
00:MG763354:MG763355:MG763356:MG763357:MG763358:MG763359:MG763360:MG763
361:MG763362:MG763363:MG763366:MG763367:MG763368:MG763369:MG763370:MG76
3371:MG763372:MG763373:MG763374:MG763375:MG763377:KY817748:KY817749:KY8
17750:KY817751:KY817753:KY817754:KY817752:MH068791:MH068792:MH068793:MH
068794:MH068795:MH068796:MH068797:MH068798:MH068799:MH068800:MH068801:M
H068802:MH068803:MH068804:MH068805:MH068806:MH068807:MH068808:MH068809:
MH068810:MH068811:MH068812:MH068813:MH068814:MH068815:MH068816:MH068817
:MH068818:MH068819:MH068820:MH938348:MH938349:MH938350:MH938352:MH93835
3:MG746109:MG746110:MG746111:MG746112:MG746113:MG746114:MG746115:MG7461

[Table 1-29]

16:MG746117:MG746118:MG746119:MG746120:MG746130:MG746131:MG746132:MG746
142:MG746143:MG746144:MG746145:MG746146:MG746151:MG746152:MG746153:MG74
6154:MG746155:MG746156:MG746157:MG746158:MG746159:MG746160:MG746161:MG7
46162:MG746163:MG746164:MG746165:MG746166:MG746168:MG746169:MG746170:MG
746171:MG746172:MG746173:MG746174:MG746175:MG746176:MG746178:MG746179:M
G746180:MG746181:MG746182:MG746183:MG746184:MG746185:MG746186:MG746187:
MG746188:MG746189:MG746190:MG746191:MG746192:MG746193:MG746194:MG746195
:MG746196:MG746197:MG746198:MG746199:MG746200:MG746201:MG746203:MG74620
4:MG746205:MG746206:MG746207:MG746208:MG746209:MG746210:MG746211:MG7462
12:MG746213:MG746214:MG746215:MG746216:MG746217:MG746218:MG746219:MG746
220:MG746221:MG746222:MG746223:MG746224:MG746225:MK614143:MK614144:MK61
4145:MK614146:MK614147:MK614148:MK614149:MK614150:MK614152:MK614153:MK6
14154:MK614156:MK614159:MK614161:LC349984:LC349985:MK752935:MK614130:MK
614131:MK614132:MK614133:MK614134:MK614135:MK614136:MK614137:MK764022:M
K762628:MK773571:MK614139:MK614140:MK614141:LC413801:LC413802:LC413803:
LC413804:MK614155:MK614157:MK614158:MK614160:MN394542:MN394544

| | | | | |
|---|---|---|---|---|
| GII. 2 | A3 | IGTWQTDDLKVNQP | SEQ ID NO: 168 | GII. 2-JX846925:JN699037 |
| GII. 2 | A3 | IGTWQTDDITVDQP | SEQ ID NO: 169 | GII. 2-JQ320072:KC998960 |
| GII. 2 | A3 | IGTWQTDDLQVNQP | SEQ ID NO: 170 | GII. 2-AB195225:LC209435:AB662850:AB662851:AB662856:AB662855 |
| GII. 2 | A3 | IGTWQTDDLTVSQP | SEQ ID NO: 171 | GII. 2-AB662858:AB662864:AB662865:AB662860:AB662866:AB662869:AB535749:AB662862:AB662871:AB662872:AB662873:AB662874:AB662882:AB662883:AB662885:AB662886:LC209465:LC209472:LC209473:LC209474:AB662875:AB662877:AB662878:AB662879:AB662887:AB662888:AB662890:AB662891:AB662892:AB662898:AB662901:AB662902:LC209478:LC209457:MK614124 |
| GII. 2 | A3 | IGTWQTDDLTDNQP | SEQ ID NO: 172 | GII. 2-AB662861 |
| GII. 2 | A3 | IGTWQTDDLTNNQP | SEQ ID NO: 173 | GII. 2-AB662868 |

[Table 1-30]

| | | | | |
|---|---|---|---|---|
| GII. 2 | A3 | IGTWQTDDLAVNQP | SEQ ID NO: 174 | GII. 2-LC209433:LC145788:LC209456:LC209470:LC145804:LC145805:LC145806 |
| GII. 2 | A3 | IGTWQTDDLTINQP | SEQ ID NO: 175 | GII. 2-LC145795 |
| GII. 2 | A3 | IGTWQTNDLLDNQP | SEQ ID NO: 176 | GII. 2-MH218648 |
| GII. 2 | A3 | IGTWQTDDLTVNQS | SEQ ID NO: 177 | GII. 2-KY457729:MG746069 |
| GII. 2 | A3 | IGTWQIDDLTVNQP | SEQ ID NO: 178 | GII. 2-LC213887:LC213888:LC213899 |
| GII. 2 | A3 | IGTWQTDDLIVNQP | SEQ ID NO: 179 | GII. 2-MG746041:MG746202 |
| GII. 2 | A3 | IGTWQTDDITVNQP | SEQ ID NO: 180 | GII. 2-MK762625:MH938351:MH979229:MK864096 |
| GII. 2 | A3 | IGTWQTDDLNVNQP | SEQ ID NO: 181 | GII. 2-MK752940 |
| GII. 2 | A3 | VGTWOTDDLTVNQP | SEQ ID NO: 182 | GII. 2-MG763364:MG763376:MG746177 |
| GII. 3 | A3 | ISTESSDFDSNQP | SEQ ID NO: 183 | GII. 3-KY442319:KY442320:HM072045:HM072046 |
| GII. 3 | A3 | ISTESGDFDPNQP | SEQ ID NO: 184 | GII. 3-KC597144:JN699040 |
| GII. 3 | A3 | ISTESDDFDPNQP | SEQ ID NO: 185 | GII. 3-JX846924:JN699039:MK396777 |
| GII. 3 | A3 | ISTESDDFDSNQP | SEQ ID NO: 186 | GII. 3-HM072042 |
| GII. 3 | A3 | ITTESNDFDSNQP | SEQ ID NO: 187 | GII. 3-HM072043 |
| GII. 3 | A3 | ITTESDDFDPNQS | SEQ ID NO: 188 | GII. 3-HM072044 |
| GII. 3 | A3 | IITESDDFDLNQS | SEQ ID NO: 189 | GII. 3-HM072040 |
| GII. 3 | A3 | ISTETDDFNQNQP | SEQ ID NO: 190 | GII. 3-HM072041 |
| GII. 3 | A3 | ITTESDDFDTNQS | SEQ ID NO: 191 | GII. 3-AB067542:JQ743333 |
| GII. 3 | A3 | ITTESDDFNQNKP | SEQ ID NO: 192 | GII. 3-DQ093063 |
| GII. 3 | A3 | ISTESDDFDQNQP | SEQ ID NO: 193 | GII. 3-KC464324:KF006265:KC464325:KF931180:GU292851:AB385634:LC101823:KJ184256:KF944179:KF944147:KF895859:MG892076:KY348697:KJ499443:KY406927:KY406931:KY407176:KY407177:KY407178:KY407179:KY407180:KY905334:MG892920:KY406928:KY406940:KY406945:MG892952:MG892949:MG892956:MH279487 |
| GII. 3 | A3 | ISTESGDFDQNQP | SEQ ID NO: 194 | GII. 3-JN899244:HM590538:GU980585:KF9312438:KF931267:KF931234:AB758450:KJ |

[Table 1-31]

184223：KC464495：JN565063：JX984948：KF944111：KF944110：KF944165：KF944166：K
F944203：KF944119：KX355506：KT779557：KF944227：KF944232：KF895848：KF944266：
KJ634708：KF306213：KJ499441：KJ499442：KY407172：KY407173：KY407174：KY407175
：MN199033：MH218578：MH218579：MK762640：MK907787：KY348698：LC133339：LC13334
3：KJ499444：KJ499445：MH218597：KT732274：KY767664：KY767665：KY406922：KY4069
23：KY406924：KY406925：KY406926：KY406930：KY406932：KY406933：KY406934：KY406
937：KY406938：KY406939：KY407190：KY407191：KY407192：KY407193：KY407194：MG89
2915：MG892911：MG892910：MK073886：KX989464：KX989465：KX989466：KX989467：KX9
89468：KY406935：KY406936：KY406941：KY406942：KY406943：KY406944：KY406946：KY
406947：KY407195：KY407196：KY407197：KY407198：MG892953：MG892947：MG892951：M
G892950

| GII. 3 | A3 | ISTESDDFHQNQP | SEQ ID NO: 195 | GII. 3-AB365435 |
|---|---|---|---|---|
| GII. 3 | A3 | ISTESDDFDQNKP | SEQ ID NO: 196 | GII. 3–AB385626：AB385627：AB385641：AB385642：KC464326：KC464327：MK907798：KC464328：KP064097：GU138208：KF931324：KC464329：KM056394：MH218573：MH218584：MH218585：MH218586 |
| GII. 3 | A3 | ISTESDDFDQNQS | SEQ ID NO: 197 | GII. 3-KF944065 |
| GII. 3 | A3 | ISTESDDFDENKP | SEQ ID NO: 198 | GII. 3-KC597140 |
| GII. 3 | A3 | MSTESDDFDQNQP | SEQ ID NO: 199 | GII. 3-KF895841 |
| GII. 3 | A3 | ISTESSDFDQNQP | SEQ ID NO: 200 | GII. 3–MH702287：MK396772：MH218570：MH218738：MH218571：MH218712：MH218572：MH218574：MH218575：MH218576：MH218577：MH218580：MH218581：MH218582：MH218587：MH218588：MH218732：MH218592：MH702259：MH218593：MH218594：MH218599：MH218600：MH218601：MH218604：MH218630：KY887597：KY887606：KY887598：KY210918：MG892946：MG892955：MK773588 |
| GII. 3 | A3 | ISTESGDFEQNQP | SEQ ID NO: 201 | GII. 3-MH218583 |
| GII. 3 | A3 | ISTESDDFDENQP | SEQ ID NO: 202 | GII. 3-MH218589 |

[Table 1-32]

| GII. 3 | A3 | MSTESSDFDQNQP | SEQ ID NO: 203 | GII. 3-MH218590 |
|---|---|---|---|---|
| GII. 3 | A3 | ISTESDDFAQNQP | SEQ ID NO: 204 | GII. 3-MK764020 |
| GII. 3 | A3 | ISTESDDFEHNQP | SEQ ID NO: 205 | GII. 3-MH218595:MH218653:MH218654 |
| GII. 3 | A3 | ISTESDDFEQNQP | SEQ ID NO: 206 | GII. 3-MH218596:MH218602 |
| GII. 3 | A3 | ISTESGDFDQNKP | SEQ ID NO: 207 | GII. 3-MH218598:MH218603 |
| GII. 3 | A3 | ISTESHDFDENEP | SEQ ID NO: 208 | GII. 3-MH218618 |
| GII. 3 | A3 | ISTESSDFDQSQP | SEQ ID NO: 209 | GII.3-MH218660:MH218668:MH218671:MH218672:MH218675:MH218676:MH218677:MH218678:MH218679:MH218680:MH218681:MH218682:MH218683:MH218686:MH218688:MH218690:MH218693:KY210919:MG892954 |
| GII. 3 | A3 | ISTESHDFDQNEP | SEQ ID NO: 210 | GII. 3-MH218696 |
| GII. 3 | A3 | ISTESGDFDQNEP | SEQ ID NO: 211 | GII. 3-MH218717 |
| GII. 3 | A3 | ISTESDDFNQNQP | SEQ ID NO: 212 | GII. 3-KY406929 |
| GII. 3 | A3 | ISTESDDFELNQP | SEQ ID NO: 213 | GII. 3-MH218646 |
| GII. 5 | A3 | GTWNTN | SEQ ID NO: 214 | |
| GII. 6 | A3 | MHSESDDFVTG | SEQ ID NO: 215 | GII.5-JN699044:KJ196277:KJ196288:KM386680:HM596590:KM386681:KM036379:KM036378:KU311160<br><br>GII.6-KY424345:KY424346:KY424347:KY424348:GU969054:GU969055:GU969056:GU969057:HM633213:AB818398:AB758451:AB685739:AB685740:KJ407072:AB818400:MH218667:MK301293:MK956198:MK956199:MK956197 |
| GII. 6 | A3 | IKSESNDFTTN | SEQ ID NO: 216 | GII. 6-KC576910:JN699035:JN699036 |
| GII. 6 | A3 | MYSESTDFDDG | SEQ ID NO: 217 | GII.6-JN699041:KY424341:KY424342:KY424343:KY424344:MH218639:MK907789:MK907786:MH702288:MH279837:MH279838:KM036375:LN854568:MH218719:MH279828:KY406918:MH218687 |
| GII. 6 | A3 | IKSGSDDFNTN | SEQ ID NO: 218 | GII. 6-AB078337 |
| GII. 6 | A3 | IKSESNDFITN | SEQ ID NO: 219 | GII. 6-DQ093064 |

[Table 1-33]

| | | | | |
|---|---|---|---|---|
| GII. 6 | A3 | IKSDSNDFNTN | SEQ ID NO: 220 | GII. 6-AB818404 |
| GII. 6 | A3 | MHSESSDFDDG | SEQ ID NO: 221 | GII. 6-AB818403:AB685742:JX989075:JX984953 |
| GII. 6 | A3 | MNSESSDFDDG | SEQ ID NO: 222 | GII. 6-AB818402:AB818401:AB685741 |
| GII. 6 | A3 | MYSESSDFDDG | SEQ ID NO: 223 | GII. 6-HQ169542:KM036374:KM036373 |
| GII. 6 | A3 | IKSESTDFTTN | SEQ ID NO: 224 | GII. 6-JN183165:KM461694:KM461693:KP064098:MH279834:MH279831 |
| GII. 6 | A3 | IKSESPDFNTN | SEQ ID NO: 225 | GII. 6-AB685738:KX752057 |
| GII. 6 | A3 | MRSESDDFVTG | SEQ ID NO: 226 | GII. 6-AB682736 |
| GII. 6 | A3 | MHSESGDFVTG | SEQ ID NO: 227 | GII. 6-AB818397 |
| GII. 6 | A3 | IHSESDDFVTG | SEQ ID NO: 228 | GII. 6-AB818399 |
| GII. 6 | A3 | MYSESDDFDDG | SEQ ID NO: 229 | GII. 6-JX984945:JX984949 |
| GII. 6 | A3 | IKSESTDFTTS | SEQ ID NO: 230 | GII. 6-MH279832 |
| GII. 6 | A3 | MYSESDDFVTG | SEQ ID NO: 231 | GII. 6-KC464321 |
| GII. 6 | A3 | MHSESTDFDDG | SEQ ID NO: 232 | GII. 6-KX268709:MH218641:MG571778:KY406921 |
| GII. 6 | A3 | MYSESNDFDDG | SEQ ID NO: 233 | GII. 6-LC133342:LC133338:KU935739:MH218640:MH218643:MH702284:MH702286:KY406920:KY407213:KY407214: KY407215:KY407216 |
| GII. 6 | A3 | MYSESDDFDNG | SEQ ID NO: 234 | GII. 6-MH218644 |
| GII. 6 | A3 | MYSESGDFDDG | SEQ ID NO: 235 | GII. 6-MH218650:MH279827:MH279839:MH279835:KY406919 |
| GII. 6 | A3 | MYSESPDFDDG | SEQ ID NO: 236 | GII. 6-MN248516:MN248517:MN248514 |
| GII. 6 | A3 | MYSESEDFVTG | SEQ ID NO: 237 | GII. 6-MH218642:MH218645:MH218666:MH218673 |
| GII. 6 | A3 | IKSESPDFTTN | SEQ ID NO: 238 | GII. 6-MH114014 |
| GII. 7 | A3 | FSESQDFEV | SEQ ID NO: 239 | GII. 7-JN699042:KC832474:GQ849129:MH279833:MH279830:GQ849130:GU134965:KJ196295:MH279829:MH218658: MH218661:MH218692:MN038126 |
| GII. 7 | A3 | FSESDDFDE | SEQ ID NO: 240 | GII. 7-KF006266 |
| GII. 12 | A3 | GTWEED | SEQ ID NO: 241 | GII. 12-LC342059:KF006267:H0688986:KP064099 |

[Table 1-34]

| | | | | |
|---|---|---|---|---|
| GII. 12 | A3 | GTWEQD | SEQ ID NO: 242 | GII. 12-KJ196294：GQ845370：JQ613568：JQ613569：HQ449728：HQ664990：KC464496：KC464500：KC464499：KC464497：HQ401025：MK754445：MK762627：MK753036：MK754447：MK764041：MK616558：MK616561：MK616560：MK616559：MK355712：MK355713 |
| GII. 12 | A3 | GTWEEN | SEQ ID NO: 243 | GII. 12-KJ196282:KJ196299:KC464498 |
| GII. 13 | A3 | HSITEHVHP | SEQ ID NO: 244 | GII. 13-KJ196276 |
| GII. 13 | A3 | HSITGDVHH | SEQ ID NO: 245 | GII. 13-AB809973:AB809974 |
| GII. 13 | A3 | HSITENVHP | SEQ ID NO: 246 | GII. 13-AB809993：AB809997：AB809990：AB809975：AB809976：AB809994：AB809977：AB809992：AB809985：AB809996：AB809987：AB809978：AB809988：AB809979：AB809986：AB809991：AB810004：AB810001：AB810014：AB810006：AB810011：AB810003：MK753009：MK753008：MH702276：MH702277：KM036380：MK753010：MK754443：MK762560：MK752947：MK753020：MK762559：MH702263：MH702283：MH218651：MK752946：MK762745：MK764014：KY406982：KY406983：KY406984：KY210920：KY947548：MK762565：MK396778：KY406985：KY406986：MG892908：MK775031：MN394543：MN394545 |
| GII. 13 | A3 | HSITEDVRP | SEQ ID NO: 247 | GII. 13-JN899242 |
| GII. 14 | A3 | KSSSND | SEQ ID NO: 248 | GII. 14-JN699038：KJ196278：KJ196297：GU017900：GU017903：GU594162：GU017901：GU017902：GU017904：GU017905：GU017906：GU017907：GU017908：MH279826：MK850443：MK641589：LC556388：LC556389：LC556391：LC556390：LC556392 |
| GII. 14 | A3 | KSSSSD | SEQ ID NO: 249 | GII. 14-MK588004 |
| GII. 17 | A3 | FGSTSDDFQLQQP | SEQ ID NO: 250 | GII. 17-KC597139:JN699043 |
| GII. 17 | A3 | FGSTSTDFQLQQP | SEQ ID NO: 251 | GII. 17-KJ196286 |
| GII. 17 | A3 | FGSESEDFQVGPP | SEQ ID NO: 252 | GII. 17-DQ438972 |
| GII. 17 | A3 | LRISDNDDFQFQP | SEQ ID NO: 253 | GII. 17-MK907801：MK907800：KU557801：KP998539：KT380915：KT780394：KT780395：KT780396：KT780397：KT780398：KT780399：KU561252：KU561253：KR083017：KY424349： |

[Table 1-35]

KY424350:KR020503:KT326180:KU557790:KU557785:KU557786:KU557787:KP698928
:KP698929:KP902566:KP902567:KP902568:KP902569:KP902570:KP902571:KP90257
2:KP902573:KP902574:KP902576:KP902577:KP902578:KT315668:KT315669:KT3156
70:KT315671:KT315672:KT315673:KP864103:KP864104:KU561224:KU561225:KU561
226:KU561227:KU561228:KU561229:KU557797:KU557798:KU557799:KU557802:KU55
7803:KU557806:KU557807:KU557808:KU557809:KU557810:KU557811:KU557815:KU5
57816:KU557817:KU557822:KU557823:KU557824:KU557827:KU557828:KU557829:KU
557833:KU557834:KU557835:KU557846:KU557847:KU557848:KU557849:KU557885:K
Y069114:KX346699:KP902575:LC369228:LC369214:MG692610:LC369229:LC369230:
KT970369:KT970370:MK907791:MK396775:MK907792:MK907793:LC433721:KT149168
:KT149169:MK077685:MK077706:MF918359:KT780400:KT780401:KT780402:KT78040
3:KT780404:KT780405:KT780406:KT780407:KT780408:KT780409:KT780410:KT7804
11:KT780412:KT780413:KT780414:KT780415:KT780416:KU561248:KU561249:KT253
245:KU561254:KU561255:KU561256:KX356908:NC:LC037415:KU557783:KU557784:K
R154230:KR154231:KT992790:KT326181:KT326182:KT992789:KT992786:KT992787:
KT992785:KT992788:KY392867:KY392868:KR052021:KR052019:KR052020:KR052022
:LC349987:LC349988:LC349992:KT346356:LC486739:LC486740:LC486741:LC48674
7:LC486751:LC486761:LC486762:LC486763:LC148844:LC148845:LC148846:LC1488
47:LC148848:LC148849:LC148850:LC148851:LC101820:LC177662:KX171414:KX420
892:KX420891:KP698930:KP698931:KP902579:KP902580:KP902581:KP902582:KP90
2583:KP902584:KP902585:KP902586:KP902587:KP902588:KP902589:KP902590:KT3
15674:KT315675:KT315676:KT315677:KT315678:KT315679:KT315680:KT315681:KT
315682:KT315683:KT315684:KT315685:KT315686:KT315687:KT315688:KT315689:K
T315690:KT315691:KT315692:KT315693:KT315694:KT315695:KT315696:KT315697:
KT315698:KT315699:KT315700:KT315701:KT315702:KT315703:KT315704:KT315705
:KT315706:KT315707:KT315708:KT315709:KT315710:KT315711:KT315712:KT31571

[Table 1-36]

3:KT315714:KT315715:KT315716:KT315717:KT315719:KP864102:KT591501:KU9533
91:KU953392:KU953393:KU561230:KU561231:KU561232:KU561233:KU561234:KU561
235:KU561236:KU561237:KU561238:KU561239:KU561240:KU561241:KU561242:KU56
1243:KU561244:KU561245:KU561246:KU561247:KU557800:KU557804:KU557805:KU5
57812:KU557813:KU557814:KU557818:KU557819:KU557820:KU557821:KU557825:KU
557826:KU557830:KU557831:KU557832:KU557836:KU557837:KU557838:KU557840:K
U557841:KU557842:KU557843:KU557844:KU557845:KU557850:KU557851:KU557852:
KU557853:KU557854:KU557855:KU557856:KU557857:KU557858:KU557859:KU557860
:KU557861:KU557862:KU557863:KU557864:KU557865:KU557866:KU557867:KU55786
8:KU557869:KU557870:KU557871:KU557872:KU557873:KU557874:KU557875:KU5578
76:KU557877:KU557878:KU557879:KU557880:KU557881:KU557882:KU557883:KU557
884:KU557886:KU557887:KU557888:KU557889:KU557890:KU557891:KU557892:KU55
7893:KX244850:KX244851:KX244852:KX244853:KU587626:KU587627:KU587628:KU5
87629:KX134669:KX134670:KX168437:KX168438:KX371107:KX371108:KX371109:KX
371110:KY397953:KY397954:KY397955:KY397956:KY397957:KY397958:KX346700:K
X346701:KX346702:KX346703:KX346704:KX346705:MF172092:MF172093:MF172094:
KY406954:KY406955:KY406956:KY406957:KY406958:KY406959:KY406960:KY407181
:KY407182:KY407183:KY407184:KY407185:KY407186:LC369234:LC369237:LC36923
3:LC369257:LC369242:LC369255:LC369235:LC369222:LC369215:LC369227:LC3692
19:LC369236:LC369244:LC369220:LC369232:LC369238:MN853414:LC369245:LC369
256:LC369224:LC369240:LC369241:MH997861:LC369231:LC369243:LC369225:LC36
9239:LC369249:LC369246:LC369247:LC369248:LC369254:LC369253:LC369252:MK7
89431:LC369218:LC369221:LC369216:LC369217:KT970371:KT970372:KT970373:KT
970374:KT970375:KT970376:KT970377:MH747479:MH747481:MH890538:MH747480:L
C369258:MH747482:MH890539:LC433696:LC433698:LC433699:LC433709:LC433710:
LC433711:LC433712:LC433713:LC433722:LC433723:LC433724:LC433726:LC433727

[Table 1-37]

EP 4 317 436 A1

:LC433729:LC433730:LC433731:LC433732:KX424646:KX424647:KX424648:KX42464
9:KX424650:KT149170:KT149171:KT149172:KT149173:KT149174:KT149175:KT1491
76:KX216782:KX216783:KX216784:KX216785:KX216786:KX216788:KX216789:KX216
790:KX216791:KX216793:KX216794:KX216795:KX216796:KX216797:KX216798:KX21
6799:KX216800:KX216801:KX216802:KX216803:KX216804:KX216805:KX216806:MF0
73239:MF073240:MF073241:MK077686:MK077687:MK077688:MK077689:MK077690:MK
077691:MK077701:MK077702:MK077703:MK077704:MK077705:MK077708:LC349986:L
C349989:LC349990:LC349991:LC349993:LC486742:LC486746:LC486748:LC486749:
LC486750:LC486752:LC486754:LC486764:LC486765:LC486766:LC148852:LC148853
:LC148854:LC148855:LC148856:KX420895:KX420894:KX420893:KU953394:KU95339
5:KU953396:KU953397:KU953398:KX244854:KX134671:KY069115:KX168439:KX1684
40:KX168441:KX168442:KX168443:KX168444:KX168445:KX168446:KX168447:KX168
448:KX168449:KX168450:KX168451:KX168452:KX168453:KX168454:KX168455:KX16
8456:KX371111:KX371112:MF172095:MF172096:KX989474:KX989475:KX989476:KX9
89477:KX989478:KY406961:KY406962:KY406963:KY406964:KY406965:KY406966:KY
406967:KY406968:KY406969:KY406970:KY406971:KY406972:KY406973:KY406975:K
Y406976:KY406977:KY406978:KY406979:KY407203:KY407204:KY407205:KY407206:
KU555841:KY905330:MH746922:MH746923:MH746924:MH746925:LC318746:LC318745
:LC318747:LC318748:LC333866:LC333867:LC333868:LC333869:LC333870:LC33387
1:LC333872:LC333873:LC333874:LC333875:LC333876:LC333877:LC333878:LC3338
79:LC333880:LC333881:LC333882:LC333883:LC333884:LC333885:LC433716:LC433
718:LC433719:LC433733:LC433734:LC433735:LC433736:MF073242:MF073243:MF07
3244:MF073245:MF073246:MF073247:MF073248:MF073249:MF073250:MF073254:MH3
75801:MH375802:MH375803:MH375804:MH375805:MH572223:MH572224:MH572225:MH
572226:MH572227:MH572228:MH572229:MH572230:MH572231:MK077707:MK077709:M
K077710:MK077712:MK077713:LC318750:MF421538:MF421539:MF421540:MF421541:

[Table 1-38]

MF421542:LC258403:LC311767:LC311768:LC311769:LC311770:LC311771:LC311772 :LC311773:LC486743:LC486744:LC486745:LC486755:LC486756:LC486757:LC48675 8:LC486759:LC486760:LC486767:LC486768:LC486769:MK789432:MK789433:LC3187 53:LC318754:LC318752:LC333886:LC333888:LC333890:LC333891:LC333892:LC333 894:LC333896:LC333897:LC333899:LC333900:LC318755:LC318756:LC318757:LC31 8758:LC333895:MH375806:MK077692:MK077693:MK077711:MK077714:MH375813

| GII.17 | A3 | FRSNDNDFQLQP | SEQ ID NO: 254 | GII.17-LC433694:KU561250:KU561251:LC043168:LC043167:KU557788:KJ156329:LC486737:KU557839:MK282256:MK282258:LC433720:MK077684:LC043139:LC043305:LC486738:KT285173:KP902563:KP902564:KP902565:LC433695:AB983218:KX171419:KX171418:KX171416:KY905332 |
| GII.17 | A3 | FRSNDDDFQLQP | SEQ ID NO: 255 | GII.17-MK282257:KX171413:KX171412:KX171417:KU587625:MK907790 |
| GII.17 | A3 | FGSTSEDFIVGQP | SEQ ID NO: 256 | GII.17-MH218591:KT589391:KT346358:KY406980:KY406981:MH375799:LC333898:MH375809:MH375810:MH375811: MH375814 |
| GII.17 | A3 | LRISDNDDFQFHP | SEQ ID NO: 257 | GII.17-KX024652 |
| GII.17 | A3 | FRSNDDDFQWQP | SEQ ID NO: 258 | GII.17-KX171415 |
| GII.17 | A3 | LRISNNDDFQFQP | SEQ ID NO: 259 | GII.17-KT315718:KY406974:LC486770:LC333887:LC333889:LC333893:LC333901:MH375808:MH572232:MK077694:MK077696:MK077697:MK077695:MK077698:MK077699:MK077700:MN394546 |
| GII.17 | A3 | FGSNDNDFQLQP | SEQ ID NO: 260 | GII.17-L0369251 |
| GII.17 | A3 | FRISDNDDFQFQP | SEQ ID NO: 261 | GII.17-KX216792 |
| GII.17 | A3 | LRISDNDDFQLQP | SEQ ID NO: 262 | GII.17-LC486753 |
| GII.17 | A3 | FKISSDDDFQLQP | SEQ ID NO: 263 | GII.17-MH218689 |
| GII.17 | A3 | LRISDNNDFQFQP | SEQ ID NO: 264 | GII.17-MF073251:MF073252:MF073253 |
| GII.17 | A3 | FRISNNDDFQFQP | SEQ ID NO: 265 | GII.17-MG995040 |

[Table 1-39]

| | | | | |
|---|---|---|---|---|
| GII.21 | A3 | HQVQGDI | SEQ ID NO: 266 | GII.21-GU138162:GU138163:HM590541:GU138176:GU138177:HM590519:KJ196284:JN899245 |
| GII.21 | A3 | HQVQGDT | SEQ ID NO: 267 | GII.21-MH702268:MH702279:MK396776:MH702281:MH702280:MH702278:MH702282:MH702264:MK396773:KR921935:KR921936:KR921937:MH702285:MK396771:KX079488:KT962982:KR921938:KR921939:KR921940:KR921941:KR921942:KY406949:KY406950:MH702257:KY210925:KY406951:KY406952:KY406953:KY407199:KY407200:KY407201:KY407202 |
| GII.1 | D | FNTDH | SEQ ID NO: 268 | GII.1-JX289822 |
| GII.1 | D | YDTGH | SEQ ID NO: 269 | GII.1-JN797508 |
| GII.1 | D | YDTNH | SEQ ID NO: 270 | GII.1-KC463911:KC464322:KC464323:MH218731:MF668937:MK753033:MK753034:MK616585:MK616584 |
| GII.1 | D | YDTEH | SEQ ID NO: 271 | GII.1-MG572182:MK483908 |
| GII.2 | D | LNDVEHFN | SEQ ID NO: 272 | GII.2-MF405169 |
| GII.2 | D | LNDTEHFN | SEQ ID NO: 273 | GII.2-JX846925:JN699037:AB281081:AB281082:AB281083:AB281084:AB281086:AB281087:AB195225:AB281088:LC209464:LC209435:LC209436:LC209438:LC209437:DQ456824:AB662850:AB662851:AB662852:AB662853:AB281089:AB281090:LC209462:AB662854:AB662856:AB662863:AB662855:AB662858:AB662864:AB662865:LC209463:AB662860:AB662861:AB662866:AB662869:AB535749:AB662862:AB662871:AB662872:AB662873:AB662874:AB662882:AB662883:AB662885:AB662886:LC209480:LC209481:LC209460:LC209465:LC209459:LC209472:LC209473:LC209474:AB662875:AB662877:AB662878:AB662879:AB662887:AB662888:AB662890:AB662891:AB662892:AB662898:AB662901:AB662902:LC209451:LC209449:LC209479:LC209467:LC209452:LC209447:LC209453:LC209471:LC209448:KJ407074:LC209468:LC209478:LC209446:LC145787:MH218733:MK762626:MK775028:MH702265:MH702261:MH702260:MH938341:LC209439:LC209469:MH218697:MH218655:MH218735:MH218736:MH218737:MH218734: |

[Table 1-40]

MH218657:MK729086:MK752944:MK752945:MK753011:MH938338:LC209457:LC209440:KT962983:MG745995:MG745996:MG745997:MG745998:MG745999:MG746000:MG746001:MG746002:MG746003:MG746004:MG746008:MG746009:MG746010:MG746013:MG746014:MG746015:MG746016:MG746017:MG746027:MG746028:MG746029:MG746033:MG746035:MG746036:MG746037:MG746038:MG746040:MK753015:MK789430:MK753007:MK753035:MK764040:MK764042:KY905336:KY905337:KY905338:MK764039:MK753012:MK775029:KY865306:KY865307:MK773583:MK753013:MK764043:MK773581:MK762641:MK773580:MK773582:MK753014:MK753031:MK907802:KY457727:KY457734:KY457731:KY457729:KY457735:LC413793:LC413794:LC413791:MG763365:KY457728:KY457730:KY677827:KY677829:KY677830:KY677831:KY677832:KY677833:KY817742:KY817743:KY817744:KY817745:KY817746:KY817747:MH938342:MH938343:MH938344:MH938345:MH938346:MH938347:MG746046:MG746047:MG746048:MG746049:MG746050:MG746051:MG746052:MG746053:MG746054:MG746055:MG746056:MG746057:MG746058:MG746059:MG746060:MG746061:MG746062:MG746063:MG746064:MG746065:MG746066:MG746067:MG746068:MG746069:MG746070:MG746071:MG746072:MG746073:MG746074:MG746075:MG746076:MG746077:MG746078:MG746079:MG746080:MG746081:MG746082:MG746083:MG746084:MG746085:MG746086:MG746087:MG746088:MG746092:MG746093:MG746094:MG746095:MG746096:MG746097:MG746098:MG746099:MG746100:MG746101:MG746102:MG746103:MG746104:MG746105:MG746106:MG746107:MG746108:MG746121:MG746122:MG746123:MG746124:MG746125:MG746126:MG746127:MG746128:MG746129:MG746133:MG746134:MG746135:MG746136:MG746137:MG746138:MG746139:MG746140:MG746141:MG746147:MG746148:MG746149:MG746150:MG746167:KY421121:KY421122:NC:LC349981:LC349982:LC349983:LC279234:LC279235:LC279236:LC279237:LC279239:LC279240:LC279241:LC279242:LC279243:LC279238:LC213886:LC213887:LC213888:LC213889:LC213890:LC213891:LC213892:LC213893:LC213894:LC213895:LC213896:LC213897:LC213898:LC213899:LC213900:LC213901:LC215413:LC215541

[Table 1-41]

```
4:KY421123:KY421124:KY421125:KY421126:KY421127:KY421128:KY421129:KY4211
30:KY421131:KY421132:KY421133:KY421134:KY421135:KY421136:KY421137:KY421
138:KY421139:KY421140:KY421141:KY421142:KY421143:KY421144:KY421145:KY42
1146:KY421147:KY421150:KY421151:KY421152:KY421153:KY421154:KY421155:KY4
21156:KY421157:LC325213:LC325214:LC325215:LC325216:KY421044:KY485115:KY
485116:KY485117:KY485118:KY485119:KY485120:KY485121:KY485122:KY485123:K
Y485124:KY485125:KY485126:KY407217:KY407218:KY407219:KY407220:KY407221:
KY457580:KY457581:KY457582:KY457583:KY457584:KY457585:KY457586:MG892974
:MG745985:MG745986:MG745987:MG745988:MG745989:MG745990:MG745991:MG74599
2:MG745993:MG745994:MG746005:MG746006:MG746007:MG746011:MG746012:MG7460
18:MG746019:MG746020:MG746021:MG746022:MG746023:MG746024:MG746025:MG746
026:MG746030:MG746031:MG746032:MG746034:MG746039:MG746041:MG746042:MG74
6043:MG746044:MG746045:MH321825:MK614124:MK614125:MK614126:MK614127:MK6
14128:MK614129:MH041321:MH041322:MN493873:MK753016:MK753587:MK762633:MK
752941:MK752939:MK590974:MK762625:MK762631:MK764017:MK752940:MK775030:M
K340748:MK590973:MK762634:MK753021:MK590972:MK614142:LC413795:LC413796:
LC413797:LC413798:LC413799:LC413800:MG763354:MG763355:MG763356:MG763357
:MG763358:MG763359:MG763360:MG763361:MG763362:MG763364:MG763366:MG763336
7:MG763368:MG763369:MG763370:MG763371:MG763372:MG763373:MG763374:MG7633
76:MG763377:KY817748:KY817749:KY817750:KY817751:KY817753:KY817754:KY817
752:MH068791:MH068792:MH068793:MH068794:MH068795:MH068796:MH068797:MH06
8798:MH068799:MH068800:MH068801:MH068802:MH068803:MH068804:MH068805:MHO
68806:MH068807:MH068808:MH068809:MH068810:MH068811:MH068812:MH068813:MH
068814:MH068815:MH068816:MH068817:MH068818:MH068819:MH068820:MH938348:M
H938349:MH938350:MH938351:MH938352:MH938353:MG746109:MG746110:MG746111:
MG746112:MG746113:MG746114:MG746115:MG746116:MG746117:MG746118:MG746119
```

[Table 1-42]

:MG746120:MG746130:MG746131:MG746132:MG746142:MG746143:MG746144:MG74614
5:MG746146:MG746151:MG746152:MG746153:MG746154:MG746155:MG746156:MG7461
57:MG746158:MG746159:MG746160:MG746161:MG746162:MG746163:MG746164:MG746
165:MG746166:MG746168:MG746169:MG746170:MG746171:MG746172:MG746173:MG74
6174:MG746175:MG746176:MG746177:MG746178:MG746179:MG746180:MG746181:MG7
46182:MG746183:MG746184:MG746185:MG746186:MG746187:MG746188:MG746189:MG
746190:MG746191:MG746192:MG746193:MG746194:MG746195:MG746196:MG746197:M
G746198:MG746199:MG746200:MG746201:MG746202:MG746203:MG746204:MG746205:
MG746206:MG746207:MG746208:MG746209:MG746210:MG746211:MG746212:MG746213
:MG746214:MG746215:MG746216:MG746217:MG746218:MG746219:MG746220:MG74622
1:MG746222:MG746224:MG746225:MK614143:MK614144:MK614145:MK614146:MK6141
47:MK614148:MK614149:MK614150:MK614151:MK614152:MK614153:MK614154:MK614
156:MK614159:MK614161:LC349984:LC349985:MK752935:MK614130:MK614131:MK61
4132:MK614133:MK614134:MK614135:MK614136:MK614137:MK764022:MK762628:MK7
73571:MK614139:MK614140:MK614141:LC413801:LC413802:LC413803:LC413804:MK
614155:MK614157:MK614158:MK614160:MN394542:MN394544

| GII. 2 | D | LNDTDHFN | SEQ ID NO: 274 | GII.2-AB281085:LC209454:AB662880:AB662893:AB662897:AB662899:KC464505:LC209433:LC209445:LC209466:LC209432:LC145786:LC145788:LC145789:LC145790:LC145791:LC145792:LC145793:LC145794:LC145795:LC145796:LC145797:MK752949:MH938340:LC209442:LC209455:LC209476:LC209456:LC209444:LC209443:LC209475:LC209477:LC209431:KY457721:LC209450:LC209441:LC209434:LC209470:LC209458:LC228948:LC145798:LC145799:LC145800:LC145801:LC145803:LC145804:LC145805:LC145806:LC145807:LC145808:KY457724:KY457725:MH938339:LC213885 |
| GII. 2 | D | LSDTDHSN | SEQ ID NO: 275 | GII.2-J0320072 |
| GII. 2 | D | LSDTDHFN | SEQ ID NO: 276 | GII.2-KC998960:LC209461:AB662870:AB662881:AB662884:AB662876:AB662889:AB662894:AB662895:AB662896: AB662900:KY457722 |

[Table 1-43]

| | | | | |
|---|---|---|---|---|
| GII. 2 | D | LNDIEHFN | SEQ ID NO: 277 | GII.2-AB662859:AB662867:MH671553:KY677828:MH158635:MH671554:MG746089:MG 746090:MG746091:KY421148: KY421149:MH979229:MK864096 |
| GII. 2 | D | LNDTEHFD | SEQ ID NO: 278 | GII. 2-AB662868 |
| GII. 2 | D | LNDTDHFD | SEQ ID NO: 279 | GII. 2-LC145802 |
| GII. 2 | D | LNDTGHFN | SEQ ID NO: 280 | GII. 2-MH218648 |
| GII. 2 | D | LNDTEYFN | SEQ ID NO: 281 | GII. 2-LC413792 |
| GII. 2 | D | LNDTEHLN | SEQ ID NO: 282 | GII. 2-LC215415 |
| GII. 2 | D | LNDNEHFN | SEQ ID NO: 283 | GII. 2-MG763363 |
| GII. 2 | D | FNDTEHFN | SEQ ID NO: 284 | GII. 2-MG763375 |
| GII. 2 | D | LNDAEHFN | SEQ ID NO: 285 | GII. 2-MG746223 |
| GII. 3 | D | DNEADFQ | SEQ ID NO: 286 | GII.3-KY442319:KY442320:HM072045:HM072046:KC597144:JN699040:JX846924:JN 699039:HM072044:HM072041:DQ093063:KF006265:JN899244:AB365435:KF931243:K F931234:AB758450:JN565063:KF944111:KF944110:KF895841:KT779557:MH702267: MH702287:MK396777:MK396772:MK764020:MK762640:MH702259:KY348698:LC133339 :LC133343:MH218604:MK073886:MH279487 |
| GII. 3 | D | DRETDFQ | SEQ ID NO: 287 | GII. 3-HM072042 |
| GII. 3 | D | DNEVDFQ | SEQ ID NO: 288 | GII. 3-HM072043 |
| GII. 3 | D | DNEAEFQ | SEQ ID NO: 289 | GII. 3-HM072040:AB067542:JQ743333 |
| GII. 3 | D | DREADFQ | SEQ ID NO: 290 | GII.3-KC464324:KC464325:KC464495:JX984948:KF944266:KJ634708:KJ499441:MH 218573:MH218583:MH218584:MH218585:MH218586:MH218589:KY406922:KY406934:K Y407176:KY407177:KY407178:KY407179:KY407180:KY905334:MK773588 |
| GII. 3 | D | DHEADFQ | SEQ ID NO: 291 | GII.3-KF931180:GU292851:AB385634:HM590538:GU980585:KC464326:LC101823:MK 907798:KC464328:KP064097:GU138208:KF931324:KJ184223:KF944065:KC464329:K J184256:KF944203:KF944179:KF944119:KF944147:KX355506:KF895859:KF944227: KF944232:MG892076:KM056394:KY348697:KJ499442:KY407172:KY407173:KY407174 |

[Table 1-44]

:KY407175:MN199033:MH218570:MH218738:MH218571:MH218712:MH218572:MH21857
4:MH218575:MH218576:MH218577:MH218580:MH218581:MH218582:MH218587:MH2185
88:MH218590:MH218732:MH218592:MK907787:KJ499444:KJ499445:MH218594:MH218
597:MH218598:MH218599:MH218601:MH218603:MH218660:MH218668:MH218671:MH21
8672:MH218675:MH218676:MH218677:MH218678:MH218679:MH218680:MH218630:KY4
06923:KY406924:KY406925:KY406926:KY406927:KY406929:KY406930:KY406931:KY
406932:KY406933:KY407190:KY407191:KY407192:KY407193:KY407194:KY887597:K
Y887606:MH218681:MH218682:MH218683:MH218686:MH218688:MH218690:MH218693:
KY887598:KY210918:KY210919:KY406928:KY406935:KY406936:KY407195:KY407196
:KY407197:KY407198:MG892946:MG892954:MG892955

| | | | | |
|---|---|---|---|---|
| GII. 3 | D | DHEEDFQ | SEQ ID NO: 292 | GII. 3-AB385626:AB385627 |
| GII. 3 | D | DREVDFQ | SEQ ID NO: 293 | GII. 3-AB385641 |
| GII. 3 | D | DHEVDFQ | SEQ ID NO: 294 | GII. 3-AB385642:KF895848 |
| GII. 3 | D | DQEADFQ | SEQ ID NO: 295 | GII. 3-KF931267:MH218593 |
| GII. 3 | D | DHETDFQ | SEQ ID NO: 296 | GII. 3-KC464327 |
| GII. 3 | D | DHESDFQ | SEQ ID NO: 297 | GII. 3-KF944165:KF944166:KY767664:KY767665:KY406937:KY406938:KY406939:MG892915:MG892920:MG892911:MG892910:KX989464:KX989465:KX989466:KX989467:KX989468:KY406940:KY406941:KY406942:KY406943:KY406944:KY406945:KY406946:KY406947:MG892952:MG892947:MG892951:MG892949:MG892950:MG892956 |
| GII. 3 | D | DNEREFQ | SEQ ID NO: 298 | GII. 3-KC597140 |
| GII. 3 | D | DHEPDFQ | SEQ ID NO: 299 | GII. 3-KF306213:KJ499443 |
| GII. 3 | D | DHEEHFQ | SEQ ID NO: 300 | GII. 3-MH218578:MH218579 |
| GII. 3 | D | DHEEDFK | SEQ ID NO: 301 | GII. 3-MH218595:MH218596 |
| GII. 3 | D | DREADFE | SEQ ID NO: 302 | GII. 3-MH218600 |
| GII. 3 | D | DHEEDFR | SEQ ID NO: 303 | GII. 3-MH218602:MH218653:MH218654:MH218646 |

[Table 1-45]

| | | | | |
|---|---|---|---|---|
| GII. 3 | D | DNEAHFK | SEQ ID NO: 304 | GII. 3-MH218618:MH218696:MH218717 |
| GII. 3 | D | DSEADFQ | SEQ ID NO: 305 | GII. 3-KT732274 |
| GII. 3 | D | DQESDFQ | SEQ ID NO: 306 | GII. 3-MG892953 |
| GII. 5 | D | VANGHR | SEQ ID NO: 307 | GII. 5-JN699044:KJ196277:KJ196288:KM386680:KM386681:KM036379:KM036378:KU311160 |
| GII. 5 | D | VSDGHR | SEQ ID NO: 308 | GII. 5-HM596590 |
| GII. 6 | D | DNDWH | SEQ ID NO: 309 | GII. 6-KY424345:KY424346:KY424347:KY424348:GU969054:GU969055:GU969056:GU969057:HM633213:AB682736:AB818397:AB818398:AB818399:AB758451:AB685739:AB685740:KJ407072:AB818400:KC464321:MH218642:MH218645:MH218666:MH218667:MH218673:MK301293:MK956198:MK956199:MK956197 |
| GII. 6 | D | DNNWR | SEQ ID NO: 310 | GII. 6-KC576910:JN699035:JN699036:AB078337:DQ093064:AB818404:JN183165:AB685738:KX752057:MH279832:KM461694:KM461693:KP064098:MH279834:MH279831:MH114014 |
| GII. 6 | D | ADDWR | SEQ ID NO: 311 | GII. 6-JN699041:AB818403:HQ169542:MH218650:KY406918:KY406919 |
| GII. 6 | D | ADDWH | SEQ ID NO: 312 | GII. 6-AB818402:AB685742:AB818401:AB685741:JX984945:JX984949:JX989075:JX984953:KY424341:KY424342:KY424343:KY424344:MH218639:MK907789:MK907786:MH702288:MH279837:MH279838:KM036374:KM036373:KM036375:LN854568:KX268709:LC133342:LC133338:KU935739:MH218640:MH218641:MH218719:MN248515:MN248516:MN248517:MN248514:MH279827:MH279839:MH279835:MH279828:MG571778:MH218687:MH702284:MH702286:KY406920:KY406921:KY407213:KY407214:KY407215:KY407216 |
| GII. 6 | D | DDDWH | SEQ ID NO: 313 | GII. 6-MH218643 |
| GII. 6 | D | DDDWR | SEQ ID NO: 314 | GII. 6-MH218644 |
| GII. 7 | D | DTSHP | SEQ ID NO: 315 | GII. 7-JN699042:GQ849129:MH279833:MH279830:GQ849130:GU134965:KJ196295:MH279829:MH218658:MN038126 |

[Table 1-46]

| | | | | |
|---|---|---|---|---|
| GII. 7 | D | DDSHP | SEQ ID NO:316 | GII. 7-KF006266 |
| GII. 7 | D | DASHP | SEQ ID NO:317 | GII. 7-KC832474 |
| GII. 7 | D | DPSHP | SEQ ID NO:318 | GII. 7-MH218661 |
| GII. 7 | D | DNSHP | SEQ ID NO:319 | GII. 7-MH218692 |
| GII. 12 | D | EHEDFS | SEQ ID NO:320 | GII. 12-LC342059 |
| GII. 12 | D | ENEGFN | SEQ ID NO:321 | GII. 12-KJ196294:MK754445:MK762627:MK753036:MK754447:MK764041:MK616558:MK616561:MK616560:MK616559: MK355712:MK355713 |
| GII. 12 | D | DTDDFN | SEQ ID NO:322 | GII. 12-KF006267 |
| GII. 12 | D | EDEGFN | SEQ ID NO:323 | GII. 12-KJ196282:KJ196299 |
| GII. 12 | D | ESEGFN | SEQ ID NO:324 | GII. 12-GQ845370:JQ613568:HQ688986:JQ613569:HQ449728:HQ664990:KC464498:KC464496:KC464500:KC464499: KC464497:HQ401025 |
| GII. 12 | D | EREGFN | SEQ ID NO:325 | GII. 12-KP064099 |
| GII. 13 | D | VNENTPFQ | SEQ ID NO:326 | GII. 13-KJ196276 |
| GII. 13 | D | VNENTPFK | SEQ ID NO:327 | GII. 13-AB809973:AB809974 |
| GII. 13 | D | QNENTPFQ | SEQ ID NO:328 | GII. 13-AB809993:AB809997:AB809990:AB809975:AB809976:AB809994:AB809977:AB809992:AB809985:AB809996:AB809987:AB809978:AB809988:AB809979:AB809986:AB809991:JN899242:AB810004:AB810001:AB810014:AB810006:AB810011:AB810003:MK753009:MK753008:MH702276:MH702277:KM036380:MK753010:MK754443:MK762560:MK752947:MK753020:MK762559:MH702263:MH702283:MH218651:MK752946:MK762745:MK764014:KY406982:KY406983:KY406984:KY210920:KY947548:MK762565:MK396778:KY406985:KY406986:MG892908:MK775031:MN394543:MN394545 |
| GII. 14 | D | DDQHPFR | SEQ ID NO:329 | GII. 14-JN699038:KJ196278:KJ196297:GU017900:GU017903:GU594162:GU017901:GU017902:GU017904:GU017905:GU017906:GU017907:GU017908:MH279826:MK850443:MK588004:MK641589 |

[Table 1-47]

| | | | | |
|---|---|---|---|---|
| GII. 14 | D | DDQHPFK | SEQ ID NO: 330 | GII. 14-LC556388:LC556389:LC556391:LC556390:LC556392 |
| GII. 17 | D | GIKVESGHDFD | SEQ ID NO: 331 | GII. 17-KC597139:JN699043 |
| GII. 17 | D | GIKIESGHEFD | SEQ ID NO: 332 | GII. 17-KJ196286 |
| GII. 17 | D | GIKIETGHSFR | SEQ ID NO: 333 | GII. 17-DQ438972 |
| GII. 17 | D | GVNDDDDGHPFR | SEQ ID NO: 334 | GII. 17-MK907801:MK907800:KU557801:KP998539:KT380915:KT780394:KT780395:KT780396:KT780397:KT780398:KT780399:KU561252:KR083017:KY424349:KY424350:KR020503:KT326180:KU557790:KU557785:KU557786:KU557787:KP698928:KP698929:KP902566:KP902567:KP902568:KP902569:KP902570:KP902571:KP902572:KP902573:KP902574:KP902576:KP902577:KP902578:KT315668:KT315669:KT315670:KT315671:KT315672:KT315673:KP864103:KP864104:KU561224:KU561225:KU561226:KU561227:KU561228:KU561229:KU557797:KU557798:KU557799:KU557802:KU557803:KU557806:KU557807:KU557808:KU557809:KU557810:KU557811:KU557815:KU557816:KU557817:KU557822:KU557823:KU557824:KU557827:KU557828:KU557829:KU557833:KU557834:KU557835:KU557846:KU557847:KU557848:KU557849:KU557885:KY069114:KX346699:KP902575:LC369228:LC369214:MG692610:LC369229:LC369230:KT970369:KT970370:MK907791:MK396775:MK907792:MK907793:LC433721:KT149168:KT149169:MK077685:MK077706:MF918359:KT780400:KT780401:KT780402:KT780403:KT780404:KT780405:KT780406:KT780407:KT780408:KT780409:KT780410:KT780411:KT780412:KT780413:KT780414:KT780415:KT780416:KU561248:KU561249:KT253245:KU561254:KU561255:KU561256:KX356908:NC:LC037415:KU557783:KU557784:KR154230:KR154231:KT992790:KT326181:KT326182:KT992789:KT992786:KT992787:KT992785:KT992788:KY392867:KY392868:KR052021:KR052019:KR052020:KR052022:LC349987:LC349988:LC349992:KT346356:LC486739:LC486740:LC486741:LC486747:LC486751:LC486761:LC486762:LC486763:LC148844:LC148845:LC148846:LC148847:LC148848:LC148849:LC148850:LC148851:LC101820:LC177662:KX171414:KX420892:KX420 |

[Table 1-48]

891:KP698930:KP698931:KP902579:KP902580:KP902581:KP902582:KP902583:KP90
2584:KP902585:KP902586:KP902587:KP902588:KP902589:KP902590:KT315674:KT3
15675:KT315676:KT315677:KT315678:KT315679:KT315680:KT315681:KT315682:KT
315683:KT315684:KT315685:KT315686:KT315687:KT315688:KT315689:KT315690:K
T315691:KT315692:KT315693:KT315694:KT315695:KT315696:KT315697:KT315698:
KT315699:KT315700:KT315701:KT315702:KT315703:KT315704:KT315705:KT315706
:KT315707:KT315708:KT315709:KT315710:KT315711:KT315712:KT315713:KT31571
4:KT315715:KT315716:KT315717:KT315718:KT315719:KP864102:KT591501:KU9533
91:KU953392:KU953393:KU561230:KU561231:KU561232:KU561233:KU561234:KU561
235:KU561236:KU561237:KU561238:KU561239:KU561240:KU561241:KU561242:KU56
1243:KU561244:KU561245:KU561246:KU561247:KU557800:KU557804:KU557805:KU5
57812:KU557813:KU557814:KU557818:KU557819:KU557820:KU557821:KU557825:KU
557826:KU557830:KU557831:KU557832:KU557836:KU557837:KU557838:KU557840:K
U557841:KU557842:KU557843:KU557844:KU557845:KU557850:KU557851:KU557852:
KU557853:KU557854:KU557855:KU557856:KU557857:KU557858:KU557859:KU557860
:KU557861:KU557862:KU557863:KU557864:KU557865:KU557866:KU557867:KU55786
8:KU557869:KU557870:KU557871:KU557872:KU557873:KU557874:KU557875:KU5578
76:KU557877:KU557878:KU557879:KU557880:KU557881:KU557882:KU557883:KU557
884:KU557886:KU557887:KU557888:KU557889:KU557890:KU557891:KU557892:KU55
7893:KX244850:KX244851:KX244852:KX244853:KX587626:KU587627:KU587628:KU5
87629:KX134669:KX134670:KX168437:KX168438:KX371107:KX371108:KX371109:KX
371110:KY397953:KY397954:KY397955:KY397956:KY397957:KY397958:KX346700:K
X346701:KX346702:KX346703:KX346704:KX346705:MF172092:MF172093:MF172094:
KY406954:KY406955:KY406956:KY406957:KY406958:KY406959:KY406960:KY407181
:KY407182:KY407183:KY407184:KY407185:KY407186:LC369234:LC369237:LC36923
3:LC369257:LC369242:LC369255:LC369235:LC369222:LC369215:LC369227:LC3692

[Table 1-49]

19:LC369236:LC369244:LC369220:LC369232:LC369238:MN853414:LC369245:LC369
256:LC369224:LC369240:LC369241:MH997861:LC369231:LC369243:LC369225:LC36
9239:LC369249:LC369247:LC369248:LC369254:LC369253:LC369252:MK789431:LC3
69218:LC369221:LC369216:LC369217:KT970371:KT970372:KT970373:KT970374:KT
970375:KT970376:KT970377:MH747479:MH747481:MH890538:MH747480:LC369258:L
C433696:LC433698:LC433699:LC433709:LC433710:LC433711:LC433712:LC433713:
LC433722:LC433723:LC433724:LC433726:LC433727:LC433729:LC433730:LC433731
:LC433732:KX424646:KX424647:KX424648:KX424649:KX424650:KT149170:KT14917
1:KT149172:KT149173:KT149174:KT149175:KT149176:KX216782:KX216783:KX2167
84:KX216785:KX216786:KX216788:KX216789:KX216790:KX216791:KX216792:KX216
793:KX216794:KX216795:KX216796:KX216797:KX216798:KX216799:KX216800:KX21
6801:KX216802:KX216803:KX216804:KX216805:KX216806:MF073239:MF073240:MF0
73241:MK077686:MK077687:MK077688:MK077689:MK077690:MK077691:MK077701:MK
077702:MK077703:MK077704:MK077705:MK077708:LC349986:LC349989:LC349990:L
C349991:LC349993:LC486742:LC486746:LC486748:LC486749:LC486750:LC486752:
LC486753:LC486754:LC486764:LC486765:LC486766:LC148852:LC148853:LC148854
:LC148855:LC148856:KX420895:KX420894:KX420893:KU953394:KU953395:KU95339
6:KU953397:KU953398:KX244854:KX134671:KY069115:KX168439:KX168440:KX1684
41:KX168442:KX168443:KX168444:KX168445:KX168446:KX168447:KX168448:KX168
449:KX168450:KX168451:KX168452:KX168453:KX168454:KX168455:KX371111:KX37
1112:MF172095:MF172096:KX989474:KX989475:KX989476:KX989477:KX989478:KY4
06961:KY406962:KY406963:KY406964:KY406965:KY406966:KY406967:KY406968:KY
406969:KY406970:KY406971:KY406972:KY406973:KY406974:KY406975:KY406976:K
Y406977:KY406978:KY406979:KY407203:KY407204:KY407205:KY407206:KY905330:
MH746922:MH746923:MH746924:MH746925:LC318746:LC318745:LC318747:LC333866
:LC333867:LC333868:LC333869:LC333870:LC333871:LC333872:LC333873:LC33387

[Table 1-50]

4:LC333875:LC333876:LC333877:LC333878:LC333879:LC333880:LC333881:LC3338

82:LC333883:LC333884:LC333885:LC433716:LC433718:LC433719:LC433733:LC433

734:LC433735:LC433736:MF073242:MF073243:MF073244:MF073245:MF073246:MF07

3247:MF073248:MF073249:MF073250:MF073251:MF073252:MF073253:MF073254:MH3

75801:MH375802:MH375803:MH375804:MH375805:MH572223:MH572224:MH572225:MH

572226:MH572227:MH572228:MH572229:MH572230:MH572231:MK077707:MK077709:M

K077710:MK077712:MK077713:LC318750:MF421538:MF421539:MF421540:MF421541:

MF421542:LC258403:LC311767:LC311768:LC311769:LC311770:LC311771:LC311772

:LC311773:LC486743:LC486744:LC486745:LC486755:LC486756:LC486757:LC48675

8:LC486759:LC486760:LC486767:LC486768:LC486769:LC486770:MK789432:MK7894

33:LC318753:LC318754:LC318752:LC333886:LC333888:LC333890:LC333891:LC333

892:LC333894:LC333896:LC333897:LC333899:LC333900:LC318755:LC318756:LC31

8757:LC318758:MH375806:MK077692:MK077693:MK077711:MK077714:MH375813

| | | | | |
|---|---|---|---|---|
| GII.17 | D | GINDDGDHPFR | SEQ ID NO: 335 | GII.17-LC433694:KU561250:KU561251:LC043168:LC043167:KU557788:KJ156329:LC486737:KU557839:MK282256:MK282258:LC433720:MK077684:LC043139:LC043305:LC486738:KT285173:KX171413:KX171412:KP902563:KP902564:KP902565:LC433695:AB983218:KX171415:KX171416:LC369251:KY905332 |
| GII.17 | D | GINDDDDHPFR | SEQ ID NO: 336 | GII.17-MK282257:KX171419:KX171418 |
| GII.17 | D | GVNDDDDGHTFR | SEQ ID NO: 337 | GII.17-KU561253 |
| GII.17 | D | CINDDDDHPFR | SEQ ID NO: 338 | GII.17-KX171417 |
| GII.17 | D | GINDDGNHPFR | SEQ ID NO: 339 | GII.17-KU587625:MK907790 |
| GII.17 | D | GIKIEDGHAFD | SEQ ID NO: 340 | GII.17-MH218591:KT589391:KT346358:KY406980:KY406981:MH375799:LC333898:MH375809:MH375810:MH375811: MH375814 |
| GII.17 | D | GVNDDNDGPPFR | SEQ ID NO: 341 | GII.17-KX024652 |
| GII.17 | D | GVNDDDDGHSFR | SEQ ID NO: 342 | GII.17-LC369246 |

[Table 1-51]

| | | | | |
|---|---|---|---|---|
| GII. 17 | D | GVNDDDDGNPFR | SEQ ID NO: 343 | GII. 17-MH747482:MH890539 |
| GII. 17 | D | GVNDDDGGHPFR | SEQ ID NO: 344 | GII. 17-KX168456 |
| GII. 17 | D | GVNDDDGHPFR | SEQ ID NO: 345 | GII. 17-KU555841:LC333895 |
| GII. 17 | D | AINEEDDDHPFR | SEQ ID NO: 346 | GII. 17-MH218689 |
| GII. 17 | D | SVNDDDDGHPFR | SEQ ID NO: 347 | GII. 17-LC318748 |
| GII. 17 | D | GVNDDDDDHPFR | SEQ ID NO: 348 | GII. 17-LC333887:LC333889:LC333893:LC333901:MH375808:MH572232:MK077694:MK077696:MK077697:MK077695:MK077698:MK077699:MK077700:MN394546 |
| GII. 17 | D | GVNDDDDGGRPFR | SEQ ID NO: 349 | GII. 17-MG995040 |
| GII. 21 | D | NGNTPFR | SEQ ID NO: 350 | GII. 21-GU138162:GU138163:HM590541:GU138176:GU138177:HM590519:KJ196284:JN899245 |
| GII. 21 | D | NQNTPFK | SEQ ID NO: 351 | GII. 21-MH702268:MH702279:MK396776:MH702281:MH702280:MH702278:MH702282:MH702264:MK396773:KR921935:KR921936:KR921937:MH702285:MK396771:KX079488:KT962982:KR921938:KR921939:KR921940:KR921941:KR921942:KY406949:KY406950:MH702257:KY210925:KY406951:KY406952:KY406953:KY407199:KY407200:KY407201:KY407202 |
| GII. 1 | E | SYSGRLT | SEQ ID NO: 352 | GII. 1-JX289822 |
| GII. 1 | E | NYSGRLT | SEQ ID NO: 353 | |
| GII. 2 | E | RYAGALN | SEQ ID NO: 354 | GII. 1-JN797508:KC463911:KC464322:KC464323:MH218731:MF668937:MK753033:MG572182:MK753034:MK483908: MK616585:MK616584<br><br>GII. 2-MF405169:JX846925:JN699037:AB281081:AB281082:AB281083:AB281084:AB281085:JQ320072:KC998960:AB281086:AB281087:AB195225:AB281088:LC209464:LC209435:LC209436:LC209438:LC209437:DQ456824:AB662850:AB662851:AB662852:AB662853:AB281089:AB281090:LC209462:AB662854:AB662856:AB662863:AB662855:AB662858:AB662864:AB662865:LC209463:AB662859:AB662860:AB662861:AB662866:AB662867:AB662868:AB662869:AB535749:LC209461:AB662862:AB662870:AB662871:AB662872:AB662873:AB662874:AB662881:AB662882:AB662883:AB662884:AB662 |

[Table 1-52]

EP 4 317 436 A1

885:AB662886:LC209480:LC209481:LC209460:LC209465:LC209459:LC209472:LC20
9473:LC209474:LC209454:AB662875:AB662876:AB662877:AB662878:AB662879:AB6
62880:AB662887:AB662888:AB662889:AB662890:AB662891:AB662892:AB662893:AB
662894:AB662895:AB662896:AB662897:AB662898:AB662899:AB662900:AB662901:A
B662902:LC209451:LC209449:LC209479:LC209467:LC209452:LC209447:LC209453:
LC209471:LC209448:KJ407074:KC464505:LC209468:LC209433:LC209445:LC209466
:LC209478:LC209446:LC209432:LC145787:LC145786:LC145788:LC145789:LC14579
0:LC145791:LC145792:LC145793:LC145794:LC145795:LC145796:LC145797:MK7529
49:MH938340:LC209442:LC209455:LC209476:LC209456:LC209444:LC209443:LC209
475:LC209477:LC209431:MH218733:MK762626:MK775028:MH702265:MH702261:MH70
2260:KY457721:KY457722:MH938341:LC209450:LC209439:LC209469:LC209441:LC2
09434:LC209470:LC209458:LC228948:LC145798:LC145799:LC145800:LC145801:LC
145802:LC145803:LC145804:LC145805:LC145806:LC145807:LC145808:MH218697:M
H218648:MH218655:MH218735:MH218736:MH218737:MH218734:MH218657:MK729086:
MK752945:MK752944:MK753011:KY457724:KY457725:MH938338:MH938339:LC209457
:LC209440:LC213885:KT962983:MG745995:MG745996:MG745997:MG745998:MG74599
9:MG746000:MG746001:MG746002:MG746003:MG746004:MG746008:MG746009:MG7460
10:MG746013:MG746014:MG746015:MG746016:MG746017:MG746027:MG746028:MG746
029:MG746033:MG746035:MG746036:MG746037:MG746038:MG746040:MH671553:MK75
3015:MK789430:MK753007:MK753035:MK764040:MK764042:KY905336:KY905337:KY9
05338:MK764039:MK753012:MK775029:KY865306:KY865307:MK773583:MK753013:MK
764043:MK773581:MK762641:MK773580:MK773582:MK753014:MK753031:MK907802:K
Y457727:KY457734:KY457731:KY457729:KY457735:LC413792:LC413793:LC413794:
LC413791:MG763365:KY457728:KY457730:KY677827:KY677828:KY677829:KY677830
:KY677831:KY677832:KY677833:KY817742:KY817743:KY817744:KY817745:KY81774
6:KY817747:MH158635:MH671554:MH938342:MH938343:MH938344:MH938345:MH9383

68

[Table 1-53]

EP 4 317 436 A1

46:MH938347:MG746046:MG746047:MG746048:MG746049:MG746050:MG746051:MG746
052:MG746053:MG746054:MG746055:MG746056:MG746057:MG746058:MG746059:MG74
6060:MG746061:MG746062:MG746063:MG746064:MG746065:MG746066:MG746067:MG7
46068:MG746069:MG746070:MG746071:MG746072:MG746073:MG746074:MG746075:MG
746076:MG746077:MG746078:MG746079:MG746080:MG746081:MG746082:MG746083:M
G746084:MG746085:MG746086:MG746087:MG746088:MG746089:MG746090:MG746091:
MG746092:MG746094:MG746095:MG746096:MG746097:MG746098:MG746099:MG746100
:MG746101:MG746102:MG746103:MG746104:MG746105:MG746106:MG746107:MG74610
8:MG746121:MG746122:MG746123:MG746124:MG746125:MG746126:MG746127:MG7461
28:MG746129:MG746133:MG746134:MG746135:MG746136:MG746137:MG746138:MG746
139:MG746140:MG746141:MG746147:MG746148:MG746149:MG746150:MG746167:KY42
1121:KY421122:NC:LC349981:LC349982:LC349983:LC279234:LC279235:LC279236:
LC279237:LC279239:LC279240:LC279241:LC279242:LC279243:LC279238:LC213886
:LC213887:LC213888:LC213889:LC213890:LC213891:LC213892:LC213893:LC21389
4:LC213895:LC213896:LC213897:LC213898:LC213899:LC213900:LC213901:LC2154
13:LC215414:LC215415:KY421123:KY421124:KY421125:KY421126:KY421127:KY421
128:KY421129:KY421130:KY421132:KY421133:KY421134:KY421135:KY421136:KY42
1137:KY421138:KY421139:KY421140:KY421141:KY421142:KY421143:KY421144:KY4
21145:KY421146:KY421147:KY421148:KY421149:KY421150:KY421151:KY421152:KY
421153:KY421154:KY421155:KY421156:KY421157:LC325213:LC325214:LC325215:L
C325216:KY421044:KY485115:KY485116:KY485117:KY485118:KY485119:KY485120:
KY485121:KY485122:KY485123:KY485124:KY485125:KY485126:KY407217:KY407218
:KY407219:KY407220:KY407221:KY457580:KY457581:KY457582:KY457583:KY45758
4:KY457585:KY457586:MG892974:MG745985:MG745986:MG745987:MG745988:MG7459
89:MG745990:MG745991:MG745992:MG745993:MG745994:MG746005:MG746006:MG746
007:MG746011:MG746012:MG746018:MG746019:MG746020:MG746021:MG746022:MG74

69

[Table 1-54]

6023:MG746024:MG746025:MG746026:MG746030:MG746031:MG746032:MG746034:MG7
46039:MG746041:MG746042:MG746043:MG746044:MG746045:MK321825:MK614124:MK
614125:MK614126:MK614127:MK614128:MK614129:MH041321:MH041322:MN493873:M
K773587:MK753016:MK762633:MK752941:MK752939:MK590974:MK762625:MK762631:
MK764017:MK752940:MK340748:MK590973:MK762634:MK753021:MK590972:MK614142
:LC413795:LC413796:LC413797:LC413798:LC413799:LC413800:MG763354:MG76335
5:MG763356:MG763357:MG763358:MG763359:MG763360:MG763361:MG763362:MG7633
63:MG763364:MG763366:MG763367:MG763368:MG763369:MG763370:MG763371:MG763
372:MG763373:MG763374:MG763375:MG763376:MG763377:KY817748:KY817749:KY81
7750:KY817751:KY817752:KY817753:MH068791:MH068792:MH068793:MH068794:MHO
68795:MH068796:MH068797:MH068798:MH068799:MH068800:MH068801:MH068802:MH
068803:MH068804:MH068805:MH068806:MH068807:MH068808:MH068809:MH068810:M
H068811:MH068812:MH068813:MH068814:MH068815:MH068816:MH068817:MH068818:
MH068819:MH068820:MH938348:MH938349:MH938350:MH938351:MH938352:MH938353
:MG746109:MG746110:MG746111:MG746112:MG746113:MG746114:MG746115:MG74611
6:MG746117:MG746118:MG746119:MG746120:MG746130:MG746131:MG746132:MG7461
42:MG746143:MG746144:MG746145:MG746146:MG746151:MG746152:MG746153:MG746
154:MG746155:MG746156:MG746157:MG746158:MG746159:MG746160:MG746161:MG74
6162:MG746163:MG746164:MG746165:MG746166:MG746168:MG746169:MG746170:MG7
46171:MG746172:MG746173:MG746174:MG746175:MG746176:MG746177:MG746178:MG
746179:MG746180:MG746181:MG746182:MG746183:MG746184:MG746185:MG746186:M
G746187:MG746188:MG746189:MG746190:MG746191:MG746192:MG746193:MG746194:
MG746195:MG746196:MG746197:MG746198:MG746199:MG746200:MG746201:MG746202
:MG746203:MG746204:MG746205:MG746206:MG746207:MG746208:MG746209:MG74621
0:MG746211:MG746212:MG746213:MG746214:MG746215:MG746216:MG746217:MG7462
18:MG746219:MG746220:MG746221:MG746222:MG746223:MG746224:MG746225:MK614

[Table 1-55]

| | | | | |
|---|---|---|---|---|
| | | | | 143:MK614144:MK614145:MK614146:MK614147:MK614148:MK614149:MK614150:MK614151:MK614152:MK614153:MK614154:MK614156:MK614159:MK614161:LC349984:LC349985:MH979229:MK752935:MK614130:MK614131:MK614132:MK614133:MK614134:MK614135:MK614136:MK614137:MK764022:MK762628:MK773571:MK614139:MK614140:MK614141:MK864096:LC413801:LC413802:LC413803:LC413804:MK614155:MK614157:MK614158:MK614160:MN394542 |
| GII. 2 | E | KYAGALN | SEQ ID NO: 355 | GII. 2-MG746093:KY421131:MK775030 |
| GII. 2 | E | RFAGALN | SEQ ID NO: 356 | GII. 2-KY817754 |
| GII. 2 | E | GYAGALN | SEQ ID NO: 357 | GII. 2-MN394544 |
| GII. 3 | E | DYSGQFT | SEQ ID NO: 358 | GII. 3-KY442319:KY442320:HM072045:HM072046:KC597144:JN699040:JX846924:JN699039:HM072044:HM072041:DQ093063:KC464324:KF006265:KC464325:KF931180:GU292851:JN899244:AB365435:AB385634:HM590538:GU980585:KF931243:KF931267:KF931234:LC101823:AB758450:KC464495:JN565063:KF944111:KF944110:KF944203:KC597140:KF895841:KT779557:KF895859:KF944227:KF944232:KF895848:MH702267:MH702287:MK396777:MK396772:KY348697:KJ499441:KJ499442:KY407172:KY407173:KY407174:KY407175:MN199033:MK764020:MK762640:MH702259:KY348698:LC133339:LC133343:KJ499444:KJ499445:KT732274:KY767664:KY406922:KY406923:KY406924:KY406925:KY406926:KY406927:KY406929:KY406930:KY406931:KY406932:KY406933:KY406934:KY406937:KY406938:KY406939:KY407176:KY407177:KY407178:KY407179:KY407180:KY407190:KY407191:KY407192:KY407193:KY407194:KY905334:MG892915:MG892920:MG892911:MG892910:MK073886:KX989464:KX989465:KX989466:KX989467:KX989468:KY406928:KY406935:KY406936:KY406940:KY406941:KY406942:KY406943:KY406944:KY406945:KY406946:KY406947:KY407195:KY407196:KY407197:KY407198:MG892953:MG892952:MG892947:MG892951:MG892949:MG892950:MG892956:MH279487 |
| GII. 3 | E | EYSGQFT | SEQ ID NO: 359 | GII. 3-HM072042 |

[Table 1-56]

| | | | | |
|---|---|---|---|---|
| GII. 3 | E | DYSGQLT | SEQ ID NO: 360 | GII. 3-HM072043 |
| GII. 3 | E | NYSGQFT | SEQ ID NO: 361 | GII. 3-HM072040:AB067542:JQ743333 |
| GII. 3 | E | DYAGQFT | SEQ ID NO: 362 | GII. 3-AB385626:AB385641:AB385642:KC464326:MK907798:KC464328:KP064097:GU138208:KF931324:KJ184223:KF944065:JX984948:KC464329:KJ184256:KF944165:KF944166:KF944179:KF944119:KF944147:KX355506:KF944266:MG892076:KJ634708:KM056394:KF306213:KJ499443:MH218570:MH218738:MH218571:MH218572:MH218574:MH218575:MH218576:MH218577:MH218579:MH218580:MH218581:MH218582:MH218585:MH218587:MH218588:MH218590:MH218592:MK907787:MH218593:MH218594:MH218597:MH218598:MH218599:MH218600:MH218601:MH218603:MH218604:MH218660:MH218668:MH218671:MH218672:MH218675:MH218676:MH218677:MH218678:MH218679:MH218680:MH218630:KY767665:KY887597:KY887606:MH218681:MH218682:MH218683:MH218686:MH218688:MH218690:MH218693:KY887598:KY210918:KY210919:MG892946:MG892954:MG892955:MK773588 |
| GII. 3 | E | NYAGQFT | SEQ ID NO: 363 | GII. 3-AB385627:MH218573:MH218583:MH218584:MH218586:MH218589 |
| GII. 3 | E | DYAGQLT | SEQ ID NO: 364 | GII. 3-KC464327:MH218712:MH218732:MH218653:MH218646 |
| GII. 3 | E | DYAGQIT | SEQ ID NO: 365 | GII. 3-MH218578 |
| GII. 3 | E | NYAGQLT | SEQ ID NO: 366 | GII. 3-MH218595:MH218596:MH218602:MH218654 |
| GII. 3 | E | NYAGQIS | SEQ ID NO: 367 | GII. 3-MH218618 |
| GII. 3 | E | NYAGQIT | SEQ ID NO: 368 | GII. 3-MH218696:MH218717 |
| GII. 5 | E | RYSGALT | SEQ ID NO: 369 | GII. 5-JN699044:KJ196277:KJ196288:KM386680:HM596590:KM386681:KM036379:KM036378:KU311160 |
| GII. 6 | E | DYSGHLT | SEQ ID NO: 370 | GII. 6-KY424345:KY424346:KY424347:KY424348:GU969054:GU969055:GU969056:GU969057:HM633213:AB682736:AB818397:AB818398:AB818399:AB758451:AB685739:AB685740:KJ407072:AB818400:MH218642:MH218645:MH218666:MH218667:MH218673:MK301293 |

[Table 1-57]

| | | | | |
|---|---|---|---|---|
| GII. 6 | E | DYSGRLT | SEQ ID NO: 371 | GII. 6-KC576910:JN699035:JN699036:AB078337:DQ093064:AB818404:JN183165:AB 685738:KX752057:KP064098: MK956198:MK956199:MK956197 |
| GII. 6 | E | EYSGHLT | SEQ ID NO: 372 | GII. 6-JN699041:AB818403:AB818402:AB685742:HQ169542:AB818401:AB685741:JX 984945:JX984949:JX989075:JX984953:KY424341:KY424342:KY424343:KY424344:M H218639:MK907789:MK907786:MH702288:MH279837:MH279838:KM036374:KM036373: KM036375:LN854568:KX268709:LC133342:LC133338:KU935739:MH218640:MH218641 :MH218643:MH218644:MH218650:MH218719:MN248515:MN248516:MN248517:MN24851 4:MH279827:MH279839:MH279835:MH279828:MG571778:KY406918:MH218687:MH7022 84:MH702286:KY406919:KY406920:KY406921:KY407213:KY407214:KY407215:KY407 216 |
| GII. 6 | E | DYSGSLT | SEQ ID NO: 373 | GII. 6-MH279832:KM461694:KM461693:MH279834:MH279831:MH114014 |
| GII. 6 | E | DYPGHLT | SEQ ID NO: 374 | GII. 6-KC464321 |
| GII.7 | E | RYGGHLT | SEQ ID NO: 375 | GII. 7-JN699042:KF006266:KC832474:GQ849129:MH279833:MH279830:GQ849130:GU 134965:KJ196295:MH279829:MH218658:MH218661:MH218692:MN038126 |
| GII. 12, GII. 21 | E | NYSGALT | SEQ ID NO: 376 | GII. 12-LC342059:KJ196294:KF006267:KJ196282:KJ196299:GQ845370:JQ613568:H Q688986:JQ613569:HQ449728:HQ664990:KC464498:KC464496:KC464500:KC464499: KC464497:HQ401025:KP064099:MK754445:MK762627:MK753036:MK754447:MK764041 :MK616558:MK616561:MK616560:MK616559:MK355712:MK355713_GII. 21-GU138162: GU138163:HM590541:GU138176:GU138177:HM590519:KJ196284:MH702268:MH702279 :MK396776:JN899245:MH702281:MH702280:MH702278:MH702282:MH702264:MK39677 3:KR921935:KR921936:KR921937:MH702285:MK396771:KX079488:KT962982:KR9219 38:KR921939:KR921940:KR921941:KR921942:KY406949:KY406950:MH702257:KY210 925:KY406951:KY406952:KY406953:KY407199:KY407200:KY407201:KY407202 |
| GII. 13 | E | HYAGSLA | SEQ ID NO: 377 | GII. 13-KJ196276 |
| GII. 13 | E | HYSGSLA | SEQ ID NO: 378 | GII. 13-AB809973:AB809974 |

[Table 1-58]

| | | | | |
|---|---|---|---|---|
| GII. 13 | E | HYAGALA | SEQ ID NO: 379 | GII.13-AB809993:AB809997:AB809990:AB809975:AB809976:AB809994:AB809977:AB809992:AB809985:AB809996:AB809987:AB809978:AB809988:AB809979:AB809986:AB809991:JN899242:AB810004:AB810001:AB810014:AB810006:AB810011:AB810003:MK753009:MK753008:MH702276:MH702277:KM036380:MK753010:MK754443:MK762560:MK752947:MK753020:MK762559:MH702263:MH702283:MH218651:MK752946:MK762745:MK764014:KY406982:KY406983:KY406984:KY947548:MK762565:MK396778:KY406985:KY406986:MG892908:MK775031:MN394543:MN394545 |
| GII. 13 | E | HYARALA | SEQ ID NO: 380 | GII. 13-KY210920 |
| GII. 14 | E | NYGGHLA | SEQ ID NO: 381 | GII.14-JN699038:KJ196278:KJ196297:GU017900:GU017903:GU594162:GU017901:GU017902:GU017904:GU017905:GU017906:GU017907:GU017908:MH279826:MK850443:MK588004:MK641589:LC556388:LC556389:LC556391:LC556390:LC556392 |
| GII. 17 | E | RYSGHLTL | SEQ ID NO: 382 | GII.17-KC597139:JN699043:KJ196286:MH218591:KT589391:KT346358:KY406980:KY406981:MH375799:LC333898:MH375809:MH375810:MH375811:MH375814 |
| GII. 17 | E | NYSGALTL | SEQ ID NO: 383 | GII. 17-D0438972 |
| GII. 17 | E | NYSGELTL | SEQ ID NO: 384 | GII.17-MK907801:MK907800:KU557801:KP998539:KT380915:KT780394:KT780395:KT780396:KT780397:KT780398:KT780399:KU561252:KU561253:KR083017:KY424349:KY424350:KR020503:KT326180:KU557790:KU557785:KU557786:KU557787:KP698928:KP698929:KP902566:KP902567:KP902568:KP902569:KP902570:KP902571:KP902572:KP902573:KP902574:KP902576:KP902577:KP902578:KT315668:KT315669:KT315670:KT315671:KT315672:KT315673:KP864103:KP864104:KU561224:KU561225:KU561226:KU561227:KU561228:KU561229:KU557797:KU557798:KU557799:KU557802:KU557803:KU557806:KU557807:KU557808:KU557809:KU557810:KU557811:KU557815:KU557816:KU557817:KU557822:KU557823:KU557824:KU557827:KU557828:KU557829:KU557833:KU557834:KU557835:KU557846:KU557847:KU557848:KU557849:KU557885:KY069114:KX346699:KP902575:LC369228:LC369214:MG692610:LC369229:LC369230:KT970369:KT970370:MK907791:MK396775:MK907792:MK907793:LC433721:KT149168 |

[Table 1-59]

:KT149169:MK077685:MK077706:MF918359:KT780400:KT780401:KT780402:KT78040
3:KT780404:KT780405:KT780406:KT780407:KT780408:KT780409:KT780410:KT7804
11:KT780412:KT780413:KT780414:KT780415:KT780416:KU561248:KU561249:KT253
245:KU561254:KU561255:KU561256:KX356908:NC:LC037415:KU557783:KU557784:K
R154230:KR154231:KT992790:KT326181:KT326182:KT992789:KT992786:KT992787:
KT992785:KT992788:KY392867:KY392868:KR052021:KR052019:KR052020:KR052022
:LC349987:LC349988:LC349992:KT346356:KX024652:LC486739:LC486740:LC48674
1:LC486747:LC486751:LC486761:LC486762:LC486763:LC148844:LC148845:LC1488
46:LC148847:LC148848:LC148849:LC148850:LC148851:LC101820:LC177662:KX171
414:KX420892:KX420891:KP698930:KP698931:KP902579:KP902580:KP902581:KP90
2582:KP902583:KP902584:KP902585:KP902586:KP902587:KP902588:KP902589:KP9
02590:KT315674:KT315675:KT315676:KT315677:KT315678:KT315679:KT315680:KT
315681:KT315682:KT315683:KT315684:KT315685:KT315686:KT315687:KT315688:K
T315689:KT315690:KT315691:KT315692:KT315693:KT315694:KT315695:KT315696:
KT315697:KT315698:KT315699:KT315700:KT315701:KT315702:KT315703:KT315704
:KT315705:KT315706:KT315707:KT315708:KT315709:KT315710:KT315711:KT31571
2:KT315713:KT315714:KT315715:KT315716:KT315717:KT315718:KT315719:KP8641
02:KT591501:KU953391:KU953392:KU953393:KU561230:KU561231:KU561232:KU561
233:KU561234:KU561235:KU561236:KU561237:KU561238:KU561239:KU561240:KU56
1241:KU561242:KU561243:KU561244:KU561245:KU561246:KU561247:KU557800:KU5
57804:KU557805:KU557812:KU557813:KU557814:KU557818:KU557819:KU557820:KU
557821:KU557825:KU557826:KU557830:KU557831:KU557832:KU557836:KU557837:K
U557838:KU557840:KU557841:KU557842:KU557843:KU557844:KU557845:KU557850:
KU557851:KU557852:KU557853:KU557854:KU557855:KU557856:KU557857:KU557858
:KU557859:KU557860:KU557861:KU557862:KU557863:KU557864:KU557865:KU55786
6:KU557867:KU557868:KU557869:KU557870:KU557871:KU557872:KU557873:KU5578

[Table 1-60]

```
74:KU557875:KU557876:KU557877:KU557878:KU557879:KU557880:KU557881:KU557
882:KU557883:KU557884:KU557886:KU557887:KU557888:KU557889:KU557890:KU55
7891:KU557892:KU557893:KX244850:KX244851:KX244852:KX244853:KX587626:KU5
87627:KU587628:KU587629:KX134669:KX134670:KX168437:KX168438:KX371107:KX
371108:KX371109:KX371110:KY397953:KY397954:KY397955:KY397956:KY397957:K
Y397958:KX346700:KX346701:KX346702:KX346703:KX346704:KX346705:MF172092:
MF172093:MF172094:KY406954:KY406955:KY406956:KY406957:KY406958:KY406959
:KY406960:KY407181:KY407182:KY407183:KY407184:KY407185:KY407186:LC36923
4:LC369237:LC369233:LC369257:LC369242:LC369255:LC369235:LC369222:LC3692
15:LC369227:LC369219:LC369236:LC369244:LC369220:LC369232:LC369238:MN853
414:LC369245:LC369224:LC369240:LC369241:MH997861:LC369231:LC369243:LC36
9225:LC369239:LC369249:LC369246:LC369247:LC369248:LC369254:LC369253:LC3
69252:MK789431:LC369218:LC369221:LC369216:LC369217:KT970371:KT970372:KT
970373:KT970374:KT970375:KT970376:KT970377:MH747479:MH747481:MH890538:M
H747480:LC369258:MH747482:MH890539:LC433698:LC433699:LC433709:
LC433710:LC433711:LC433712:LC433713:LC433722:LC433723:LC433724:LC433726
:LC433727:LC433729:LC433730:LC433731:LC433732:KX424646:KX424647:KX42464
9:KX424650:KT149170:KT149171:KT149172:KT149173:KT149174:KT149175:KT1491
76:KX216782:KX216783:KX216784:KX216785:KX216786:KX216788:KX216789:KX216
790:KX216791:KX216792:KX216793:KX216794:KX216795:KX216796:KX216797:KX21
6798:KX216799:KX216800:KX216801:KX216802:KX216803:KX216804:KX216805:KX2
16806:MF073239:MF073240:MF073241:MK077686:MK077687:MK077688:MK077689:MK
077690:MK077691:MK077701:MK077702:MK077703:MK077704:MK077705:MK077708:L
C349986:LC349989:LC349990:LC349991:LC349993:LC486742:LC486746:LC486748:
LC486749:LC486750:LC486752:LC486753:LC486754:LC486764:LC486765:LC486766
:LC148852:LC148853:LC148854:LC148855:LC148856:KX420894:KX420895:KX42089
```

[Table 1-61]

3：KU953394：KU953395：KU953396：KU953397：KU953398：KX244854：KX134671：KY0691
15：KX168439：KX168440：KX168441：KX168442：KX168443：KX168444：KX168445：KX168
446：KX168447：KX168448：KX168449：KX168450：KX168451：KX168452：KX168453：KX16
8454：KX168455：KX168456：KX371111：KX371112：MF172095：MF172096：KX989474：KX9
89475：KX989476：KX989477：KX989478：KY406961：KY406962：KY406963：KY406964：KY
406965：KY406966：KY406967：KY406968：KY406969：KY406970：KY406971：KY406972：K
Y406973：KY406974：KY406975：KY406976：KY406977：KY406978：KY406979：KY407203：
KY407204：KY407205：KY407206：KU555841：KY905330：MH746922：MH746923：MH746924
：MH746925：LC318746：LC318745：LC318747：LC318748：LC333866：LC333867：LC33386
8：LC333869：LC333870：LC333871：LC333872：LC333873：LC333874：LC333875：LC3338
76：LC333877：LC333878：LC333879：LC333880：LC333881：LC333882：LC333883：LC333
884：LC333885：LC433716：LC433718：LC433719：LC433733：LC433734：LC433735：LC43
3736：MF073243：MF073244：MF073245：MF073246：MF073247：MF073248：MF073249：MF0
73250：MF073251：MF073252：MF073253：MF073254：MH375801：MH375802：MH375803：MH
375804：MH375805：MH572223：MH572224：MH572225：MH572226：MH572227：MH572228：M
H572229：MH572230：MH572231：MK077707：MK077709：MK077710：MK077712：MK077713：
LC318750：MF421538：MF421539：MF421540：MF421541：MF421542：LC258403：LC311767
：LC311768：LC311769：LC311770：LC311771：LC311772：LC311773：LC486743：LC48674
4：LC486745：LC486755：LC486756：LC486757：LC486758：LC486759：LC486760：LC4867
67：LC486768：LC486769：LC486770：MK789432：MK789433：LC318753：LC318754：LC318
752：LC333886：LC333887：LC333888：LC333889：LC333890：LC333891：LC333892：LC33
3893：LC333894：LC333896：LC333897：LC333899：LC333900：LC333901：LC318755：LC3
18756：LC318757：LC318758：LC333895：MH375806：MH375808：MH572232：MK077692：MK
077693：MK077694：MK077696：MK077697：MK077711：MK077714：MG995040：MH375813：M
K077695：MK077698：MK077699：MK077700：MN394546

| GII. 17 | E | DYSGLLTL | SEQ ID NO: 385 | GII. 17-LC433694:KU561250:KU561251:LC043168:LC043167:KU557788: KJ156329:L |

[Table 1-62]

C486737:KU557839:MK282256:MK282257:MK282258:LC433720:MK077684:LC043139:
LC043305:LC486738:KX171412:KX171417:KP902563:KP902564:KP902565:LC433695
:AB983218:KX171415:KX171416:LC369251:KY905332

| | | | | |
|---|---|---|---|---|
| GII. 17 | E | DYSGPLTL | SEQ ID NO: 386 | GII. 17-KT285173:KX171413:KX171419:KX171418 |
| GII. 17 | E | DYSGVLTL | SEQ ID NO: 387 | GII. 17-KU587625:MK907790:MH218689 |
| GII. 17 | E | NYSGELTS | SEQ ID NO: 388 | GII. 17-LC369256 |
| GII. 17 | E | NYSGEFTL | SEQ ID NO: 389 | GII. 17-KX424648 |
| GII. 17 | E | SYSGELTL | SEQ ID NO: 390 | GII. 17-MF073242 |
| GII. 1 | F1 | DFLANI | SEQ ID NO: 391 | GII. 1-JX289822:JN797508:KC463911:KC464322:KC464323:MH218731:MF668937:MK753033:MG572182:MK753034: MK483908:MK616585:MK616584 |
| GII. 2, GII. 3 | F1 | DFQGRV | SEQ ID NO: 392 | GII. 2-MF405169:JX846925:JN699037:AB281081:AB281082:AB281083:AB281084:AB281085:AB281086:AB281087:AB195225:AB281088:LC209464:LC209435:LC209436:LC209438:LC209437:DQ456824:AB662850:AB662851:AB662852:AB662853:AB281089:AB281090:LC209462:AB662854:AB662856:AB662863:AB662855:AB662858:AB662864:AB662865:LC209463:AB662859:AB662860:AB662861:AB662866:AB662867:AB662868:AB662869:AB535749:LC209461:AB662862:AB662870:AB662871:AB662872:AB662873:AB662874:AB662881:AB662882:AB662883:AB662884:AB662885:AB662886:LC209480:LC209481:LC209460:LC209465:LC209459:LC209472:LC209473:LC209474:LC209454:AB662875:AB662876:AB662877:AB662878:AB662879:AB662880:AB662887:AB662888:AB662889:AB662890:AB662891:AB662892:AB662893:AB662894:AB662895:AB662896:AB662897:AB662898:AB662899:AB662900:AB662901:AB662902:LC209451:LC209449:LC209479:LC209467:LC209452:LC209447:LC209453:LC209471:LC209448:KJ407074:KC464505:LC209468:LC209433:LC209445:LC209466:LC209478:LC209446:LC209432:LC145787:LC145786:LC145788:LC145789:LC145790:LC145791:LC145792:LC145793:LC145794:LC145795:LC145796:LC145797:MK752949:MH938340:LC2094 |

[Table 1-63]

42:LC209455:LC209476:LC209456:LC209444:LC209443:LC209475:LC209477:LC209
431:MH218733:MK762626:MK775028:MH702265:MH702261:MH702260:KY457721:KY45
7722:MH938341:LC209450:LC209439:LC209469:LC209441:LC209434:LC209470:LC2
09458:LC228948:LC145798:LC145799:LC145800:LC145801:LC145802:LC145803:LC
145804:LC145805:LC145806:LC145807:LC145808:MH218697:MH218648:MH218655:M
H218735:MH218736:MH218737:MH218734:MH218657:MK729086:MK752945:MK753011:
KY457724:KY457725:MH938338:MH938339:LC209457:LC209440:LC213885:KT962983
:MG745995:MG745996:MG745997:MG745998:MG745999:MG746000:MG746001:MG74600
2:MG746003:MG746004:MG746008:MG746009:MG746010:MG746013:MG746014:MG7460
15:MG746016:MG746017:MG746027:MG746028:MG746029:MG746033:MG746035:MG746
036:MG746037:MG746038:MG746040:MH671553:MK753015:MK789430:MK753007:MK75
3035:MK764040:MK764042:KY905336:KY905337:KY905338:MK764039:MK753012:MK7
75029:KY865306:KY865307:MK773583:MK753013:MK764043:MK773581:MK762641:MK
773580:MK773582:MK753014:MK753031:MK907802:KY457727:KY457734:KY457731:K
Y457729:KY457735:LC413793:LC413794:LC413791:MG763365:KY457728:KY457730:
KY677827:KY677828:KY677829:KY677830:KY677831:KY677832:KY677833:KY817742
:KY817743:KY817744:KY817745:KY817746:KY817747:MH158635:MH671554:MH93834
2:MH938343:MH938344:MH938345:MH938346:MH938347:MG746046:MG746047:MG7460
48:MG746049:MG746050:MG746051:MG746052:MG746053:MG746054:MG746055:MG746
056:MG746057:MG746058:MG746059:MG746060:MG746061:MG746062:MG746063:MG74
6064:MG746065:MG746066:MG746067:MG746068:MG746069:MG746070:MG746071:MG7
46072:MG746073:MG746074:MG746075:MG746076:MG746077:MG746078:MG746079:MG
746080:MG746081:MG746082:MG746083:MG746084:MG746085:MG746086:MG746087:M
G746088:MG746089:MG746090:MG746091:MG746092:MG746093:MG746094:MG746095:
MG746096:MG746097:MG746098:MG746099:MG746100:MG746101:MG746102:MG746103
:MG746104:MG746105:MG746106:MG746107:MG746108:MG746121:MG746122:MG74612

[Table 1-64]

EP 4 317 436 A1

3:MG746124:MG746125:MG746126:MG746127:MG746128:MG746129:MG746133:MG7461
34:MG746135:MG746136:MG746137:MG746138:MG746139:MG746140:MG746141:MG746
147:MG746148:MG746149:MG746150:MG746167:KY421121:KY421122:NC:LC349981:L
C349982:LC349983:LC279234:LC279235:LC279236:LC279237:LC279239:LC279240:
LC279241:LC279242:LC279243:LC279238:LC213886:LC213887:LC213888:LC213889
:LC213890:LC213891:LC213892:LC213893:LC213894:LC213895:LC213896:LC21389
7:LC213898:LC213899:LC213900:LC213901:LC215413:LC215414:LC215415:KY4211
23:KY421124:KY421125:KY421126:KY421127:KY421128:KY421129:KY421130:KY421
131:KY421132:KY421133:KY421134:KY421135:KY421136:KY421137:KY421138:KY42
1139:KY421140:KY421141:KY421142:KY421143:KY421144:KY421145:KY421146:KY4
21147:KY421148:KY421149:KY421150:KY421151:KY421152:KY421153:KY421154:KY
421155:KY421156:KY421157:LC325213:LC325214:LC325215:LC325216:KY421044:K
Y485115:KY485116:KY485117:KY485118:KY485119:KY485120:KY485121:KY485122:
KY485123:KY485124:KY485125:KY485126:KY407217:KY407218:KY407219:KY407220
:KY407221:KY457580:KY457581:KY457582:KY457583:KY457584:KY457585:KY45758
6:MG892974:MG745985:MG745986:MG745987:MG745988:MG745989:MG745990:MG7459
91:MG745992:MG745993:MG745994:MG746005:MG746006:MG746007:MG746011:MG746
012:MG746018:MG746019:MG746020:MG746021:MG746022:MG746023:MG746024:MG74
6025:MG746026:MG746030:MG746031:MG746032:MG746034:MG746039:MG746041:MG7
46042:MG746043:MG746044:MG746045:MH321825:MK614124:MK614126:MK614127:MK
614128:MK614129:MH041321:MH041322:MN493873:MK773587:MK753016:MK762633:M
K752941:MK752939:MK590974:MK762625:MK762631:MK764017:MK752940:MK775030:
MK340748:MK590973:MK762634:MK753021:MK590972:MK614142:LC413795:LC413796
:LC413797:LC413798:LC413799:LC413800:MG763354:MG763355:MG763356:MG76335
7:MG763358:MG763359:MG763360:MG763361:MG763362:MG763363:MG763364:MG7633
66:MG763367:MG763368:MG763369:MG763370:MG763371:MG763372:MG763373:MG763

[Table 1-65]

374:MG763375:MG763376:MG763377:KY817748:KY817749:KY817750:KY817751:KY81
7753:KY817754:KY817752:MH068791:MH068792:MH068793:MH068794:MH068795:MH0
68796:MH068797:MH068798:MH068799:MH068800:MH068801:MH068802:MH068803:MH
068804:MH068805:MH068806:MH068807:MH068808:MH068809:MH068810:MH068811:M
H068812:MH068813:MH068814:MH068815:MH068816:MH068817:MH068818:MH068819:
MH068820:MH938348:MH938349:MH938350:MH938351:MH938352:MH938353:MG746109
:MG746110:MG746111:MG746112:MG746113:MG746114:MG746115:MG746116:MG74611
7:MG746118:MG746119:MG746120:MG746130:MG746131:MG746132:MG746142:MG7461
43:MG746144:MG746145:MG746146:MG746151:MG746152:MG746153:MG746154:MG746
155:MG746156:MG746157:MG746158:MG746159:MG746160:MG746161:MG746162:MG74
6163:MG746164:MG746165:MG746166:MG746168:MG746169:MG746170:MG746171:MG7
46172:MG746173:MG746174:MG746175:MG746176:MG746177:MG746178:MG746179:MG
746180:MG746181:MG746182:MG746183:MG746184:MG746185:MG746186:MG746187:M
G746188:MG746189:MG746190:MG746191:MG746192:MG746193:MG746194:MG746195:
MG746196:MG746197:MG746198:MG746199:MG746200:MG746201:MG746202:MG746203
:MG746204:MG746205:MG746206:MG746207:MG746208:MG746209:MG746210:MG74621
1:MG746212:MG746213:MG746214:MG746215:MG746216:MG746217:MG746218:MG7462
19:MG746220:MG746221:MG746222:MG746223:MG746224:MG746225:MK614143:MK614
144:MK614145:MK614146:MK614147:MK614148:MK614149:MK614150:MK614151:MK61
4152:MK614153:MK614154:MK614156:MK614159:MK614161:LC349984:LC349985:MH9
79229:MK752935:MK614130:MK614131:MK614132:MK614133:MK614134:MK614135:MK
614136:MK614137:MK764022:MK762628:MK773571:MK614139:MK614140:MK614141:M
K864096:LC413801:LC413802:LC413803:LC413804:MK614155:MK614157:MK614158:
MK614160:MN394542:MN394544_GII.3-MH218618

| GII.2 | F1 | DFEGRV | SEQ ID NO: 393 | GII.2-JQ320072:KC998960 |
| --- | --- | --- | --- | --- |

EP 4 317 436 A1

[Table 1-66]

| | | | | |
|---|---|---|---|---|
| GII. 2, GII. 3 | F1 | DFQGKV | SEQ ID NO:394 | GII. 2-MK752944_GII. 3-KJ634708:MH218573:MH218584:MH218585:MH218586:MH218598:MH218603: MH218696:MH218717 |
| GII. 2 | F1 | DFQGRI | SEQ ID NO:395 | GII. 2-LC413792 |
| GII. 2 | F1 | DFQERV | SEQ ID NO:396 | GII. 2-MK614125 |
| GII. 3 | F1 | DFRGKV | SEQ ID NO:397 | GII. 3-KY442319:KY442320:HM072045:HM072046:KC597144:JN699040:JX846924:JN699039:HM072042:HM072043:HM072044:HM072040:HM072041:AB067542:JQ743333:DQ093063:KC464324:KF006265:KC464325:KF931180:GU292851:AB365435:AB385634:HM590538:GU980585:AB385626:AB385627:AB385641:AB385642:KC464326:KF931243:KF931267:KF931234:KC464327:LC101823:MK907798:KC464328:KP064097:GU138208:KF931324:AB758450:KJ184223:KF944065:KC464495:JN565063:JX984948:KC464329:KF944111:KF944110:KJ184256:KF944165:KF944166:KF944203:KF944179:KF944119:KF944147:KX355506:KF895841:KT779557:KF895859:KF944227:KF944232:KF895848:KF944266:MG892076:KM056394:MH702267:MH702287:MK396777:MK396772:KY348697:KF306213:KJ499441:KJ499442:KJ499443:KY407172:KY407173:KY407174:KY407175:MN199033:MH218570:MH218738:MH218571:MH218712:MH218572:MH218574:MH218575:MH218576:MH218577:MH218578:MH218579:MH218580:MH218581:MH218582:MH218583:MH218587:MH218588:MH218589:MH218590:MH218732:MH218592:MK764020:MK762640:MK907787:MH702259:KY348698:LC133339:LC133343:KJ499444:KJ499445:MH218593:MH218594:MH218595:MH218596:MH218597:MH218599:MH218600:MH218601:MH218604:MH218653:MH218654:MH218660:MH218668:MH218671:MH218672:MH218675:MH218676:MH218677:MH218678:MH218679:MH218680:MH218630:KT732274:KY767664:KY767665:KY406922:KY406923:KY406924:KY406925:KY406926:KY406927:KY406929:KY406930:KY406931:KY406932:KY406933:KY406934:KY406937:KY406938:KY406939:KY407176:KY407177:KY407178:KY407179:KY407180:KY407190:KY407191:KY407192:KY407193:KY407194:KY887597:KY887606:MH218646:MH218681:MH218682:MH218683:MH218686:MH218688:MH218690:MH218693:KY887598:KY905334:KY210918 |

[Table 1-67]

:KY210919:MG892915:MG892920:MG892911:MG892910:MKC73886:KX989464:KX98946
5:KX989466:KX989467:KX989468:KY406928:KY406935:KY406936:KY406940:KY4069
41:KY406942:KY406943:KY406944:KY406945:KY406946:KY406947:KY407195:KY407
196:KY407197:KY407198:MG892946:MG892953:MG892954:MG892955:MG892952:MG89
2947:MG892951:MG892949:MG892950:MG892956:MK773588:MH279487

| GII. 3 | F1 | DFRGKI | SEQ ID NO: 398 | GII. 3-JN899244 |
|---|---|---|---|---|
| GII. 3 | F1 | DFRGRV | SEQ ID NO: 399 | GII. 3-KC597140 |
| GII. 3 | F1 | DFHGKV | SEQ ID NO: 400 | GII. 3-MH218602 |
| GII. 5 | F1 | DFAGEV | SEQ ID NO: 401 | GII. 5-JN699044:KJ196277:KJ196288:KM386680:HM596590:KM386681:KM036379:KM036378:KU311160 |
| GII. 6 | F1 | DFKGAV | SEQ ID NO: 402 | GII. 6-KY424345:KY424346:KY424347:KY424348 |
| GII. 6 | F1 | DFKGTV | SEQ ID NO: 403 | GII. 6-KC576910:JN699035:JN699036:JN699041:AB078337:DQ093064:AB818404:AB818403:AB818402:AB685742:HQ169542:AB818401:AB685741:JN183165:AB685738:GU969054:GU969055:GU969056:GU969057:HM633213:KX752057:AB682736:AB818397:AB818398:AB818399:AB758451:AB685739:AB685740:KJ407072:JX984945:JX984949:MH279832:JX989075:KM461694:KM461693:KP064098:JX984953:MH279834:KY424341:KY424342:KY424343:KY424344:AB818400:KC464321:MH279831:MH218639:MK907789:MK907786:MH702288:MH279837:MH279838:KM036374:KM036373:KM036375:LN854568:KX268709:LC133342:LC133338:KU935739:MH218640:MH218641:MH218643:MH218644:MH218650:MH218719:MN248515:MN248516:MN248517:MN248514:MH218642:MH218645:MH218666:MH218667:MH218673:MH279827:MH279839:MH279835:MH279828:MG571778:KY406918:MH218687:MH702284:MH702286:KY406919:KY406920:KY406921:KY407213:KY407214:KY407215:KY407216:MH114014:MK301293:MK956198:MK956199:MK956197 |
| GII. 7, | F1 | DFRGIL | SEQ ID NO: 404 | GII. 13-AB810014_GII. 21-GU138162:GU138163:HM590541 :GU138176:GU138177:HM5 |

EP 4 317 436 A1

[Table 1-68]

| | | | | |
|---|---|---|---|---|
| GII. 13, GII. 21 | | | | 90519:KJ196284:MH702268:MH702279:MK396776:JN899245:MH702281:MH702280:MH 702278:MH702282:MH702264:MK396773:KR921935:KR921936:KR921937:MH702285:M K396771:KX079488:KT962982:KR921938:KR921939:KR921940:KR921941:KR921942: KY406949:KY406950:MH702257:KY210925:KY406951:KY406952:KY406953:KY407199 :KY407200:KY407201:KY407202_GII.7-KF006266 |
| GII. 7 | F1 | DFQGIL | SEQ ID NO: 405 | GII.7-JN699042:KC832474:GQ849129:MH279833:MH279830:GQ849130:GU134965:KJ 196295:MH279829:  MH218658:MH218661:MH218692:MN038126 |
| GII. 12 | F1 | DFSGKL | SEQ ID NO: 406 | GII.12-LC342059:KJ196294:KJ196282:KJ196299:GQ845370:JQ613568:HQ688986:J Q613569:HQ449728:HQ664990:KC464498:KC464496:KC464500:KC464499:KC464497: HQ401025:KP064099:MK754445:MK762627:MK753036:MK754447:MK764041:MK616558 :MK616561:MK616560:MK616559:MK355712:MK355713 |
| GII. 12 | F1 | DFSGRL | SEQ ID NO: 407 | GII. 12-KF006267 |
| GII. 13 | F1 | DFSGML | SEQ ID NO: 408 | GII. 13-KJ196276:MK753009:MK753008:MK753010:MK754443:MK764014 |
| GII. 13 | F1 | DFSGIL | SEQ ID NO: 409 | GII.13-AB809973:AB809974:AB809993:AB809997:AB809990:AB809975:AB809976:A B809994:AB809977:AB809992:AB809985:AB809996:AB809987:AB809978:AB809988: AB809979:AB809986:AB809991:JN899242:AB810004:AB810001:AB810006:AB810011 :AB810003:MH702276:MH702277:KM036380:MK762560:MK752947:MK753020:MK76255 9:MH702263:MH702283:MH218651:KY406982:KY406983:KY406984:KY210920:KY9475 48:MK396778:KY406985:KY406986:MG892908:MK775031:MN394543:MN394545 |
| GII. 13 | F1 | DFSGLL | SEQ ID NO: 410 | GII. 13-MK752946:MK762745:MK762565 |
| GII. 14 | F1 | DFQAML | SEQ ID NO: 411 | GII.14-JN699038:GU017900:GU017903:GU594162:GU017901:GU017902:GU017904:G U017905:GU017906:GU017907:GU017908:MH279826:MK850443:MK588004:MK641589 |
| GII. 14 | F1 | DFQAVL | SEQ ID NO: 412 | GII. 14-KJ196278:KJ196297:LC556388:LC556389:LC556391:LC556390: LC556392 |
| GII. 17 | F1 | DFTGLL | SEQ ID NO: 413 | GII. 17-KC597139:JN699043:KJ196286:DQ438972:MH218591 :KT589391 :KT346358:K |

84

[Table 1-69]

| GII. 17 | F1 | DFKGVV | SEQ ID NO: 414 | Y406980:KY406981:MH375799:LC333898:MH375809:MH375810:MH375811:MH375814 GII.17-MK907801:MK907800:LC433694:KU561250:KU561251:LC043168:LC043167:KU557788:KJ156329:LC486737:KU557801:KU557839:MK282256:MK282257:MK282258:LC433720:MK077684:KP998539:KT380915:KT780394:KT780395:KT780396:KT780397:KT780398:KT780399:KU561252:KU561253:LC043139:LC043305:KR083017:KY424349:KY424350:KR020503:KT326180:KU557790:KU557785:KU557786:KU557787:LC486738:KT285173:KX171413:KX171412:KX171417:KP698928:KP698929:KP902566:KP902567:KP902568:KP902569:KP902570:KP902571:KP902572:KP902573:KP902574:KP902576:KP902577:KP902578:KT315668:KT315669:KT315670:KT315671:KT315672:KT315673:KP864103:KP864104:KU561224:KU561225:KU561226:KU561227:KU561228:KU561229:KU557797:KU557798:KU557799:KU557802:KU557803:KU557806:KU557807:KU557808:KU557809:KU557810:KU557811:KU557815:KU557816:KU557817:KU557822:KU557823:KU557824:KU557827:KU557828:KU557829:KU557833:KU557834:KU557835:KU557846:KU557847:KU557848:KU557849:KU557885:KY069114:KX346699:KP902575:KP902563:KP902564:KP902565:KU587625:LC369228:LC369214:MG692610:LC369229:LC369230:KT970369:KT970370:MK907790:MK907791:MK396775:MK907792:MK907793:LC433721:LC433695:KT149168:KT149169:MK077685:MK077706:AB983218:MF918359:KT780400:KT780401:KT780402:KT780403:KT780404:KT780405:KT780406:KT780407:KT780408:KT780409:KT780410:KT780411:KT780412:KT780413:KT780414:KT780415:KT780416:KU561248:KU561249:KT253245:KU561254:KU561255:KU561256:KX356908:NC:LC037415:KU557783:KU557784:KR154230:KR154231:KT992790:KT326181:KT326182:KT992789:KT992786:KT992787:KT992785:KT992788:KY392867:KY392868:KR052021:KR052019:KR052020:KR052022:LC349987:LC349988:LC349992:KT346356:KX024652:LC486739:LC486740:LC486741:LC486747:LC486751:LC486761:LC486762:LC486763:LC148844:LC148845:LC148846:LC148847:LC148848:LC148849:LC148850:LC148851:LC101820:LC177662:KX171414:KX171415:KX171419:KX420892:K |

[Table 1-70]

X420891：KX171418：KX171416：KP698930：KP698931：KP902579：KP902580：KP902581：
KP902582：KP902583：KP902584：KP902585：KP902586：KP902587：KP902588：KP902589
：KP902590：KT315674：KT315675：KT315676：KT315677：KT315678：KT315679：KT31568
0：KT315681：KT315682：KT315683：KT315684：KT315685：KT315686：KT315687：KT3156
88：KT315689：KT315690：KT315691：KT315692：KT315693：KT315694：KT315695：KT315
696：KT315697：KT315698：KT315699：KT315700：KT315701：KT315702：KT315703：KT31
5704：KT315705：KT315706：KT315707：KT315708：KT315709：KT315710：KT315711：KT3
15712：KT315713：KT315714：KT315715：KT315716：KT315717：KT315718：KT315719：KP
864102：KT591501：KU953391：KU953392：KU953393：KU561230：KU561231：KU561232：K
U561233：KU561234：KU561235：KU561236：KU561237：KU561238：KU561239：KU561240：
KU561241：KU561242：KU561243：KU561244：KU561245：KU561246：KU561247：KU557800
：KU557804：KU557805：KU557812：KU557813：KU557814：KU557818：KU557819：KU55782
0：KU557821：KU557825：KU557826：KU557830：KU557831：KU557832：KU557836：KU5578
37：KU557838：KU557840：KU557841：KU557842：KU557843：KU557844：KU557845：KU557
850：KU557851：KU557852：KU557853：KU557854：KU557855：KU557856：KU557857：KU55
7858：KU557859：KU557860：KU557861：KU557862：KU557863：KU557864：KU557865：KU5
57866：KU557867：KU557868：KU557869：KU557870：KU557871：KU557872：KU557873：KU
557874：KU557875：KU557876：KU557877：KU557878：KU557879：KU557880：KU557881：K
U557882：KU557883：KU557884：KU557886：KU557887：KU557888：KU557889：KU557890：
KU557891：KU557892：KU557893：KX244850：KX244851：KX244852：KX244853：KU587626
：KU587627：KU587628：KU587629：KX134669：KX134670：KX168437：KX168438：KX37110
7：KX371108：KX371109：KX371110：KY397953：KY397954：KY397955：KY397956：KY3979
57：KY397958：KX346700：KX346701：KX346702：KX346703：KX346704：KX346705：MF172
092：MF172093：MF172094：KY406954：KY406955：KY406956：KY406957：KY406958：KY40
6959：KY406960：KY407181：KY407182：KY407183：KY407184：KY407185：KY407186：LC3
69234：LC369237：LC369233：LC369257：LC369242：LC369255：LC369235：LC369222：LC

[Table 1-71]

369215:LC369227:LC369219:LC369236:LC369244:LC369220:LC369232:LC369238:M
N853414:LC369245:LC369256:LC369224:LC369240:LC369241:MH997861:LC369231:
LC369243:LC369225:LC369239:LC369249:LC369246:LC369247:LC369248:LC369254
:LC369253:LC369252:LC369251:KY905332:MK789431:LC369218:LC369221:LC36921
6:LC369217:KT970371:KT970372:KT970373:KT970374:KT970375:KT970376:KT9703
77:MH747479:MH747481:MH890538:MH747480:LC369258:MH747482:MH890539:LC433
696:LC433698:LC433699:LC433709:LC433710:LC433711:LC433712:LC433713:LC43
3722:LC433723:LC433724:LC433726:LC433727:LC433729:LC433730:LC433731:LC4
33732:KX424646:KX424647:KX424648:KX424649:KX424650:KT149170:KT149171:KT
149172:KT149173:KT149174:KT149175:KT149176:KX216782:KX216783:KX216784:K
X216785:KX216786:KX216788:KX216789:KX216790:KX216791:KX216792:KX216793:
KX216794:KX216795:KX216796:KX216797:KX216798:KX216799:KX216800:KX216801
:KX216802:KX216803:KX216804:KX216805:KX216806:MF073239:MF073240:MF07324
1:MK077686:MK077687:MK077688:MK077689:MK077690:MK077691:MK077701:MK0777
02:MK077703:MK077704:MK077705:MK077708:LC349986:LC349989:LC349990:LC349
991:LC349993:LC486742:LC486746:LC486748:LC486749:LC486750:LC486752:LC48
6753:LC486754:LC486764:LC486765:LC486766:LC148852:LC148853:LC148854:LC1
48855:LC148856:KX420895:KX420894:KX420893:KU953394:KU953395:KU953396:KU
953397:KU953398:KX244854:KX134671:KY069115:KX168439:KX168440:KX168441:K
X168442:KX168443:KX168444:KX168445:KX168446:KX168447:KX168448:KX168449:
KX168450:KX168451:KX168452:KX168453:KX168454:KX168455:KX168456:KX371111
:KX371112:MF172095:MF172096:KX989474:KX989475:KX989476:KX989477:KX98947
8:KY406961:KY406962:KY406963:KY406964:KY406965:KY406966:KY406967:KY4069
68:KY406969:KY406970:KY406971:KY406972:KY406973:KY406974:KY406975:KY406
976:KY406977:KY406978:KY406979:KY407203:KY407204:KY407205:KY407206:KU55
5841:KY905330:MH746922:MH746923:MH746924:MH746925:LC318746:LC318745:LC3

[Table 1-72]

| Genotype | | | | |
|---|---|---|---|---|
| GII. 17 | F1 | DFKGFV | SEQ ID NO: 415 | GII. 17-MH218689:MH218747:LC318748:LC333866:LC333867:LC333868:LC333869:LC333870:LC333871:LC333872:LC333873:LC333874:LC333875:LC333876:LC333877:LC333878:LC333879:LC333880:LC333881:LC333882:LC333883:LC333884:LC333885:LC433716:LC433718:LC433719:LC433733:LC433734:LC433735:LC433736:MF073242:MF073243:MF073244:MF073245:MF073246:MF073247:MF073248:MF073249:MF073250:MF073251:MF073252:MF073253:MF073254:MH375801:MH375802:MH375803:MH375804:MH375805:MH572223:MH572224:MH572225:MH572226:MH572227:MH572228:MH572229:MH572230:MH572231:MK077707:MK077709:MK077710:MK077712:MK077713:LC318750:MF421538:MF421539:MF421540:MF421541:MF421542:LC258403:LC311767:LC311768:LC311769:LC311770:LC311771:LC311772:LC311773:LC486743:LC486744:LC486745:LC486755:LC486756:LC486757:LC486758:LC486759:LC486760:LC486767:LC486768:LC486769:LC486770:MK789432:MK789433:LC318753:LC318754:LC318752:LC333886:LC333887:LC333888:LC333889:LC333890:LC333891:LC333892:LC333893:LC333894:LC333896:LC333897:LC333899:LC333900:LC333901:LC318755:LC318756:LC318757:LC318758:LC333895:MH375806:MH375808:MH572232:MK077692:MK077693:MK077694:MK077696:MK077697:MK077711:MK077714:MG995040:MH375813:MK077695:MK077698:MK077699:MK077700:MN394546 |
| GII. 1, GII. 3, GII. 12, GII. 17 | F2 | QWAL | SEQ ID NO: 416 | GII. 12-KF006267_GII. 17-KC597139:JN699043:KJ196286_GII. 3-JX289822_GII. 3-AB385641:AB385642:KP064097:KF944165:KF944166:KF306213:MH218573:MH218584:MH218585:MH218586:MH218592:MH218593:MH218598:MH218599:MH218600:MH218603:MH218618:MH218660:MH218668:MH218671:MH218672:MH218675:MH218676:MH218677:MH218678:MH218679:MH218680:MH218696:MH218717:KY767665:MH218646:MH218681:MH218682:MH218683:MH218686:MH218688:MH218690:MH218693:MG892954 |
| GII. 1, GII. 2, | F2 | QWVL | SEQ ID NO: 417 | GII. 13-KJ196276:AB809973:AB809974:AB809975:AB809990:AB809997:AB809975:AB809976:AB809994:AB809977:AB809992:AB809985:AB809996:AB809987:AB809978: |

[Table 1-73]

EP 4 317 436 A1

| GII. 3, GII. 7, GII. 13 | | | | AB809988:AB809979:AB809986:AB809991:JN899242:AB810004:AB810001:AB810014:AB810006:AB810011:AB810003:MK753009:MK753008:MH702276:MH702277:KM036380:MK753010:MK754443:MK762560:MK752947:MK753020:MK762559:MH702263:MH702283:MH218651:MK752946:MK762745:MK764014:KY406982:KY406983:KY406984:KY210920:KY947548:MK762565:MK396778:KY406985:KY406986:MG892908:MK775031:MN394543:MN394545_GII. 1-JN797508:MG572182:MK483908_GII. 2-LC145792:LC209443_GII. 3-KJ499443_GII. 7-KF006266:KC832474:GQ849129:MH279833:MH279830:GQ849130:GU134965:KJ196295:MH279829:MH218658:MH218661:MH218692:MN038126 |
|---|---|---|---|---|
| GII. 1, GII. 3 | F2 | QWIL | SEQ ID NO: 418 | GII. 1-KC463911:KC464322:KC464323:MH218731:MF668937:MK753033:MK753034:MK616585:MK616584_GII. 3-DQ093063 |
| GII. 2 | F2 | QWVV | SEQ ID NO: 419 | GII. 2-MF405169:JX846925:JN699037:AB281081:AB281082:AB281083:AB281084:AB281085:JQ320072:KC998960:AB281086:AB281087:AB195225:AB281088:LC209464:LC209435:LC209436:LC209438:LC209437:DQ456824:AB662850:AB662851:AB662852:AB662853:AB281089:AB281090:LC209462:AB662854:AB662856:AB662863:AB662855:AB662858:AB662864:AB662865:LC209463:AB662859:AB662860:AB662861:AB662866:AB662867:AB662868:AB662869:AB535749:LC209461:AB662862:AB662870:AB662871:AB662872:AB662873:AB662874:AB662881:AB662882:AB662883:AB662884:AB662885:AB662886:LC209480:LC209481:LC209460:LC209465:LC209459:LC209472:LC209473:LC209474:LC209454:AB662875:AB662876:AB662877:AB662878:AB662879:AB662880:AB662887:AB662888:AB662889:AB662890:AB662891:AB662892:AB662893:AB662894:AB662895:AB662896:AB662897:AB662898:AB662899:AB662900:AB662901:AB662902:LC209451:LC209449:LC209479:LC209467:LC209452:LC209447:LC209453:LC209471:LC209448:KJ407074:KC464505:LC209468:LC209433:LC209445:LC209466:LC209478:LC209446:LC209432:LC145787:LC145786:LC145788:LC145789:LC145790:LC145791:LC145793:LC145794:LC145795:LC145796:LC145797:MK752949:MH938340:LC209442:LC209455:LC209476:LC209456:LC209444:LC209475:LC209477:LC209 |

[Table 1-74]

431:MH218733:MK762626:MK775028:MH702265:MH702261:MH702260:KY457721:KY45
7722:MH938341:LC209450:LC209439:LC209469:LC209441:LC209434:LC209470:LC2
09458:LC228948:LC145798:LC145799:LC145800:LC145801:LC145802:LC145803:LC
145804:LC145805:LC145806:LC145807:LC145808:MH218697:MH218648:MH218655:M
H218735:MH218736:MH218737:MH218734:MH218657:MK729086:MK752945:MK752944:
MK753011:KY457724:KY457725:MH938338:MH938339:LC209457:LC209440:LC213885
:KT962983:MG745995:MG745996:MG745997:MG745998:MG745999:MG746000:MG74600
1:MG746002:MG746003:MG746004:MG746008:MG746009:MG746010:MG746013:MG7460
14:MG746015:MG746016:MG746017:MG746027:MG746028:MG746029:MG746033:MG746
035:MG746037:MG746038:MG746040:MH671553:MK753015:MK789430:MK753007:MK75
3035:MK764040:MK764042:KY905336:KY905337:KY905338:MK764039:MK753012:MK7
75029:KY865306:KY865307:MK773583:MK753013:MK764043:MK773581:MK762641:MK
773580:MK773582:MK753014:MK753031:MK907802:KY457727:KY457734:KY457731:K
Y457729:KY457735:LC413792:LC413793:LC413794:LC413791:MG763365:KY457728:
KY457730:KY677827:KY677828:KY677829:KY677830:KY677831:KY677832:KY677833
:KY817742:KY817743:KY817744:KY817745:KY817746:KY817747:MH158635:MH67155
4:MH938342:MH938343:MH938344:MH938345:MH938346:MH938347:MG746046:MG7460
47:MG746048:MG746049:MG746050:MG746051:MG746052:MG746053:MG746054:MG746
056:MG746057:MG746058:MG746059:MG746060:MG746061:MG746062:MG746063:MG74
6064:MG746065:MG746066:MG746067:MG746068:MG746069:MG746070:MG746071:MG7
46072:MG746073:MG746074:MG746075:MG746076:MG746077:MG746078:MG746079:MG
746080:MG746081:MG746082:MG746083:MG746084:MG746085:MG746086:MG746087:M
G746088:MG746089:MG746090:MG746091:MG746092:MG746093:MG746094:MG746095:
MG746096:MG746097:MG746098:MG746099:MG746100:MG746101:MG746102:MG746103
:MG746104:MG746105:MG746106:MG746107:MG746108:MG746121:MG746122:MG74612
3:MG746124:MG746125:MG746126:MG746127:MG746128:MG746129:MG746133:MG7461

[Table 1-75]

34:MG746135:MG746136:MG746137:MG746138:MG746139:MG746140:MG746141:MG746
147:MG746148:MG746149:MG746150:MG746167:KY421121:KY421122:NC:LC349981:L
C349982:LC349983:LC279234:LC279235:LC279236:LC279237:LC279239:LC279240:
LC279241:LC279242:LC279243:LC279238:LC213886:LC213887:LC213888:LC213889
:LC213890:LC213891:LC213892:LC213893:LC213894:LC213895:LC213896:LC21389
7:LC213898:LC213899:LC213900:LC213901:LC215413:LC215414:LC215415:KY4211
23:KY421124:KY421125:KY421126:KY421127:KY421128:KY421129:KY421130:KY421
131:KY421132:KY421133:KY421134:KY421135:KY421136:KY421137:KY421138:KY42
1139:KY421140:KY421141:KY421142:KY421143:KY421144:KY421145:KY421146:KY4
21147:KY421148:KY421149:KY421150:KY421151:KY421152:KY421153:KY421154:KY
421155:KY421156:KY421157:LC325213:LC325214:LC325215:LC325216:KY421044:K
Y485115:KY485116:KY485117:KY485118:KY485119:KY485120:KY485121:KY485122:
KY485123:KY485124:KY485125:KY485126:KY407217:KY407218:KY407219:KY407220
:KY407221:KY457580:KY457581:KY457582:KY457583:KY457584:KY457585:KY45758
6:MG892974:MG745985:MG745986:MG745987:MG745988:MG745989:MG745990:MG7459
91:MG745992:MG745993:MG745994:MG746005:MG746006:MG746007:MG746011:MG746
012:MG746018:MG746019:MG746020:MG746021:MG746022:MG746023:MG746024:MG74
6025:MG746026:MG746030:MG746031:MG746032:MG746034:MG746039:MG746041:MG7
46042:MG746043:MG746045:MH321825:MK614124:MK614125:MK614126:MK614127:MK
614128:MK614129:MH041321:MH041322:MN493873:MK773587:MK753016:MK762633:M
K752941:MK752939:MK590974:MK762625:MK762631:MK764017:MK752940:MK775030:
MK340748:MK590973:MK762634:MK753021:MK590972:MK614142:LC413795:LC413796
:LC413797:LC413798:LC413799:LC413800:MG763354:MG763355:MG763356:MG76335
7:MG763358:MG763359:MG763360:MG763361:MG763362:MG763363:MG763364:MG7633
66:MG763367:MG763368:MG763369:MG763370:MG763371:MG763372:MG763373:MG763
374:MG763375:MG763376:MG763377:KY817748:KY817749:KY817750:KY817751:KY81

[Table 1-76]

7753:KY817754:KY817752:MH068791:MH068792:MH068793:MH068794:MH068795:MHO
68796:MH068797:MH068798:MH068799:MH068800:MH068801:MH068802:MH068803:MH
068804:MH068805:MH068806:MH068807:MH068808:MH068809:MH068810:MH068811:M
H068812:MH068813:MH068814:MH068815:MH068816:MH068817:MH068818:MH068819:
MH068820:MH938348:MH938349:MH938350:MH938351:MH938352:MH938353:MG746109
:MG746110:MG746111:MG746112:MG746113:MG746114:MG746115:MG746116:MG74611
7:MG746118:MG746119:MG746120:MG746130:MG746131:MG746132:MG746142:MG7461
43:MG746144:MG746145:MG746146:MG746151:MG746152:MG746153:MG746154:MG746
155:MG746156:MG746157:MG746158:MG746159:MG746160:MG746161:MG746162:MG74
6163:MG746164:MG746165:MG746166:MG746168:MG746169:MG746170:MG746171:MG7
46172:MG746173:MG746174:MG746175:MG746176:MG746177:MG746178:MG746179:MG
746180:MG746181:MG746182:MG746183:MG746184:MG746185:MG746186:MG746187:M
G746188:MG746189:MG746190:MG746191:MG746192:MG746193:MG746194:MG746195:
MG746196:MG746197:MG746198:MG746199:MG746200:MG746201:MG746202:MG746203
:MG746204:MG746206:MG746208:MG746209:MG746210:MG746212:MG746213:MG74621
4:MG746215:MG746216:MG746217:MG746218:MG746219:MG746220:MG746221:MG7462
22:MG746223:MG746224:MG746225:MK614143:MK614144:MK614145:MK614146:MK614
147:MK614148:MK614149:MK614150:MK614151:MK614152:MK614153:MK614154:MK61
4156:MK614159:MK614161:LC349984:LC349985:MH979229:MK752935:MK614130:MK6
14131:MK614132:MK614133:MK614134:MK614135:MK614136:MK614137:MK764022:MK
762628:MK773571:MK614139:MK614140:MK614141:MK864096:LC413801:LC413802:L
C413803:LC413804:MK614155:MK614157:MK614158:MK614160:MN394542:MN394544

| GII. 2 | F2 | QWMV | SEQ ID NO: 420 | GI I. 2-MG746036:MG746055:MG746044:MG746205:MG746207 |
| GII. 2 | F2 | QWAV | SEQ ID NO: 421 | GII. 2-MG746211 |
| GII. 3, GII. 5, | F2 | QWTL | SEQ ID NO: 422 | GI I. 12–LC342059:KJ196294:KJ196282:KJ196299:GQ845370:JQ613568:HQ688986:J Q613569:HQ449728:HQ664990:KC464498:KC464496:KC464500:KC464499:KC464497: |

[Table 1-77]

| | | | | |
|---|---|---|---|---|
| GII. 7, GII. 12, GII. 17 | | | | HQ401025:KP064099:MK754445:MK762627:MK753036:MK754447:MK764041:MK616558:MK616561:MK616560:MK616559:MK355712:MK355713_GII.17—MH218591:KT589391:KT346358:KY406980:KY406981:MH375799:LC333898:MH375809:MH375810:MH375811:MH375814_GII.3—HM072042:AB385626:AB385527:KC464326:KC464327:MK907798:KC464328:GU138208:KF931324:KJ184223:KF944065:JN565063:JX984948:KC464329:KF944111:KF944110:KJ184256:KF944179:KF944119:KF944147:KX355506:KF895841:KT779557:KF944266:MG892076:KJ634708:KM056394:MH702267:MH702287:MK39677:7:MK396772:MH218570:MH218738:MH218571:MH218712:MH218572:MH218574:MH218185:75:MH218576:MH218577:MH218578:MH218579:MH218580:MH218581:MH218582:MH218:583:MH218587:MH218588:MH218589:MH218590:MH218596:MH218597:MH218732:MK762640:MK90:7787:MH702259:MH218594:MH218595:MH218596:MH218630:KY887597:MH218601:MH218602:MH2:18604:MH218653:MH218654:MH218630:KY887597:KY887598:KY210918:KY:210919:MG892946:MG892955:MK773588:MH279487_GII.5—JN699044:KJ196277:KJ19:6288:KM386680:HM596590:KM386681:KM036378:KU311160_GII.7—JN6990:42 |
| GII. 3, GII. 14 | F2 | QWSL | SEQ ID NO: 423 | GII.14—JN699038:KJ196278:KJ196297:GU017900:GU017903:GU594162:GU017901:GU017902:GU017904:GU017905:GU017906:GU017907:GU017908:GU017826:MK850443:MK588004:MK641589:LC556388:LC556389:LC556390:LC556391:LC556392_GII.3—KY442319:KY442320:HM072320:HM072046:KC597144:JN699040:JX846924:JN699039:HM072043:HM072044:HM072040:HM072041:AB067542:JQ743333:KC464324:KF006265:KC464325:KF931180:GU292851:JN899244:AB385435:AB365634:HM590538:GU980585:KF931243:KF931267:KF931234:LC101823:AB758450:KC464495:KF944203:KC59714:0:KF895859:KF944227:KF944232:KF895848:KY348697:KJ499441:KJ499442:KY4071:72:KY407173:KY407174:KY407175:MN199033:KY348698:LC133339:LC133343:KJ499:444:KJ499445:KT732274:KY767664:KY406922:KY406923:KY406924:KY406925:KY40:6926:KY406927:KY406929:KY406930:KY406931:KY406932:KY406933:KY406934:KY4 |

EP 4 317 436 A1

[Table 1-78]

06937:KY406938:KY406939:KY407176:KY407177:KY407178:KY407179:KY407180:KY
407190:KY407191:KY407192:KY407193:KY407194:KY905334:MG892915:MG892920:M
G892911:MG892910:MK073886:KX989464:KX989465:KX989466:KX989467:KX989468:
KY406928:KY406935:KY406936:KY406940:KY406941:KY406942:KY406943:KY406944
:KY406945:KY406946:KY406947:KY407195:KY407196:KY407197:KY407198:MG89295
3:MG892952:MG892947:MG892951:MG892949:MG892950:MG892956

| | | | | |
|---|---|---|---|---|
| GII.6, GII.17, GII.21 | F2 | QWEL | SEQ ID NO: 424 | GII.17-MK907801:MK907800:LC433694:KU561250:KU561251:LC043168:LC043167:KU557788:KJ156329:LC486737:KU557801:KU557839:MK282256:MK282257:MK282258:LC433720:MK077684:KP998539:KT380915:KT780394:KT780395:KT780396:KT780397:KT780398:KT780399:KU561252:KU561253:LC043139:LC043305:KR083017:KY424349:KY424350:KR020503:KT326180:KU557790:KU557785:KU557786:KU557787:LC486738:KT285173:KX171413:KX171412:KX171417:KP698928:KP698929:KP902566:KP902567:KP902568:KP902569:KP902570:KP902571:KP902572:KP902573:KP902574:KP902576:KP902577:KP902578:KT315668:KT315669:KT315670:KT315671:KT315672:KT315673:KP864103:KP864104:KU561224:KU561225:KU561226:KU561227:KU561228:KU561229:KU557797:KU557798:KU557799:KU557802:KU557803:KU557806:KU557807:KU557808:KU557809:KU557810:KU557811:KU557815:KU557816:KU557817:KU557822:KU557823:KU557824:KU557827:KU557828:KU557829:KU557833:KU557834:KU557835:KU557846:KU557847:KU557848:KU557849:KU557885:KY069114:KX346699:KP902575:KP902563:KP902564:KP902565:KU587625:LC369228:LC369214:MG692610:LC369229:LC369230:KT970369:KT970370:MK907790:MK907791:MK396775:MK907792:MK907793:LC433721:LC433695:KT149168:KT149169:MK077685:MK077706:AB983218:MF918359:KT780400:KT780401:KT780402:KT780403:KT780404:KT780405:KT780406:KT780407:KT780408:KT780409:KT780410:KT780411:KT780412:KT780413:KT780414:KT780415:KT780416:KU561248:KU561249:KT253245:KU561254:KU561255:KU561256:KX356908:NC:LC037415:KU557783:KU557784:KR154230:KR154231:KT992790:KT3261 |

94

[Table 1-79]

81:KT326182:KT992789:KT992786:KT992787:KT992785:KT992788:KY392867:KY392
868:KR052021:KR052019:KR052020:KR052022:LC349987:LC349988:LC349992:KT34
6356:KX024652:LC486739:LC486740:LC486741:LC486747:LC486751:LC486761:LC4
86762:LC486763:LC148844:LC148845:LC148846:LC148847:LC148848:LC148849:LC
148850:LC148851:LC101820:LC177662:KX171414:KX171415:KX171419:KX420892:K
X420891:KX171418:KX171416:KP698930:KP698931:KP902579:KP902580:KP902581:
KP902582:KP902583:KP902584:KP902585:KP902586:KP902587:KP902588:KP902589
:KP902590:KT315674:KT315675:KT315676:KT315677:KT315678:KT315679:KT31568
0:KT315681:KT315682:KT315683:KT315684:KT315685:KT315686:KT315687:KT3156
88:KT315689:KT315690:KT315691:KT315692:KT315693:KT315694:KT315695:KT315
696:KT315697:KT315698:KT315699:KT315700:KT315701:KT315702:KT315703:KT31
5704:KT315705:KT315706:KT315707:KT315708:KT315709:KT315710:KT315711:KT3
15712:KT315713:KT315714:KT315715:KT315716:KT315717:KT315718:KT315719:KP
864102:KT591501:KU953391:KU953392:KU953393:KU561230:KU561231:KU561232:K
U561233:KU561234:KU561235:KU561236:KU561237:KU561238:KU561239:KU561240:
KU561241:KU561242:KU561243:KU561244:KU561245:KU561246:KU561247:KU557800
:KU557804:KU557805:KU557812:KU557813:KU557814:KU557818:KU557819:KU55782
0:KU557821:KU557825:KU557826:KU557830:KU557831:KU557832:KU557836:KU5578
37:KU557838:KU557840:KU557841:KU557842:KU557843:KU557844:KU557845:KU557
850:KU557851:KU557852:KU557853:KU557854:KU557855:KU557856:KU557857:KU55
7858:KU557859:KU557860:KU557861:KU557862:KU557863:KU557864:KU557865:KU5
57866:KU557867:KU557868:KU557869:KU557870:KU557871:KU557872:KU557873:KU
557874:KU557875:KU557876:KU557877:KU557878:KU557879:KU557880:KU557881:K
U557882:KU557883:KU557884:KU557886:KU557887:KU557888:KU557889:KU557890:
KU557891:KU557892:KU557893:KX244850:KX244851:KX244852:KX244853:KU587626
:KU587627:KU587628:KU587629:KX134669:KX134670:KX168437:KX168438:KX37110

[Table 1-80]

7:KX371108:KX371109:KX371110:KY397953:KY397954:KY397955:KY397956:KY3979
57:KY397958:KX346700:KX346701:KX346702:KX346703:KX346704:KX346705:MF172
092:MF172093:MF172094:KY406954:KY406955:KY406956:KY406957:KY406958:KY40
6959:KY406960:KY407181:KY407182:KY407183:KY407184:KY407185:KY407186:LC3
69234:LC369237:LC369233:LC369257:LC369242:LC369255:LC369235:LC369222:LC
369215:LC369227:LC369219:LC369236:LC369244:LC369220:LC369232:LC369238:M
N853414:LC369245:LC369256:LC369224:LC369240:LC369241:MH997861:LC369231:
LC369243:LC369225:LC369239:LC369249:LC369246:LC369247:LC369248:LC369254
:LC369253:LC369252:LC369251:KY905332:MK789431:LC369218:LC369221:LC36921
6:LC369217:KT970371:KT970372:KT970373:KT970374:KT970375:KT970376:KT9703
77:MH747479:MH747481:MH890538:MH747480:LC369258:MH747482:MH890539:LC433
696:LC433698:LC433699:LC433709:LC433710:LC433711:LC433712:LC433713:LC43
3722:LC433723:LC433724:LC433726:LC433727:LC433729:LC433730:LC433731:LC4
33732:KX424646:KX424647:KX424648:KX424649:KX424650:KT149170:KT149171:KT
149172:KT149173:KT149174:KT149175:KT149176:KX216782:KX216783:KX216784:K
X216785:KX216786:KX216788:KX216789:KX216790:KX216791:KX216792:KX216793:
KX216794:KX216795:KX216796:KX216797:KX216798:KX216799:KX216800:KX216801
:KX216802:KX216803:KX216804:KX216805:KX216806:MF073239:MF073240:MF07324
1:MK077686:MK077687:MK077688:MK077689:MK077690:MK077691:MK077701:MK0777
02:MK077703:MK077704:MK077705:MK077708:LC349986:LC349989:LC349990:LC349
991:LC349993:LC486742:LC486746:LC486748:LC486749:LC486750:LC486752:LC48
6753:LC486754:LC486764:LC486765:LC486766:LC148852:LC148853:LC148854:LC1
48855:LC148856:KX420895:KX420894:KX420893:KU953394:KU953395:KU953396:KU
953397:KU953398:KX244854:KX134671:KY069115:KX168439:KX168440:KX168441:K
X168442:KX168443:KX168444:KX168445:KX168446:KX168447:KX168448:KX168449:
KX168450:KX168451:KX168452:KX168453:KX168454:KX168455:KX168456:KX371111

[Table 1-81]

:KX371112:MF172095:MF172096:KX989474:KX989475:KX989476:KX989477:KX98947
8:KY406961:KY406962:KY406963:KY406964:KY406965:KY406966:KY406967:KY4069
68:KY406969:KY406970:KY406971:KY406972:KY406973:KY406974:KY406975:KY406
976:KY406977:KY406978:KY406979:KY407203:KY407204:KY407205:KY407206:KU55
5841:MH218689:KY905330:MH746922:MH746923:MH746924:MH746925:LC318746:LC3
18745:LC318747:LC318748:LC333866:LC333867:LC333868:LC333869:LC333870:LC
333871:LC333872:LC333873:LC333874:LC333875:LC333876:LC333877:LC333878:L
C333879:LC333880:LC333881:LC333882:LC333883:LC333884:LC333885:LC433716:
LC433718:LC433719:LC433733:LC433734:LC433735:LC433736:MF073242:MF073243
:MF073244:MF073245:MF073246:MF073247:MF073248:MF073249:MF073250:MF07325
1:MF073252:MF073253:MF073254:MH375801:MH375802:MH375803:MH375804:MH3758
05:MH572223:MH572224:MH572225:MH572226:MH572227:MH572228:MH572229:MH572
230:MH572231:MK077707:MK077709:MK077710:MK077712:MK077713:LC318750:MF42
1538:MF421539:MF421540:MF421541:MF421542:LC258403:LC311767:LC311768:LC3
11769:LC311770:LC311771:LC311772:LC311773:LC486743:LC486744:LC486745:LC
486755:LC486756:LC486757:LC486758:LC486759:LC486760:LC486767:LC486768:L
C486769:LC486770:MK789432:MK789433:LC318753:LC318754:LC318752:LC333886:
LC333887:LC333888:LC333889:LC333890:LC333891:LC333892:LC333893:LC333894
:LC333896:LC333897:LC333899:LC333900:LC333901:LC318755:LC318756:LC31875
7:LC318758:LC333895:MH375806:MH375808:MH572232:MK077692:MK077693:MK0776
94:MK077696:MK077697:MK077711:MK077714:MG995040:MH375813:MK077695:MK077
698:MK077699:MK077700:MN394546_GII.21-GU138162:GU138163:HM590541:GU1381
76:GU138177:HM590519:KJ196284:MH702268:MH702279:MK396776:JN899245:MH702
281:MH702280:MH702278:MH702282:MH702264:MK396773:KR921935:KR921936:KR92
1937:MH702285:MK396771:KX079488:KT962982:KR921938:KR921939:KR921940:KR9
21941:KR921942:KY406949:KY406950:MH702257:KY210925:KY406951:KY406952:KY

Table 1-82]

406953:KY407199:KY407200:KY407201:KY407202_GII.6-KY424345:KY424346:KY42
4347:KY424348:KC576910:JN699035:JN699036:JN699041:AB078337:DQ093064:AB8
18404:AB818403:AB818402:AB685742:HQ169542:AB818401:AB685741:JN183165:AB
685738:GU969054:GU969055:GU969056:GU969057:HM633213:KX752057:AB682736:A
B818397:AB818398:AB818399:AB758451:AB685739:AB685740:KJ407072:JX984945:
JX984949:MH279832:JX989075:KM461694:KM461693:KP064098:JX984953:MH279834
:KY424341:KY424342:KY424343:KY424344:AB818400:KC464321:MH279831:MH21863
9:MK907789:MK907786:MH702288:MH279837:MH279838:KM036374:KM036373:KM0363
75:LN854568:KX268709:LC133342:LC133338:KU935739:MH218640:MH218641:MH218
643:MH218644:MH218650:MH218719:MN248515:MN248516:MN248517:MN248514:MH21
8642:MH218645:MH218666:MH218667:MH218673:MH279827:MH279839:MH279835:MH2
79828:MG571778:KY406918:MH218687:MH702284:MH702286:KY406919:KY406920:KY
406921:KY407213:KY407214:KY407215:KY407216:MH114014:MK301293:MK956198:M
K956199:MK956197

| GII. 17 | F2 | QWDP | SEQ ID NO: 425 | G11. 17-DQ438972 |

A1: first region of epitope A; A2: second region of epitope A; A3: third region of epitope A F1: first region of epitope F; F2: second region of epitope F Writing of accession number: (genotype)-(accession No.). If there is a plurality of accession numbers, they are written side by side, separated by a colon ":". If there is a plurality of genotypes for a single amino acid sequence, an underscore "_" is added after the last accession number of the first genotype, followed by (second genotype)-(accession number) (the same applies for the third and subsequent genotypes).

[Example 3] Preparation of VLPs

GII.2 VLPs, GII.6 VLPs, and GII.17 VLPs were prepared as follows.

The VP1 genes derived from the following norovirus strains were used:

(1) VP1 gene (SEQ ID NO: 432) derived from Norovirus Hu/GII.2/MK04/2004/JP (Accession number: DQ456824).
(2) VP1 gene (SEQ ID NO: 433) derived from Norovirus Hu/GII.6/GZ2010-L96/Guangzhou/CHN/2011 (Accession number: JX989075).
(3) VP1 gene (SEQ ID NO: 434) derived from Norovirus Hu/GII/JP/2015/GII.P17_GII.17/Kawasaki308 (Accession number: LC037415).

These VP1 genes were incorporated into baculovirus genomic DNA, introduced into Sf9 cells, and recombinant baculovirus was recovered. Recombinant baculovirus was inoculated into High Five cells at the appropriate MOI (Multiplicity of Infection). Four to seven days after infection, the culture solution was collected and centrifuged at 10,000 x g for 60 minutes, and the culture supernatant was subjected to density gradient centrifugation using cesium chloride to obtain VLPs. The obtained VLPs were used in the evaluation of neutralizing activity shown below.

[Example 4] Evaluation of neutralizing activity of identified epitopes

Peptides having the amino acid sequences of GII.6 epitope A, GII.6 epitope D, GII.6 epitope E, GII.17 epitope A, GII.17 epitope D, and GII.17 epitope E identified in Example 1 or 2 were synthesized by the Fmoc solid phase synthesis method.

Examples of the synthesized peptides include the following. The position of the amino acids determined for each epitope corresponds to the position of the amino acids counted from the N-terminus of the amino acid sequence of the VP1 protein.

- GII.6 (GZ2010 (JX989075))

  Epitope A: positions 290 to 311 (SEQ ID NO: 54);
  Epitope D: positions 401 to 410 (SEQ ID NO: 426) (the sequence from positions 404 to 408 (SEQ ID NO: 312) with Gly-Met-Gly and Gln-Trp, respectively added to N-terminus and C-terminus thereof; Gly-Met-Gly and Gln-Trp can be conservative regions); and
  Epitope E: positions 413 to 423 (SEQ ID NO: 427) (the sequence from positions 414 to 420 (SEQ ID NO: 372) with Pro and Leu-Asn-Met, respectively added to N-terminus and C-terminus thereof; Pro and Leu-Asn-Met can be conservative regions).

- GII.17 (Kawasaki308 (LC037415))

  Epitope A: positions 370 to 384 (SEQ ID NO: 428) (the sequence from positions 371 to 383 (SEQ ID NO: 253) with Asn and Thr, respectively added to N-terminus and C-terminus thereof; Asn and Thr can be conservative regions);
  Epitope D: positions 388 to 403 (SEQ ID NO: 429) (the sequence from positions 390 to 401 (SEQ ID NO: 334) with Pro-Val and Gln-Trp, respectively added to N-terminus and C-terminus thereof; Pro-Val and Gln-Trp can be conservative regions); and
  Epitope E: positions 406 to 415 (SEQ ID NO: 430) (the sequence from positions 407 to 414 (SEQ ID NO: 384) with Pro and Asn, respectively added to N-terminus and C-terminus thereof; Pro and Asn can be conservative regions).

The following describes the tests using the synthetic peptides listed above.

Rabbits were immunized four times by intradermal administration using each of the synthesized peptides, and serum was collected 49 days after the first immunization. Serum was also collected before immunization.

Neutralizing activity tests were performed using the collected sera. With reference to Haynes, J et al., Viruses 2019; 11(5):392, the neutralizing activity tests were performed under the following conditions.

Porcine gastric mucin (PGM) solution (1.0 $\mu$g/mL, PBS) was prepared and 100 $\mu$L was added to each well and then allowed to stand for 2 hours at 25°C. Then, the liquid was removed and 300 $\mu$L of washing buffer (PBS with 0.05% Tween 20) was added to wash the wells (this washing procedure was repeated three times thereafter). 200 $\mu$L of 5% skim milk-PBS solution was added to each well, and then allowed to stand overnight at 4°C. A mixed solution (with serum) was prepared by mixing 50 $\mu$L each of GII.6 or GII.17 VLP solution and rabbit serum collected as described above, which was diluted from 1 to 128 times, and was allowed to stand at 25°C for one hour. After washing the wells, 100 μL of the above mixed solution was added to the wells and allowed to stand at 25°C for one hour. After washing the wells, 100 μL of primary antibody solution (Mouse anti-NoV VLP Antibody in PBS with 0.05% Tween 20 and 5% skim milk) was added to each well and allowed to stand at 25°C for one hour. After washing, 100 μL of secondary antibody solution (Anti-Mouse IgG Antibody, HRP conjugated in PBS with 0.05% Tween 20 and 5% skim milk) was added to each well and allowed to stand at 25°C for one hour. After washing, 100 μL of TMB (3,3',5,5'-tetramethylbenzidine) solution was added to each well and allowed to stand at 25°C for 30 minutes, shielded from light. The reaction was stopped by adding 100 μL of Stop Solution, and the absorbance at a wavelength of 450 nm was measured.

PBS with 0.05% Tween 20 and 5% skim milk alone was mixed with VLPs to prepare a mixed solution (without serum). The mixed solution (without serum) was used instead of the above mixed solution (with serum) to perform the same operations as above. The same operations as above were also performed using PBS with 0.05% Tween 20 and 5% skim milk (without VLPs or rabbit serum) as a blank, instead of the above mixed solution (with serum).

The binding blockade activity was calculated by applying the OD [with serum] obtained using the mixed solution with serum, the OD [without serum] obtained using the mixed solution without serum, and the OD [blank] obtained using the blank to the following equation (Fig. 2).

$$\text{Binding blockade activity} = 100\ (\%) - [(\text{OD [with serum]} - \text{OD [blank]})\ (\text{OD [without serum]} - \text{OD [blank]})] \times 100\ (\%)$$

The presence of neutralizing activity is based on the Blocking Titer (BT) 50 value. BT50 is the value of the maximum serum dilution factor at which binding blockade activity exceeds 50%. The BT50 values for the present test are shown in Table 2, and neutralizing activity was considered to be present if the BT50 value of the serum after immunization was twice or more the BT50 value of the serum before immunization. However, if the BT50 value of the serum before immunization was outside the detection range, neutralizing activity was considered to be present if the BT50 value of the serum after immunization could be detected. As shown in Table 2, all of the sera collected from rabbits immunized with the epitope peptides were found to have neutralizing activity. This suggests that immunization with the epitope peptides of GII.6 induced antibodies with neutralizing activity against GII.6 and immunization with the epitope peptides of GII.17 induced antibodies with neutralizing activity against GII.17.

[Table 2]

| Table 2: BT50 values of rabbit antisera | | | |
|---|---|---|---|
| | | BT50 before immunization | BT50 after immunization |
| GII.6 | Epitope A | 1 | 128 |
| | Epitope D | 1 | 16 |
| | Epitope E | <1 | 1 |
| GII.17 | Epitope A | 1 | 8 |
| | Epitope D | 2 | 4 |
| | Epitope E | <1 | 1 |

[Example 5] Evaluation of neutralizing activity of identified epitopes

Peptides with the amino acid sequences of GII.2 epitope A and GII.2 epitope D identified in Example 1 or 2 were synthesized by the Fmoc solid phase synthesis method.

Examples of the synthesized peptides include the following. The position of the amino acids determined for each epitope corresponds to the position of the amino acids counted from the N-terminus of the amino acid sequence of the VP1 protein.

• GII.2 (MK04 (DQ456824))

Epitope A: positions 375 to 388 (SEQ ID NO: 167); and
Epitope D: positions 395 to 405 (SEQ ID NO: 431) (the sequence from positions 396 to 403 (SEQ ID NO: 273) with Gly and Gln-Trp, respectively added to N-terminus and C-terminus thereof; Gly and Gln-Trp can be conservative regions).

The following describes the tests using the synthetic peptides listed above.

Rabbits were immunized four times by intradermal administration using each of the synthesized peptides, and serum was collected 49 days after the first immunization. Serum was also collected before immunization.

Neutralizing activity tests were performed using the collected sera. With reference to Haynes, J et al., Viruses 2019; 11(5):392, the neutralizing activity tests were performed under the following conditions.

Porcine gastric mucin (PGM) solution (1.0 μg/mL, PBS) was prepared and 100 μL was added to each well and then allowed to stand for 2 hours at 25°C. Then, the liquid was removed and 300 μL of washing buffer (PBS with 0.05% Tween 20) was added to wash the wells (this washing procedure was repeated three times thereafter). 200 μL of 5% skim milk-PBS solution was added to each well, and then allowed to stand overnight at 4°C. After washing, 100 μL of glycosyltransferase reaction solution (0.25 μg/mL B-type glycosyltransferase, 50 mM HEPES, pH 6.5, 1.5 mM UDP-galactose, 5 mM MnCl$_2$) was added to each well and allowed to stand at 37°C for 2 hours. While performing the transglycosylation reaction, a mixed solution (with serum) was prepared by mixing 50 μL each of GII.2 VLP solution and rabbit serum collected as described above, which was diluted from 1 to 128 times, and was allowed to stand at 25°C for one hour. After washing the wells, 100 μL of the above mixed solution was added to the wells and allowed to stand at 25°C for one hour. After washing the wells, 100 μL of primary antibody solution (Mouse anti-NoV VLP Antibody in PBS with 0.05% Tween 20) was added to each well and allowed to stand at 25°C for one hour. After washing, 100 μL of secondary antibody solution (Anti-Mouse IgG Antibody, HRP conjugated in PBS with 0.05% Tween 20) was added to each well and allowed to stand at 25°C for one hour. After washing, 100 μL of TMB (3,3',5,5'-tetramethylbenzidine) solution was added to each well and allowed to stand at 25°C for 30 minutes, shielded from light. The reaction was stopped by adding 100 μL of Stop Solution, and the absorbance at a wavelength of 450 nm was measured.

PBS with 0.05% Tween 20 and 5% skim milk alone was mixed with VLPs to prepare a mixed solution (without serum). The mixed solution (without serum) was used instead of the above mixed solution (with serum) to perform the same operations as above. The same operations as above were also performed using PBS with 0.05% Tween 20 and 5% skim milk (without VLPs or rabbit serum) as a blank, instead of the above mixed solution (with serum).

The binding blockade activity was calculated by applying the OD [with serum] obtained using the mixed solution with serum, the OD [without serum] obtained using the mixed solution without serum, and the OD [blank] obtained using the blank to the following equation (Fig. 3).

$$\text{Binding blockade activity} = 100\ (\%) - [(\text{OD [with serum]} - \text{OD [blank]})\ (\text{OD [without serum]} - \text{OD [blank]})] \times 100\ (\%)$$

The presence of neutralizing activity is based on the Blocking Titer (BT) 50 value. BT50 is the value of the maximum serum dilution factor at which binding blockade activity exceeds 50%. The BT50 values for the present test are shown in Table 3, and neutralizing activity was considered to be present if the BT50 value of the serum after immunization was twice or more the BT50 value of the serum before immunization. However, if the BT50 value of the serum before immunization was outside the detection range, neutralizing activity was considered to be present if the BT50 value of the serum after immunization could be detected. As shown in Table 3, the sera collected from rabbits immunized with the epitope peptides were found to have neutralizing activity. This suggests that immunization with the epitope peptides of GII.2 induced antibodies with neutralizing activity against GII.2.

[Table 3]

| Table 3: BT50 values of rabbit antisera | | | |
|---|---|---|---|
| | | BT50 before immunization | BT50 after immunization |
| GII.2 | Epitope A | 4 | 8 |
| | Epitope D | 2 | 8 |

Claims

1. A peptide comprising a portion of an amino acid sequence of a P domain of a VP1 protein of a norovirus, wherein the norovirus belongs to any of genotypes of genogroup II (GII) (except genotype 4).

2. The peptide according to claim 1, wherein the norovirus belongs to a genotype selected from the group consisting ofGII.1, 2, 3, 5, 6, 7, 12, 13, 14, 17, and 21.

3. The peptide according to claim 1 or 2, wherein the portion of an amino acid sequence of a P domain comprises the whole or a portion of an amino acid sequence of a neutralizing epitope selected from the group consisting of epitope A, epitope D, epitope E, epitope F, and combinations thereof.

4. The peptide according to any one of claims 1 to 3, wherein the epitope A has an amino acid sequence selected from the group consisting of SEQ ID NOs: 1 to 267,

    the epitope D has an amino acid sequence selected from the group consisting of SEQ ID NOs: 268 to 351,
    the epitope E has an amino acid sequence selected from the group consisting of SEQ ID NOs: 352 to 390, and
    the epitope F has an amino acid sequence selected from the group consisting of SEQ ID NOs: 391 to 425.

5. The peptide according to any one of claims 1 to 4, further comprising an amino acid sequence which is not present in a P domain of a VP1 protein of a naturally occurring norovirus belonging to any of genotypes of GII (except genotype 4).

6. The peptide according to any one of claims 1 to 5, further comprising an amino acid sequence which is not present in a P domain of a VP1 protein of a naturally occurring norovirus belonging to a genotype selected from the group consisting of GII.1, 2, 3, 5, 6, 7, 12, 13, 14, 17, and 21.

7. A polynucleotide encoding the peptide according to any one of claims 1 to 6.

8. The polynucleotide according to claim 7, incorporated into an expression vector.

9. The polynucleotide according to claim 7 or 8, introduced into a host cell.

10. An antibody that recognizes the peptide according to any one of claims 1 to 6.

11. The antibody according to claim 10, wherein the antibody recognizes the P domain of a VP1 protein of a norovirus.

12. A composition comprising the peptide according to any one of claims 1 to 6.

13. A composition comprising the polynucleotide according to any one of claims 7 to 9.

14. The composition according to claim 12 or 13, which is a vaccine.

15. The composition according to any one of claims 12 to 14, for use in inducing protective immunity to a norovirus belonging to any of genotypes of GII (except genotype 4).

16. The composition according to any one of claims 12 to 15, for use in inducing protective immunity to a norovirus belonging to a genotype selected from the group consisting of GII.1, 2, 3, 5, 6, 7, 12, 13, 14, 17, and 21.

17. A composition comprising the antibody according to claim 10 or 11.

18. The composition according to any one of claims 12 to 17, for use in treating an infection with a norovirus belonging to any of genotypes of GII (except genotype 4).

19. The composition according to any one of claims 12 to 18, for use in treating an infection with a norovirus belonging to a genotype selected from the group consisting of GII.1, 2, 3, 5, 6, 7, 12, 13, 14, 17, and 21.

20. A method for identifying a neutralizing epitope for a norovirus, the method comprising:

    collecting genome sequences of one or more norovirus strains belonging to a specific genotype, and extracting gene sequences of a VP1 protein from the genome sequences,
    aligning amino acid sequences of the VP1 protein, and classifying the norovirus strains into one or more clusters based on the similarity of the amino acid sequences, the phylogenetic closeness of the strains from which these amino acid sequences are derived, or a combination thereof,

predicting, by homology modeling, a three-dimensional structure of a P domain of the VP1 protein of the norovirus strains representing the clusters,

comparing the predicted three-dimensional structure of the P domain with a three-dimensional structure of a P domain of a VP1 protein of a reference strain of norovirus to identify, as a neutralizing epitope, a predicted portion of the P domain that structurally corresponds to a neutralizing epitope of the P domain of the reference strain and an amino acid sequence in the vicinity of that portion, which is a highly variable region between genotypes, and

comparing an amino acid sequence of the P domain of the VP1, which comprises the identified neutralizing epitope, with an amino acid sequence of a P domain of VP1 from a different strain that belongs to the same genotype as the above-described strain from which the amino acid sequence is derived and whose three-dimensional structure was not compared above, to determine a portion in the amino acid sequence of the P domain of VP1 from the different strain corresponding to the amino acid sequence of the neutralizing epitope, and identify that portion as the neutralizing epitope of the P domain of VP1 from the different strain,

wherein the reference strain of norovirus belongs to a genotype different from one or more norovirus strains belonging to that specific genotype, and

the neutralizing epitope of the P domain of the VP1 protein of the reference strain is known.

21. The method according to claim 20, wherein the norovirus belonging to the specific genotype is a norovirus belonging to any of genotypes of genogroup II (GII) (except genotype 4).

22. The method according to claim 20 or 21, wherein the norovirus belonging to the specific genotype is a norovirus belonging to a genotype selected from the group consisting of GII. 1, 2, 3, 5, 6, 7, 12, 13, 14, 17, and 21.

23. The method according to any one of claims 20 to 22, wherein the reference strain of norovirus is selected from norovirus strains belonging to GII.4.

# Fig. 1

GII.4 norovirus epitope

Identified GII.6 norovirus epitope

# Fig. 2

After immunization    Before immunization

Fig. 3

## INTERNATIONAL SEARCH REPORT

| | International application No. |
|---|---|
| | **PCT/JP2022/015581** |

**A.    CLASSIFICATION OF SUBJECT MATTER**

*C12N 15/40*(2006.01)i; *A61K 39/12*(2006.01)i; *A61P 1/12*(2006.01)i; *A61P 31/12*(2006.01)i; *C07K 14/08*(2006.01)i; *C07K 16/10*(2006.01)i; *C12N 15/63*(2006.01)i

FI:    C12N15/40; A61K39/12; A61P1/12; A61P31/12; C07K14/08 ZNA; C07K16/10; C12N15/63 Z

According to International Patent Classification (IPC) or to both national classification and IPC

**B.    FIELDS SEARCHED**

Minimum documentation searched (classification system followed by classification symbols)

C12N15/40; A61K39/12; A61P1/12; A61P31/12; C07K14/08; C07K16/10; C12N15/63

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN); CAplus/MEDLINE/EMBASE/BIOSIS (STN); UniProt/GeneSeq

**C.    DOCUMENTS CONSIDERED TO BE RELEVANT**

| Category* | Citation of document, with indication, where appropriate, of the relevant passages | Relevant to claim No. |
|---|---|---|
| X | WANG, Haibo et al. Complete nucleotide sequence analysis of the norovirus GII.17: A newly emerging and dominant variant in China, 2015. Infection, Genetics and Evolution. 10 December 2015, vol. 38, pp. 47-53<br>entire text | 1-23 |
| X | XUE, Liang et al. Molecular characterization of new emerging GII.17 norovirus strains from South China. Infection, Genetics and Evolution. 23 February 2016, vol. 40, pp. 1-7, supplementary data<br>entire text | 1-23 |
| X | XUE, Liang et al. Genome characterization of a GII.6 norovirus strain identified in China. Infection, Genetics and Evolution. 04 February 2015, vol. 31, pp. 110-117<br>entire text | 1-23 |
| X | XUE, Liang et al. Comparative phylogenetic analyses of recombinant noroviruses based on different protein-encoding regions show the recombinationassociated evolution pattern. Scientific Reports. 10 July 2017, vol. 7, 4976, pp. 1-10<br>entire text | 1-23 |

☑ Further documents are listed in the continuation of Box C.        ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **20 April 2022** | **10 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/015581** |

**C.** **DOCUMENTS CONSIDERED TO BE RELEVANT**

| Category* | Citation of document, with indication, where appropriate, of the relevant passages | Relevant to claim No. |
| --- | --- | --- |
| X | KOO, Eung-Seo et al. Occurrence of novel GII.17 and GII.21 norovirus variants in the coastal environment of South Korea in 2015. PLOS ONE. 15 February 2017, vol. 12, e0172237, pp. 1-16<br>      abstract, fig. 4-5 | 1-19 |
| A | | 20-23 |
| X | QIU, Shuxing et al. Production and characterization of monoclonal antibodies against GII.6 norovirus virus-like particles. Microbial Pathogenesis. 26 February 2020, vol. 142, 104100, pp. 1-8<br>      abstract, pp. 2-3, fig. 6 | 1-19 |
| A | | 20-23 |
| A | WO 2019/079594 A1 (THE UNIVERSITY OF NORTH CAROLINA AT CHAPEL HILL) 25 April 2019 (2019-04-25)<br>      entire text | 1-23 |

Form PCT/ISA/210 (second sheet) (January 2015)

**INTERNATIONAL SEARCH REPORT**

| International application No. |
|---|
| **PCT/JP2022/015581** |

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/015581**

| Patent document cited in search report | Publication date (day/month/year) | Patent family member(s) | Publication date (day/month/year) |
|---|---|---|---|
| WO 2019/079594 A1 | 25 April 2019 | US 2021/0371468 A1 entire text | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017214357 A

**Non-patent literature cited in the description**

- **CHHABRA et al.** *Journal of General Virology,* 2019, vol. 100, 1393-1406
- **LINDESMITH LC ; BARIC RS.** *Immunity,* 2019, vol. 50, 1530
- **HAYNES, J et al.** *Viruses,* 2019, vol. 11 (5), 392